# EUROPEAN PATENT APPLICATION

(11) **EP 4 067 490 A1**
(43) Date of publication of application: **05.10.2022**
(21) Application number: 20893086.7
(22) Date of filing: 24.11.2020
(51) Int. Cl.: C12N 15/12, A61K 35/17, A61P 35/00, A61P 37/04, C12N 5/0783, C12N 5/10, C12N 15/13, C12N 15/62

(54) **T-CELL MASTER CELL BANK**

(30) Priority: 25.11.2019 JP 2019212718
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); ARIMA, Suguru, Fujisawa-shi, Kanagawa 251-0012 (JP); TAKIGUCHI, Maiko, Fujisawa-shi, Kanagawa 251-0012 (JP); KASSAI, Yoshiaki, Fujisawa-shi, Kanagawa 251-0012 (JP); HAYASHI, Akira, Fujisawa-shi, Kanagawa 251-0012 (JP)
(74) Representative: Hoffmann Eitle
(86) International application number: PCT/JP2020/043573
(87) International publication number: WO 2021/106832

(57) **Abstract**

The present invention provides a system for providing a T cell product, including a T cell master cell bank and/or a T cell working cell bank.

## Description

### [TECHNICAL FIELD OF THE INVENTION]

The present invention relates to a master cell bank of T cell, a working cell bank of T cell, and a system and the like for providing a T cell product, including the bank.

### (Background of the Invention)

In recent years, immune cell therapy has been attracting attention as a treatment method for cancer. Immune cell therapy is a therapeutic method including proliferating and activating immune cells outside the patient's body and administering the immune cells to the patient to allow the immune cells to attack the cancer cells. Immune cell therapy is advantageous in that it causes almost no side effects compared to the conventional three major therapies of surgical treatment, radiation therapy, and chemotherapy. There are various kinds of treatment methods for immune cell therapy. Among them, off-the-shelf allogeneic T cell products are highly expected.

As a system for providing the above-mentioned allogeneic T cell products, a system has been reported in which an iPS cell prepared by introducing a CAR gene into an iPS cell in advance using an iPS cell banking technique (e.g., patent document 1 and the like) or a technique for introducing a CAR gene into an iPS cell (e.g., patent document 2 and the like) is expansion cultured, the cells are stocked to construct a master cell bank of iPS cells having the CAR gene, and T cells obtained by differentiating the iPS cells derived from the cell bank into T cells are used as an allogeneic T cell product (e.g., non-patent document 1).

In the above-mentioned system, a process of introducing an exogenous gene such as a CAR gene or the like into a pluripotent stem cell such as an iPS cell and differentiating the cell into a T cell is required. Therefore, it is necessary to maintain, differentiate, and culture cells (e.g., iPS cell, T cell, CAR-T cell) at each stage of the process while satisfying GMP (Good Manufacturing Practice) standards set by the authorities of each country. For example, since a master cell bank of iPS cells is used, preparation of T cell products requires differentiation of iPS cells into T cells. Due to the multipotency of iPS cells, however, high level of process and quality management is always required to induce uniform differentiated cells from each lot, and human, time and financial costs are extremely high. Such costs have a great influence on the stable provision of T cell products. Particularly, when plural kinds of T cell products are provided, the cost and time of each of them are accumulated and the influence thereof becomes more serious. Therefore, a system for supplying a product with a low cost while satisfying the GMP standard, and further, a system capable of providing a high-quality product with a low cost even when plural kinds of T cell products are used, have been desired.

### [Document List]

### [Patent documents]

Patent document 1: US-A-2009-0299763
Patent document 2: WO 2017/088012

### [Non-patent document]

Non-patent document 1: Bob Valamehr (Vice President of Fate Therapeutics) "Generation of off-the-shelf TCR-less CAR T cells from renewable pluripotent cells", April 14-18, 2018 (AACR Annual Meeting 2018 Press Program)

### [SUMMARY OF THE INVENTION]

### [Problems to be Solved by the Invention]

Accordingly, the present invention aims to provide a system for providing a high quality, off-the-shelf allogenic T cell product, that can reduce human, time and financial costs and minimize difference between lots. The present invention also aims to provide a master cell bank of T cell, a working cell bank of T cell, and the collection of these that can be used for the provision of the aforementioned system.

### [Means of Solving the Problems]

The present inventors have conducted intensive studies in an attempt to solve the aforementioned problems and assumed that the conventional techniques described in non-patent document 1 and the like exclusively focus on the construction method of a master cell bank of iPS cell because iPS cells are superior to T cells in the proliferative capacity. Thus, they considered that a T cell or T cells sufficiently proliferated by expansion culture can be used as a master cell bank. That is, the present inventors conceived an idea that a high-quality T cell product can be rapidly provided from a master cell bank of T cell containing an exogenous gene such as CAR gene and the like, rather than a master cell bank of iPS cell having the exogenous gene. As far as the present inventors knew, the idea of constructing a master cell bank of T cell was a completely unknown and completely new idea. The present inventors have further studied based on these findings and completed the present invention.

Therefore, the present invention provides the following.
[1] A system for providing a T cell product, comprising a master cell bank of T cell and/or a working cell bank of T cell.
[2] The system of [1], wherein the master cell bank of T cell and/or working cell bank of T cell comprise(s) a T cell derived from an induced pluripotent stem cell.
[3] The system of [1] or [2], wherein the master cell bank of T cell and/or working cell bank of T cell comprise(s) a T cell with suppressed expression of at least one kind of HLA gene.
[3a] The system of any one of [1] to [3], wherein the master cell bank of T cell and/or working cell bank of T cell comprise(s) a T cell into which an exogenous gene has been introduced.
[3b] The system of any one of [1] to [3a], further comprising a step of constructing a master cell bank collection of T cell and/or a working cell bank collection of T cell comprising two or more kinds of master cell banks of T cell and/or working cell banks of T cell by collecting the master cell banks of T cell and/or the working cell banks of T cell.
[3c] The system of any one of [1] to [3b], wherein the master cell bank of T cell and/or working cell bank of T cell comprise(s) two or more kinds thereof.
[3d] The system of any one of [1] to [3c], further comprising a step of selecting a master cell bank of T cell and/or working cell bank of T cell.
[4] The system of any one of [1] to [3d], further comprising a step of introducing a nucleic acid comprising the exogenous gene into a T cell prepared from the master cell bank of T cell and/or working cell bank of T cell.
[5] The system of [4], wherein the exogenous gene is a CAR gene or an exogenous TCR gene.
[6] The system of any one of [1] to [5], wherein the T cell product comprises two or more kinds thereof.
[6a] The system of any one of [1] to [6], further comprising a step of selecting the T cell product.
[7] The system of any one of [1] to [6a], further comprising a step of expansion culturing a T cell prepared from the master cell bank of T cell and/or working cell bank of T cell.
[8] The system of [7], wherein the step of expansion culturing the T cell comprises a process of stimulating the cell with a CD30 agonist.
[8a] The system of [7], wherein the step of expansion culturing the T cell comprises a process of culturing the cell in the presence of a CD30 agonist.
[9] The system of any one of [7] to [8a], further comprising a step of producing a frozen T cell product comprising the expansion cultured T cell.
[10] The system of any one of [6] to [9], further comprising a step of constructing a T cell product collection comprising two or more kinds of T cell products by collecting T cell products.
[10a] The system of any one of [1] to [10], further comprising a step of obtaining information of a test subject.
[10b] The system of [10a], further comprising a step of selecting an appropriate master cell bank of T cell and/or an appropriate working cell bank of T cell based on the information of the test subject.
[10c] The system of [10a], further comprising a step of selecting an appropriate T cell product based on the information of the test subject.
[11] The system of any one of [1] to [10c], further comprising a step of identifying a tumor-specific antigen or a tumor-associated antigen expressed in a tumor of the test subject.
[12] The system of [11], further comprising a step of selecting a T cell product expressing a CAR or an exogenous TCR that recognizes and binds to the identified antigen from a T cell product collection comprising two or more kinds of T cell products.
[13] A method for producing a T cell product, comprising a process of constructing a master cell bank of T cell and/or a working cell bank of T cell.
[14] The method for producing a T cell product of [13], wherein the master cell bank of T cell and/or working cell bank of T cell comprise(s) a T cell derived from an induced pluripotent stem cell.
[15] The method for producing a T cell product of [13] or [14], wherein the master cell bank of T cell and/or working cell bank of T cell comprise(s) a T cell with suppressed expression of at least one kind of HLA gene.
[15a] The method for producing a T cell product of any one of [13] to [15], wherein the master cell bank of T cell and/or working cell bank of T cell comprise(s) a T cell into which an exogenous gene has been introduced.
[16] The method for producing a T cell product of any one of [13] to [15a], further comprising a process of introducing a nucleic acid comprising the exogenous gene into a T cell prepared from the master cell bank of T cell and/or working cell bank of T cell.
[17] The method for producing a T cell product of [16], wherein the exogenous gene is a CAR gene or an exogenous TCR gene.
[18] The method for producing a T cell product of any one of [13] to [17], further comprising a process of expansion culturing a T cell.
[19] The method for producing a T cell product of [18], wherein the process of expansion culturing the T cell comprises a process of stimulating the cell with a CD30 agonist.
[19a] The method for producing a T cell product of [18], wherein the process of expansion culturing the T cell comprises a process of culturing the cell in the presence of a CD30 agonist.
[20] A master cell bank of T cell and/or a working cell bank of T cell.
[21] The master cell bank of T cell and/or working cell bank of T cell of [20], comprising a T cell derived from an induced pluripotent stem cell.
[22] The master cell bank of T cell and/or working cell bank of T cell of [20] or [21], comprising a T cell with suppressed expression of at least one kind of HLA gene.
[22a] The master cell bank of T cell and/or working cell bank of T cell of any one of [20] to [22], comprising a T cell into which an exogenous gene has been introduced.
[23] A master cell bank collection of T cell and/or a working cell bank collection of T cell comprising two or more kinds of the master cell banks of T cell and/or working cell banks of T cell of any one of [20] to [22a].
[24] A method for constructing a master cell bank of T cell and/or a working cell bank of T cell, comprising the following processes:
   (I) a process of differentiating an induced pluripotent stem cell free of a chimeric antigen receptor (CAR) gene into a T cell for CAR-T therapy,
   (II) a process of stocking the differentiated T cell, and
   (III) a process of Characterisation of the differentiated T cell.
[25] A method for constructing a master cell bank of T cell and/or a working cell bank of T cell, comprising the following processes:
   (i) a process of differentiating an induced pluripotent stem cell free of an exogenous T cell receptor (TCR) gene into a T cell for TCR-T therapy,
   (ii) a process of stocking the differentiated T cell, and
   (iii) a process of Characterisation of the differentiated T cell.
[25a] The method of [24] or [25], wherein the induced pluripotent stem cell has an exogenous gene.
[26] The method of [24] or [25a], wherein the induced pluripotent stem cell has an exogenous T cell receptor (TCR) gene.
[27] The method of any one of [24] to [26], wherein at least one kind of HLA gene is deleted in the induced pluripotent stem cell.
[27a] The method of any one of [24] to [26], wherein the induced pluripotent stem cell is suppressed in expression of at least one kind of HLA gene.
[28] The method of any one of [24] to [27a], wherein the T cell expresses CD8αβ.
[29] A master cell bank of T cell and/or a working cell bank of T cell constructed by the method of any one of [24] to [28].
[30] A method for producing a T cell product expressing a CAR or an exogenous TCR, comprising the following processes:
   (A) a process of preparing a T cell from the master cell bank of T cell and/or the working cell bank of T cell of [29],
   (B) a process of introducing a CAR gene or an exogenous TCR gene into the prepared T cell, and
   (C) a process of expansion culturing the T cell into which the CAR gene or exogenous TCR gene has been introduced.
[30a] The method of [30], further comprising (D) a process of freezing the expansion cultured T cell.
[31] The method of [30] or [30a], wherein the CAR or exogenous TCR recognizes and binds to a tumor-specific antigen or a tumor-associated antigen.
[32] The method of any one of [30] to [31], wherein the process (C) comprises a process of stimulating the T cell with a CD30 agonist.
[32a] The method of any one of [30] to [31], wherein the process (C) comprises a process of culturing the cells in the presence of a CD30 agonist.
[33] A T cell product produced by the method of any one of [30] to [32a].
[34] A method for constructing a T cell product collection comprising two or more kinds of T cell products, comprising a process of collecting the T cell product of [33].
[35] A T cell product collection constructed by the method of [34] .
[36] A method for providing a T cell product suitable for a test subject, comprising the following processes:
   (x) a process of identifying a tumor-specific antigen or a tumor-associated antigen expressed in the tumor of a test subject, and
   (y) a process of selecting a T cell product that expresses a CAR or an exogenous TCR that recognizes and binds to the identified antigen from the T cell product collection of [35] .
[37] A method for providing a T cell product suitable for a test subject, comprising the following processes:
   (p) a process of obtaining information of a test subject, and
   (q) a process of selecting an appropriate master cell bank of T cell and/or an appropriate working cell bank of T cell based on the obtained information of the test subject.

### [Effect of the Invention]

According to the present invention, a system for providing a high quality, off-the-shelf allogenic T cell product, that can reduce human, time and financial costs and minimize difference between lots, and a method for providing same are provided. Such system and method for provision are particularly superior when plural kinds of T cell products are provided. The present invention also provides a master cell bank of T cell, a working cell bank of T cell, and the collection of these that can be used for the provision of the aforementioned system or method are also provided.

Various cells used in the present specification are sometimes indicated as follows. The outline of each cell is also described.

### <induced pluripotent stem cell (iPSC)>

αβ-iPSC: iPS cell into which TCR-α chain gene (TRA gene) and TCR-β chain gene (TRB gene) have been introduced

Vγ9Vδ2-iPSC: iPS cell into which TCR-γ chain gene (TRG gene) and TCR-δ chain gene (TRD gene) encoding Vγ9Vδ2TCR G115 have been introduced

### <hematopoietic progenitor cell (HPC)>

Vγ9Vδ2-iHPC: HPC into which TCR-γ chain gene (TRG gene) and TCR-δ chain gene (TRD gene) encoding Vγ9Vδ2TCR G115 have been introduced

### <T cell>

iγδTC: T cell differentiated from iPS cell free of introduction of exogenous TCR
iαβTC: T cell differentiated from αβ-iPSC
Vγ9Vδ2-iTC: T cell differentiated from Vγ9Vδ2-iPSC
Vγ9Vδ2-iHTC: T cell differentiated from Vγ9Vδ2-iHPC

### <T cell into which CAR gene has been introduced>

CD19iαβCARTC: T cell produced by introducing gene encoding anti-CD19-CAR into iαβTC

BCMAiαβCARTC: T cell produced by introducing gene encoding anti-BCMA-CAR into iαβTC

CD19-CD30-iαβCARTC: T cell produced by introducing gene encoding anti-CD19-CAR containing CD30-derived intracellular domain into iαβTC

### <T cell into which CAR gene and IL-15Rα/IL-15 gene have been introduced>

CD19/IL15iαβCARTC: T cell produced by introducing gene encoding anti-CD19-CAR and gene encoding IL-15Rα/IL-15 chimeric protein into iαβTC

CD19/IL15iVγ9Vδ2CARTC: T cell produced by introducing gene encoding anti-CD19-CAR and gene encoding IL-15Rα/IL-15 chimeric protein into Vγ9Vδ2-iTC

CD19/IL15iγδCARTC: T cell produced by introducing gene encoding anti-CD19-CAR and gene encoding IL-15Rα/IL-15 chimeric protein into iγδTC

### [BRIEF DESCRIPTION OF THE DRAWINGS]

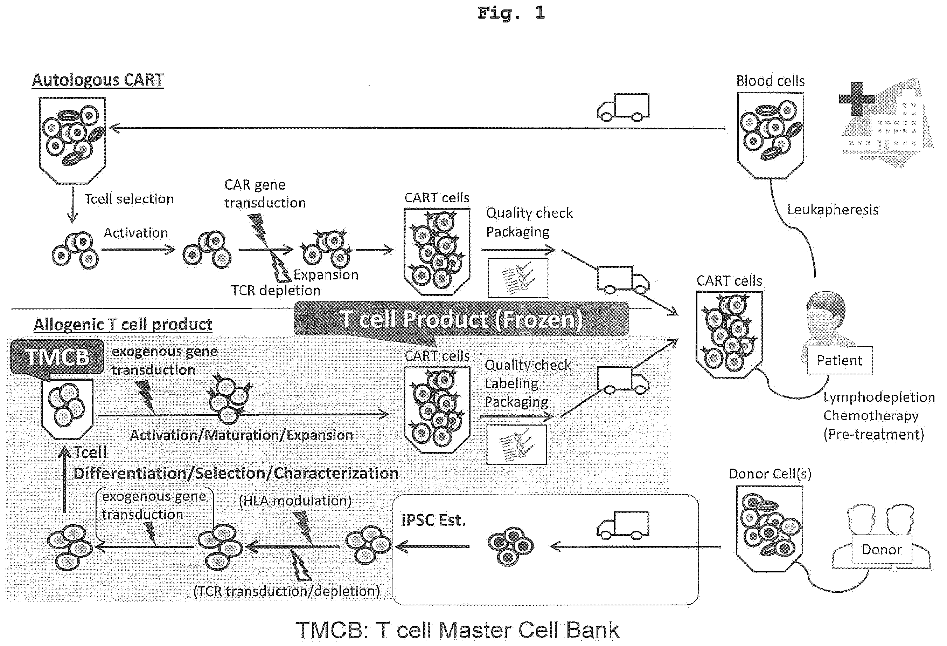

Fig. 1 shows one embodiment of a T cell product providing system including a cell bank of T cell.

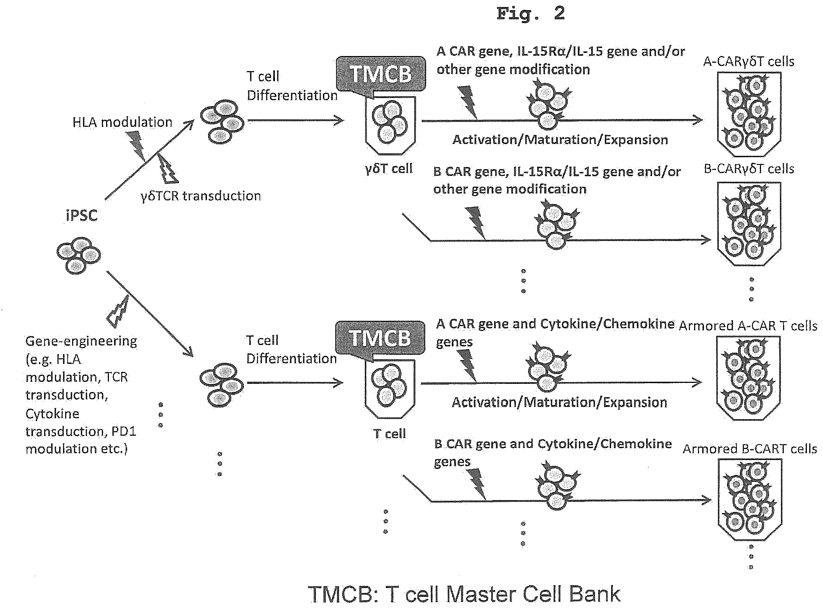

Fig. 2 shows one embodiment of a system for providing plural T cell products from a cell bank collection of T cell. In Fig. 2, "Armored" means that an exogenous gene relating to cytokine and/or chemokine secretion has been introduced.
Fig. 3 shows expression of CD3, γδTCR and αβTCR on the cellular membrane surface of iγδTC. The filled peaks show the results of the antibody non-staining group, and the blank peaks show the staining results using each antigen-specific antibody.
Fig. 4 shows expression of TCR-Vδ1 chain and TCR-V2δ chain on the cellular membrane surface of iγδTC. The horizontal axis and the vertical axis respectively show the expression of TCR-Vδ1 chain (Vdelta1) and TCR-Vδ2 chain (Vdelta2) .
Fig. 5 shows expression of CD3 and γδTCR molecule on the cellular membrane surface of Vγ9Vδ2-iTC. The horizontal axis and the vertical axis in the left Figure respectively show the expression of CD3 and pan-γδTCR. The horizontal axis and the vertical axis in the right Figure respectively show the expression of TCR-Vδ2 chain and TCR-Vy9 chain.
Fig. 6 shows expression of CD3 and γδTCR molecule on the cellular membrane surface of Vγ9Vβ2-iHTC. The horizontal axis and the vertical axis in the left Figure respectively show the expression of CD3 and pan-γδTCR. The horizontal axis and the vertical axis in the right Figure respectively show the expression of CD3 and TCR-Vy9 chain.
Fig. 7 shows cell-proliferation of iγδTC cells. The vertical axis shows cell number, and the horizontal axis shows days after the start of proliferation culture. Arrows show the day when stimulation was started with solid phased anti-CD3 agonist antibody/RetroNectin (registered trade mark) and anti-CD30 agonist antibody.
Fig. 8 shows cell-proliferation of Vγ9Vδ2-iTC. The vertical axis shows cell number, and the horizontal axis shows days after the start of proliferation culture. Arrows show the day when stimulation was started with solid phased anti-CD3 agonist antibody/RetroNectin (registered trade mark).
Fig. 9 shows a proliferation curve of CD19-iαβCARTC stimulated with solid phased anti-CD3 agonist antibody/RetroNectin (registered trade mark). The vertical axis shows cell number, and the horizontal axis shows days after the start of proliferation culture. Arrows show the day when stimulation was started with solid phased anti-CD3 agonist antibody/RetroNectin (registered trade mark).
Fig. 10 shows cytotoxic activity of CAR T cells prepared by introducing a gene encoding anti-CD19-CAR or a gene encoding anti-BCMA-CAR into iαβTC (CD19iαβCARTC(A) and BCMAiαβCARTC(B), respectively) and iαβTC against CD19 positive Raji cell (A) or BCMA positive H929 cell(B).
Fig. 11 shows cytotoxic activity of CD19-CD30-iαβCARTC against CD19 positive Raji cancer cells. The vertical axis shows the percentage of the killed cells after 2 hr, and the horizontal axis shows the mixing ratio of effector cell (CD19iαβCARTC) and the target cell (CD19 positive Raji cancer cell).
Fig. 12 shows antigen specific cytotoxic activity of CD19/IL15iγδCARTC. Black and white circles respectively indicate the cytotoxic activity against CD19 positive Raji cancer cell and CD19 negative CCRF-CEM cancer cell. The vertical axis shows the percentage of the remaining cells after 2 hr, and the horizontal axis shows the mixing ratio of effector cell (CD19/IL15iγδCARTC), and the target cell (CD19 positive Raji cancer cell or CD19 negative CCRF-CEM cancer cell).
Fig. 13 shows cell proliferation of CD19/IL15iVγ9Vδ2CARTC. The vertical axis shows cell number, and the horizontal axis shows days after the start of proliferation culture. Arrows show the day when stimulation with solid phased anti-CD3 agonist antibody/RetroNectin (registered trade mark) was started.
Fig. 14 shows the effect of CD19/IL15iγδCARTC on prolonging the survival days of human CD19-expressing Nalm6 tumor xenograft mouse.
Fig. 15 shows the effect of CD19/IL15iVγ9δ2CARTC on prolonging the survival days of human CD19-expressing Nalm6 tumor xenograft mouse.
Fig. 16 shows one embodiment of a T cell product providing system including a cell bank of T cell.
Fig. 17 shows one embodiment of a hardware constitution contained in a T cell product selection part.

### (DETAILED DESCRIPTION OF THE INVENTION)

### 1. master cell bank of T cell, working cell bank of T cell, and T cell product providing system

The present invention provides a master cell bank of T cell and/or a working cell bank of T cell. In the following, the term "cell bank of T cell" is sometimes used to encompass a master cell bank of T cell and a working cell bank of T cell. In another embodiment, the present invention provides a system for providing a T cell product, comprising a master cell bank of T cell and/or a working cell bank of T cell (hereinafter sometimes to be referred to as "the provision system of the present invention").

In the present specification, the "cell bank" means the same starting materials that have been characterised, which meet the quality standards and the like set by pharmaceutical authorities around the world in accordance with the guideline of ICH (International Council for Harmonisation of Technical Requirements for Pharmaceuticals for Human Use) (ICH guideline Q5D). Generally, a cell bank is constructed by freezing cells filled in a suitable container. However, it is distinguished in the above-mentioned points from a frozen stock obtained by simply freezing cells. Where necessary, the cell bank is tested for quality evaluation in addition to the characterization. The method for constructing the cell bank and the test items for characterisation and quality evaluation of the constructed cell bank vary depending on the biological properties (e.g., auxotrophy), culture history, and test feasibility of the cells to be the subject of the cell bank, and are appropriately determined by those of ordinary skill in the art, or based on the content of discussions between those of ordinary skill in the art and regulatory authorities, and the like. Information relating to the construction method thereof and the results of characterisation and quality evaluation are presented to the pharmaceutical authorities when applying for the manufacturing and marketing approval in each country. The "master cell bank" means a seed stock to be the source of all production cell seeds, grown under certain culture conditions through a minimum number of passages, and dispensed into multiple ampoules. When the cell is T cell, the bank is called "master cell bank of T cell". The "working cell bank" means cells obtained by pooling one or more master cell banks, further cultured under conditions confirmed to be sufficiently stable, and dispensed into multiple ampoules. When the cell is T cell, the bank is called "working cell bank of T cell" (in the present specification, master cell bank of T cell and working cell bank of T cell are sometimes referred to as "cell bank of T cell"). According to ICH Q5D, the tests of characterization are generally conducted on the master cell bank, and a part of tests of characterization is generally conducted on each working cell bank.

The tests of characterization mainly evaluate the absence of contamination with an exogenous infectious factor. In particular, since contamination with exogenous virus can lead to serious consequences in clinical use, it is evaluated according to ICH Q5A(R1). Other tests include identity test to detect cross contamination with other cell line. In addition, karyotype analysis and tumorigenicity test may also be performed.

The test items of quality evaluation include confirmation test using appropriate cell phenotype as T cell, purity test, production process-derived impurity test, and quantitative tests such as cell number, cell survival rate and the like.

The cell bank of T cell can be constructed by a person who produces and/or provides a T cell product, or the cell bank constructed by others may be introduced and used for producing a T cell product.

According to ICH Q5D, the two-step cell bank concept of constructing a working cell bank from a master cell bank is generally accepted as the most practical method for supplying cell substrates for continuous production of pharmaceutical products.

A working cell bank is derived from a master cell bank in one or more containers. The working cell bank can be regenerated from the master cell bank where necessary. It is stated in ICH Q5D that appropriate confirmation of the eligibility of a newly constructed working cell bank is necessary by performing characterisation and the like.

In one embodiment of the present invention, a master cell bank of T cell can be constructed by dispensing T cells into multiple storage containers (aseptic vial and the like), and freezing and stocking them. T cells in one container of this master cell bank of T cell can be thawed and appropriately subjected to a property analysis test. In addition, a working cell bank of T cell can be constructed by dispensing T cells from the same container or from another thawed container into multiple suitable containers, and freezing and stocking them.

In one embodiment of the present invention, a T cell product can be produced by thawing T cells in one or more containers of a cell bank of T cell, introducing nucleic acid containing an exogenous gene into the T cells, and expansion culturing them.

In the present invention, "stocking" means that a plurality of containers in which cells such as T cells are dispensed (allocated) are properly stored together, and storage conditions such as temperature, etc., balance, and the like are managed. The containers may be stored in one place or in multiple places.

In the present invention, the "T cell" means a CD3 positive cell. Examples of the T cell usable in the present invention include cytotoxic T lymphocyte (CTL) which is a CD8 positive cell, helper T cell which is a CD4 positive cell, regulatory T cell, effector T cell, γδT cell which is T cell receptor (TCR)y chain and TCR8 chain positive cell, and the like. In addition, a CD4/CD8 double positive cell is also encompassed in a T cell. The TCR in T cell may be a TCR due to the expression of an endogenous TCR gene or a TCR due to the expression of an exogenous TCR gene. The exogenous TCR may be a variant of the TCR described below. The T cells in the cell bank of T cell provided by the present invention are preferably cytotoxic T cells, more preferably CD8αβ positive cytotoxic T cells expressing CD8αβ. In the present specification, the "T cell product" means T cells filled in a storage container (aseptic vial, blood transfusion bag, etc.), or such storage container appropriately packaged or labeled. While the T cell product may or may not be frozen, it is preferably frozen during long-term storage from the aspect of the stability of the T cells. In the present specification, a frozen T cell product is sometimes particularly referred to as a "frozen T cell product". In the present specification, unless otherwise specified, the "T cell product" includes both a non-frozen T cell product and a frozen T cell product. As used herein, the "T cell product" includes both a product for clinical trials and a product for commercial use that are administered to mammalian test subjects including humans.

As described above, conventional methods for providing a T cell product by using a master cell bank or working cell bank of iPS cell require extremely high human, time and financial costs. Particularly, when two or more kinds of T cell products are provided, the cost and time of each of them are accumulated and the influence thereof becomes more serious. In contrast, since the providing system of the present invention uses a master cell bank or a working cell bank of T cell, the human, time and financial costs can be extremely reduced as compared to conventional techniques particularly when providing two or more kinds of T cell products. Therefore, while the kind of the T cell product to be provided by the providing system of the present invention may be one, particularly superior effects are obtained when two or more kinds of T cell products are provided.

In the present specification, being "positive" means that a protein or gene is expressed in an amount detectable by a method known in the art. Protein can be detected by an immunological assay using an antibody, such as ELISA, immunostaining, and flow cytometry. In the case of a protein that is intracellularly expressed and does not appear on the cell surface (e.g., transcription factor or subunit thereof, and the like), a reporter protein is expressed together with the protein, and the target protein can be detected by detecting the reporter protein. Gene can be detected by, for example, nucleic acid amplification method and/or nucleic acid detection method such as RT-PCR, biochip (e.g., microarray), RNAseq and the like.

In the present specification, being "negative" means that the expression level of the protein or gene is less than the lower limit of detection by all or any of the above-mentioned known methods. The lower limit of detection of protein or gene expression may vary depending on each method.

In the present specification, the "gene expression" encompasses both the synthesis of mRNA from a specific nucleotide sequence of the gene (also referred to as transcription or mRNA expression) and the synthesis of protein based on the information of the mRNA (also referred to as translation or protein expression). Unless otherwise specified, the "gene expression" or simple "expression" means expression of protein.

In the present specification, the "culture" refers to maintaining, proliferating (growing) and/or differentiating cells in an in vitro environment. "Culturing" means maintaining, proliferating (growing) and/or differentiating cells extra-tissue or ex-vivo, for example, in a cell culture plate, dish or flask.

T cells constituting a cell bank of T cell can be produced by inducing differentiation of stem cells with differentiation potency into T cells. Examples of the stem cell include pluripotent stem cell, multipotent stem cell, and the like. In the present specification, the "pluripotent stem cell" refers to a stem cell having the ability to differentiate into tissues and cells having various different forms and functions of a living body, and differentiate into cells of any lineage of three germ layers (endoderm, mesoderm, ectoderm). While the pluripotent stem cell is not particularly limited, for example, induced pluripotent stem cell (sometimes to be referred to as "iPS cell" in the present specification), embryonic stem cell (ES cell), embryonic stem cell derived from clone embryo obtained by nuclear transplantation (nuclear transfer Embryonic stem cell: ntES cell), pluripotent germ stem cell, embryonic germ cell (EG cell) and the like can be mentioned. In the present specification, the "multipotent stem cell" refers to a stem cell having the ability to differentiate into a plurality of limited number of lineages of cells. Examples of the "multipotent stem cell" include hematopoietic stem cell, pulp stem cell, stem cell derived from mouth cavity mucosa, hair follicle stem cell, somatic stem cell derived from culture fibroblast or myeloid stem cell, and the like. Preferred pluripotent stem cells are ES cell and iPS cell, and particularly preferred is iPS cell. When the above-mentioned pluripotent stem cell is ES cell or any cell derived from human embryo, the cell may be a cell produced by destroying the embryo or a cell prepared without destroying the embryo. Preferably, it is a cell prepared without destroying the embryo. The above-mentioned stem cell is preferably derived from a mammal (e.g., mouse, rat, hamster, guinea pig, dog, monkey, orangutan, chimpanzee, human), more preferably human. Therefore, the stem cell to be used in the present invention is most preferably human iPS cell.

The "induced pluripotent stem cell (iPS cell)" refers to a cell obtained by introducing a specific factor (nuclear reprogramming factor) into a mammalian somatic cell or an undifferentiated stem cell and reprogramming them. Currently, there are various types of the "induced pluripotent stem cell (iPS cell)", and iPS cell established by Yamanaka, et al. by introducing 4 factors of Oct3/4, Sox2, Klf4, c-Myc into mouse fibroblast (Takahashi K, Yamanaka S., Cell, (2006) 126: 663-676), iPS cell derived from a human cell established by introducing similar 4 factors into human fibroblast (Takahashi K, Yamanaka S., et al., Cell, (2007) 131: 861-872.), Nanog-iPS cell established by selecting with the expression of Nanog as an index after introduction of the above-mentioned 4 factors (Okita, K., Ichisaka, T., and Yamanaka, S. (2007). Nature 448, 313-317.), iPS cell produced by a method free of c-Myc (Nakagawa M, Yamanaka S., et al., Nature Biotechnology, (2008) 26, 101 - 106), and iPS cell established by introducing 6 factors by a virus-free method (Okita K et al., Nat. Methods 2011 May; 8(5):409-12, Okita K et al., Stem Cells. 31(3):458-66.) can also be used. In addition, induced pluripotent stem cell established by Thomson et al. by introducing 4 factors of OCT3/4, SOX2, NANOG, and LIN28 (Yu J., Thomson JA. et al., Science (2007) 318: 1917-1920.), induced pluripotent stem cell produced by Daley et al. (Park IH, Daley GQ. et al., Nature (2007) 451: 141-146), induced pluripotent stem cell produced by et al. (JP-A-2008-307007) and the like can also be used.

In addition, any of the induced pluripotent stem cells known in the art that are described in published papers (e.g., Shi Y., Ding S., et al., Cell Stem Cell, (2008) Vol3, Issue 5,568-574; Kim JB., Scholer HR., et al., Nature, (2008) 454, 646-650; Huangfu D., Melton, DA., et al., Nature Biotechnology, (2008) 26, No 7, 795-797), or patents (e.g., JP-A-2008-307007, JP-A-2008-283972, US2008-2336610, US2009-047263, WO2007/069666, WO2008/118220, WO2008/124133, WO2008/151058, WO2009/006930, WO2009/006997, WO2009/007852) can be used. As the induced pluripotent stem cell line, various iPS cell lines established by NIH, RIKEN, Kyoto University and the like can be used. Examples of the human iPS cell line include HiPS-RIKEN-1A strain, HiPS-RIKEN-2A strain, HiPS-RIKEN-12A strain, Nips-B2 strain of RIKEN, 253G1 strain, 201B7 strain, 409B2 strain, 454E2 strain, 606A1 strain, 610B1 strain, 648A1 strain, iPS cell stock for regenerative medicine (e.g., Ff-I01s04 strain, QHJI strain, etc.) of Kyoto University, and the like.

ES cell is a stem cell that is pluripotent and proliferative by self-replication and established from the inner cell mass of early mammalian embryos (e.g., blastocyst) such as human, mouse and the like. ES cell was discovered in mice in 1981 (M.J. Evans and M.H. Kaufman (1981), Nature 292:154-156), and ES cell line was subsequently established in primates such as human, monkey and the like (J.A. Thomson et al., (1998), Science 282:1145-1147; J.A. Thomson et al., (1995), Proc. Natl. Acad. Sci. USA, 92:7844-7848; J.A. Thomson et al., (1996), Biol. Reprod., 55:254-259; J.A. Thomson and V.S. Marshall (1998), Curr. Top. Dev. Biol., 38:133-165). ES cell can be established by removing the inner cell mass from the blastocyst of the fertilized egg of the target animal and culturing the inner cell mass on a fibroblast feeder. The methods for establishing and maintaining ES cells of human and monkey are described in, for example, USP5,843,780; Thomson JA, et al., (1995), Proc Natl. Acad. Sci. U S A. 92:7844-7848; Thomson JA, et al., (1998), Science. 282:1145-1147; Suemori H. et al., (2006), Biochem. Biophys. Res. Commun., 345:926-932; Ueno M. et al., (2006), Proc. Natl. Acad. Sci. USA, 103:9554-9559; Suemori H. et al., (2001), Dev. Dyn., 222:273-279; Kawasaki H. et al., (2002), Proc. Natl. Acad. Sci. USA, 99:1580-1585; Klimanskaya I. et al., (2006), Nature. 444:481-485 and the like. Alternatively, ES cell can also be established using only single blastomeres in the cleavage stage before blastocyst stage (Chung Y. et al., (2008), Cell Stem Cell 2: 113-117), or can also be established using embryos that have ceased development (Zhang X. et al., (2006), Stem Cells 24: 2669-2676.). As the "ES cell", various mouse ES cell lines established by inGenious targeting laboratory, RIKEN (Inst. of Physical and Chemical Research) and the like can be used as the mouse ES cell, and various human ES cell lines established by University of Wisconsin, NIH, RIKEN, Kyoto University, National Center for Child Health and Development and Cellartis and the like can be used as the human ES cell. For example, as the human ES cell line, CHB-1 - CHB-12 strains, RUES1 strain, RUES2 strain, HUES1 - HUES28 strains and the like provided by ESI Bio, H1 strain, H9 strain and the like provided by WiCell Research, KhES-1 strain, KhES-2 strain, KhES-3 strain, KhES-4 strain, KhES-5 strain, SSES1 strain, SSES2 strain, SSES3 strain and the like provided by RIKEN can be used.

The nt ES cell is an ES cell derived from cloned embryo produced by the nuclear transfer technology and has almost the same properties as ES cells derived from fertilized eggs (Wakayama T. et al.,(2001), Science, 292:740-743; S. Wakayama et al.,(2005), Biol. Reprod., 72:932-936; Byrne J. et al.,(2007), Nature, 450:497-502). That is, an ES cell established from the inner cell mass of blastocyst derived from a cloned embryo obtained by substituting the nucleus of an unfertilized egg with the nucleus of somatic cell is the nt ES (nuclear transfer ES) cell. For the production of the nt ES cell, a combination of nuclear transfer technique (Cibelli J.B. et al., (1998), Nature Biotechnol., 16:642-646) and ES cell production technique (above) is used (Kiyoka Wakayama et al. (2008), Experimental Medicine, Vol. 26, No. 5 (Special Edition), pp. 47-52). In the nuclear transfer, reprogramming is performed by injecting the nucleus of somatic cell into an enucleated unfertilized egg of mammal and culturing same for several hours.

The pluripotency germ stem cell is a pluripotent stem cell derived from a germ stem cell (GS cell). Similar to ES cells, this cell can induce differentiation into cells of various lineages, and has properties such as the ability to produce a chimeric mouse when transplanted into mouse blastocyst, and the like (Kanatsu-Shinohara M. et al., (2003) Biol. Reprod., 69:612-616; Shinohara K. et al., (2004), Cell, 119:1001-1012). The cell can self-replicate in a culture medium containing a glial cell line-derived neurotrophic factor (GDNF), and can produce a germ stem cell by repeated passage under the same culture conditions as those for ES cell (Masanori Takebayashi et al. (2008), Experimental Medicine, Vol. 26, No. 5 (Special Edition), pp. 41-46, YODOSHA CO., LTD. (Tokyo, Japan)).

The EG cell is a cell with pluripotency similar to that of ES cells, and is established from an embryonic primordial germ cell. It can be established by culturing primordial germ cells in the presence of a substance such as LIF, bFGF, stem cell factor, and the like (Matsui Y. et al., (1992), Cell, 70:841-847; J.L. Resnick et al., (1992), Nature, 359:550-551).

### 1-1. Differentiation induction into T cell

For differentiation induction of stem cell into T cell, a known method can be used without any particularly limitation as long as the differentiation into T cell can be performed. When a pluripotent stem cell is used as the stem cell, the above-mentioned differentiation induction into T cell may include, for example, (1) a process for differentiating pluripotent stem cells into hematopoietic progenitor cells, and (2) a process for differentiating the hematopoietic progenitor cells into T cells.

### (1) Process for differentiating induced pluripotent stem cells into hematopoietic progenitor cells

In the present specification, the "hematopoietic progenitor cell (HPC)" means CD34 positive cell, preferably, CD34/CD43 double positive (DP) cell. In the present invention, hematopoietic progenitor cell and hematopoietic stem cell are not distinguished and show the same cell unless particularly indicated.

The method of differentiating pluripotent stem cells into hematopoietic progenitor cells is not particularly limited as long as it can cause differentiation into hematopoietic progenitor cells. Examples thereof include a method including culturing pluripotent stem cells in a medium for induction of hematopoietic progenitor cells, as described in, for example, WO 2013/075222, WO 2016/076415 and Liu S. et al., Cytotherapy, 17 (2015); 344-358 and the like.

In the present invention, a medium used for induction into hematopoietic progenitor cells is not particularly limited. A medium used for culturing animal cells can be prepared into a basal medium. Examples of the basal medium include, but are not limited to, Dulbecco's Medium (e.g., IMDM), Eagle's medium (e.g., DMEM, EMEM, BME, MEM, αMEM), Ham's medium (e.g., F10 medium, F12 medium), RPMI medium (e.g., RPMI-1640 medium, RPMI-1630 medium), MCDB medium (e.g., MCDB104, 107, 131, 151, 153 medium), Fischer's medium, 199 medium, culture medium for primate ES cell (culture medium for primate ES/iPS cell, Reprocell), medium for mouse ES cell (TX-WES culture medium, Thromb-X), serum-free medium (mTeSR, Stemcell Technologies), ReproFF, StemSpan (registered trade mark) SFEM, StemSpan (registered trade mark) H3000, StemlineII, ESF-B medium, ESF-C medium, CSTI-7 medium, Neurobasal medium (Thermo Fisher Scientific), StemPro-34 medium, StemFit (registered trade mark) (e.g., StemFit AK03N, StemFit AK02N) and the like. Furthermore, these media can be mixed as necessary and used and, for example, DMEM/F12 medium and the like can be mentioned.

Where necessary, the basal medium may contain a medium additive and the like. Examples of the medium additive include serum, Vitamin C (e.g., ascorbic acid), albumin, insulin, transferrin, selenium compound (e.g., sodium selenite), fatty acid, trace elements, 2-mercaptoethanol, thioglycerol (e.g., α-monothioglycerol (MTG)), lipids, amino acids, L-glutamine, L-alanyl-L-glutamine (e.g., Glutamax (registered trade mark)), non-essential amino acids, vitamins, growth factors, low-molecular-weight compounds, antibiotics (e.g., penicillin, streptomycin), antioxidants, pyruvic acid, buffers, inorganic salts, cytokines, and the like.

In the present invention, "Vitamin C" means L-ascorbic acid and derivatives thereof, and "L-ascorbic acid derivative" means derivatives that become vitamin C by enzymatic reaction in the living body. Examples of the derivatives of L-ascorbic acid include vitamin C phosphate (e.g., ascorbic acid 2-phosphate), ascorbic acid glucoside, ascorbyl ethyl, vitamin C ester, ascorbyl tetrahexyldecanoate, ascorbyl stearate, and ascorbyl 2-phosphate 6-palmitate. Preferred is vitamin C phosphate. Examples of the vitamin C phosphate (e.g., ascorbic acid 2-phosphate) include salts of L-ascorbic acid phosphate such as L-ascorbic acid phosphate Na and L-ascorbic acid phosphate Mg.

When Vitamin C is used, the Vitamin C is preferably added (supplied) every four days, every three days, every two days, or every day. Vitamin C is more preferably added every day. In one embodiment, Vitamin C is preferably added to the medium at an amount corresponding to 5 ng/ml to 500 ng/ml (e.g., an amount corresponding to 5 ng/ml, 10 ng/ml, 25 ng/ml, 50 ng/ml, 100 ng/ml, 200 ng/ml, 300 ng/ml, 400 ng/ml, or 500 ng/ml). In another embodiment, Vitamin C is added to the culture medium at an amount corresponding to 5 µg/ml - 500 µg/ml (e.g., an amount corresponding to 5 µg/ml, 10 µg/ml, 25 µg/ml, 50 µg/ml, 100 µg/ml, 200 µg/ml, 300 µg/ml, 400 µg/ml, 500 µg/ml).

The medium to be used in process (1) may be further supplemented with at least one kind of cytokine selected from the group consisting of BMP4 (Bone morphogenetic protein 4), VEGF (vascular endothelial growth factor), SCF (Stem cell factor), TPO (thrombopoietin), FLT-3L (Flt3 Ligand) and bFGF (basic fibroblast growth factor). It is more preferably a culture supplemented with BMP4, VEGF and bFGF, and further preferably a culture supplemented with BMP4, VEGF, SCF and bFGF.

When cytokine is used, its concentration in the medium may be, for example, 5 ng/ml - 500 ng/ml for BMP4, 5 ng/ml - 500 ng/ml for VEGF, 5 ng/ml - 100 ng/ml for SCF, 1 ng/ml - 100 ng/ml for TPO, 1 ng/ml - 100 ng/ml for FLT-3L, and 5 ng/ml - 500 ng/ml for bFGF.

The aforementioned medium may be supplemented with a TGFβ inhibitor. The TGFβ inhibitor is a small molecule inhibitor that interferes with the signal transduction of TGFβ family and includes, for example, SB431542, SB202190 (both R.K. Lindemann et al., Mol. Cancer 2:20 (2003)), SB505124 (GlaxoSmithKline), NPC30345, SD093, SD908, SD208 (Scios), LY2109761, LY364947, LY580276 (Lilly Research Laboratories) and the like. For example, when the TGFβ inhibitor is SB431542, its concentration in the medium is preferably 0.5 µM - 100 µM.

The induced pluripotent stem cells may be cultured by adherent culture or suspension culture. In cases of adherent culture, the culturing may be carried out in a culture vessel coated with an extracellular matrix component, and/or may be co-cultured with feeder cells. While the feeder cell is not particularly limited, for example, fibroblast (mouse embryo fibroblast (MEF), mouse fibroblast (STO) and the like) can be mentioned. Feeder cells are preferably inactivated by a method known per se, for example, radiation (gamma-ray and the like) irradiation, treatment with anti-cancer agent (mitomycin C and the like) and the like. As the extracellular matrix component, fibrous proteins such as Matrigel (Niwa A, et al., PLoS One.6(7): e22261, 2011), gelatin, collagen, elastin and the like, glucosaminoglycan and proteoglycan such as hyaluronic acid, chondroitin sulfate and the like, cell adhesion proteins such as fibronectin, vitronectin, laminin and the like, and the like can be mentioned.

Suspension culture means culturing cells in a state of non-adhesion to a culture container and is not particularly limited. To improve adhesiveness to the cells, a culture container free of an artificial treatment (e.g., coating treatment with extracellular matrix and the like), or a culture container subjected to a treatment for artificially suppressing adhesion (e.g., coating treatment with polyhydroxyethyl methacrylic acid (poly-HEMA) or non-ionic surface-active polyol (Pluronic F-127 etc.)) can be used. In the suspension culture, embryoid (EB) is preferably formed and cultured.

In the present invention, hematopoietic progenitor cell can also be prepared from a sac-like structure (to be also referred to as ES-sac or iPS-sac) obtained by culturing pluripotent stem cells. As used herein, the "sac-like structure" is a pluripotent stem cell-derived three-dimensional saccular (with spaces inside) structure, which is formed by an endothelial cell population and the like and contains hematopoietic progenitor cells in the inside thereof.

The temperature conditions are not particularly limited. The temperature is, for example, about 37°C to about 42°C, preferably about 37°C to about 39°C. The culture period may be appropriately determined by those skilled in the art by monitoring of the number of hematopoietic progenitor cells and/or the like. The number of days of the culture is not limited as long as hematopoietic progenitor cells can be obtained. Examples of the culture period include at least 6 days, not less than 7 days, not less than 8 days, not less than 9 days, not less than 10 days, not less than 11 days, not less than 12 days, not less than 13 days, and not less than 14 days. The culture period is preferably 14 days. While a longer culture period generally does not pose a problem in the production of hematopoietic progenitor cells, it is preferably not more than 35 days, more preferably not more than 21 days. The culture may be carried out under low-oxygen conditions, and the low-oxygen condition in the present invention means, for example, oxygen concentration of 15%, 10%, 9%, 8%, 7%, 6%, 5% or lower than these.

### (2) Step of differentiating hematopoietic progenitor cells into T cells

A method for differentiating hematopoietic progenitor cells into T cells is not particularly limited as long as it can differentiate hematopoietic progenitor cells into T cells. Examples thereof include a method for culturing hematopoietic progenitor cells under the same culture conditions as those in a method of inducing T cells from hematopoietic progenitor cells, as described in WO 2016/076415, WO 2017/221975 and the like.

In the present invention, a medium for inducing differentiation into T cell is not particularly limited, and a medium used for culturing animal cells can be prepared into a basal medium. Where necessary, the basal medium may contain a medium additive and the like. Examples of the basal medium and the medium additive include those similar to the basal medium and the medium additive used in the above-mentioned process (1).

When Vitamin C is used in process (2), Vitamin C may be the same as that described in process (1) and can be added similarly. In one embodiment, the concentration of Vitamin C in the medium or a culture medium is preferably 5 µg/ml - 200 µg/ml. In another embodiment, vitamin C is added to the culture medium at an amount corresponding to 5 µg/ml - 500 µg/ml (e.g., amount corresponding to 5 µg/ml, 10 µg/ml, 25 µg/ml, 50 µg/ml, 100 µg/ml, 200 µg/ml, 300 µg/ml, 400 µg/ml, 500 µg/ml).

In process (2), p38 inhibitor and/or SDF-1 (Stromal cell-derived factor 1) are/is preferable. In the present invention, the "p38 inhibitor" means a substance that inhibits the functions of p38 protein (p38 MAP kinase). Examples thereof include, but are not limited to, chemical inhibitor of p38, dominant-negative mutant of p38 or nucleic acid encoding same and the like.

Examples of the chemical inhibitor of p38 to be used in the present invention include, but are not limited to, SB203580 (4-(4-fluorophenyl)-2-(4-methylsulfonylphenyl)-5-(4-pyridyl)-1H-imidazole), and a derivative thereof, SB202190 (4-(4-fluorophenyl)-2-(4-hydroxyphenyl)-5-(4-pyridyl)-1H-imidazole) and a derivative thereof, SB239063 (trans-4-[4-(4-fluorophenyl)-5-(2-methoxy-4-pyrimidinyl)-1H-imidazol-1-yl]cyclohexanol) and a derivative thereof, SB220025 and a derivative thereof, PD169316, RPR200765A, AMG-548, BIRB-796, SClO-469, SCIO-323, VX-702 and FR167653. These compounds are commercially available and, for example, SB203580, SB202190, SC239063, SB220025 and PD169316 are available from Calbiochem, and SClO-469 and SCIO-323 are available from Scios and the like. the chemical inhibitor of p38 is preferably SB203580 (4-(4-fluorophenyl)-2-(4-methylsulfonylphenyl)-5-(4-pyridyl)-1H-imidazole), or a derivative thereof.

Examples of the dominant-negative mutant of p38 to be used in the present invention include p38T180A obtained by point mutation of the 180-position threonine located in the DNA binding region of p38 to alanine, p38Y182F obtained by point mutation of the 182-position tyrosine of p38 in human and mouse to phenylalanine and the like. The p38 inhibitor is contained in a medium at, for example, about 1 µM - about 50 µM. When SB203580 is used as the P38 inhibitor, it may be contained in a medium at 1 µM - 50 µM, 5 µM - 30 µM, 10 µM - 20 µM.

SDF-1 to be used in the present invention may be not only SDF-1α or a mature form thereof, but also an isoform such as SDF-1β, SDF-1γ, SDF-1δ, SDF-1ε, SDF-1φ and the like or a mature form thereof, or a mixture of these at any ratio or the like. Preferably, SDF-1α is used. SDF-1 is sometimes referred to as CXCL-12 or PBSF.

In the present invention, one or several amino acids in the amino acid sequence of SDF-1 may be deleted, substituted, inserted and/or added as long as it has the activity as the chemokine (SDF-1 with such deletion, substitution, insertion and/or addition of amino acid is to be also referred to as "SDF-1 mutant"). Similarly, sugar chain may be deleted, substituted, inserted and/or added in SDF-1 or SDF-1 mutant. Examples of the mutant of the above-mentioned SDF-1 include those maintaining at least 4 cysteine residues (Cys30, Cys32, Cys55 and Cys71 in human SDF-1α) and having not less than 90% identity with amino acid sequence of a natural substance, though the amino acid mutation is not limited thereto. SDF-1 may be obtained from a mammal, for example, human or non-human mammal such as monkey, sheep, bovine, horse, swine, dog, cat, rabbit, rat, mouse and the like. For example, the protein registered as GenBank accession number:NP_954637 can be used as human SDF-1α, and the protein registered as GenBank accession number:NP_000600 can be used as SDF-1β.

SDF-1 may be commercially available, purified from nature, or produced by peptide synthesis or genetic engineering techniques. SDF-1 is contained in a medium within the range of, for example, about 10 ng/ml to about 100 ng/ml. In addition, SDF-1 alternative having an SDF-1-like activity can also be used instead of SDF-1. Examples of such SDF-1 alternative include CXCR4 agonist, and a low-molecular-weight compound having a CXCR4 agonist activity and the like may be added to the medium instead of SDF-1.

The culture medium used in process (2) may be further supplemented with at least one kind, preferably all, of cytokine selected from the group consisting of SCF, TPO (thrombopoietin), FLT-3L and IL-7. The concentration of these is, for example, 10 ng/ml to 100 ng/ml for SCF, 10 ng/ml to 200 ng/ml for TPO, 1 ng/ml to 100 ng/ml for IL-7, and 1 ng/ml to 100 ng/ml for FLT-3L.

In process (2), the hematopoietic progenitor cells may be cultured by adherent culture or suspension culture. In cases of adherent culture, a coated culture vessel may be used, and/or the hematopoietic progenitor cells may be co-cultured with feeder cells and/or the like. Examples of the feeder cells for the co-culture include a bone-marrow stromal cell line, OP9 cells (available from Riken BioResource Center). The OP9 cell is preferably OP9-DL4 cell or OP9-DL1 cell, which constantly expresses DLL4 or DLL1 (e.g., Holmes R I and Zuniga-Pflucker J C. Cold Spring Harb Protoc. 2009(2)). In the present invention, in cases where OP9 cells are used as the feeder cells, DLL1, or a separately-prepared DLL4 or DLL1, or a fusion protein of DLL4 or DLL1, and Fc or the like, may be added to the medium to perform the co-culture. When feeder cells are used, the feeder cells are preferably appropriately replaced during the culture. The replacement of the feeder cells may be carried out by transferring the subject cells that are being cultured onto feeder cells that are preliminarily plated. The replacement may be carried out every five days, every four days, every three days, or every two days. When hematopoietic progenitor cells are obtained by suspension culture of embryoid, it is preferable to perform adhesion culture after dissociation into single cells. While the cells may be co-cultured with feeder cells, culturing is preferably carried out without using feeder cells.

In the case of adhesion culture and when a culture container is coated, examples of the coating agent include Matrigel (Niwa A, et al. PLos One, 6(7):e22261, 2011)), collagen, gelatin, laminin, heparan sulfuric acid proteoglycan, RetroNectin (registered trade mark), fusion protein of DLL4 or DLL1, or DLL4 or DLL1, and Fc region of antibody (hereinafter sometimes referred to as Fc) and the like (e.g., DLL4/Fc chimera), entactin, and/or combination of these, and a combination of RetroNectin and fusion protein of DLL4 and Fc etc. is preferable.

In process (2), the culture temperature conditions are not limited. The temperature is, for example, about 37°C to about 42°C, preferably about 37°C to about 39°C. The culture period may be appropriately determined by those skilled in the art by monitoring of the number of T cells and the like. The number of days of the culture is not limited as long as T cells can be obtained. Examples of the culture period typically include at least not less than 10 days, not less than 12 days, not less than 14 days, not less than 16 days, not less than 18 days, and not less than 20 days. The culture period is preferably 21 days. In addition, not more than 90 days is preferable, and not more than 42 days is more preferable.

The cell population obtained by the above step includes T cells. Process (2) may further contain the following process (3).

### (3) Process for condensing T cells

A method for condensing T cells is not particularly limited as long as T cells can be condensed. For example, a method of culturing T cells under the same culture conditions as those in a step of inducing CD8 positive T cells from CD4CD8 double positive T cells, as described in WO 2016/076415, WO 2017/221975 and the like can be mentioned.

In the present specification, "concentrating" refers to increasing the proportion of a particular constituent component in a composition such as a cell composition and the like, and "concentrated" when used to describe a cell composition such as a cell population means that the amount of a particular constituent component in the cell population has increased from that of the component in the cell population before being concentrated. For example, a composition such as cell population and the like can be concentrated for the target cell type. Thus, the proportion of the target cell type increase as compared to the proportion of the target cell present in the cell population before being concentrated. A cell population may also be concentrated for the target cell type by a cell selecting method or sorting method known in the art. The cell population may also be concentrated by a particular culture method, sorting, or a selecting process, described in the present specification. In a particular embodiment of the present invention, the cell population is concentrated by at least 10%, 20%, 30%, 40%, 50%, 60%, 70%, 80%, 85%, 90%, 95%, 97%, 98% or 99% of the target cell population by a method of concentrating the target cell population.

In the present specification, the "cell population" means two or more cells of the same type or different types. The "Cell population" also means a mass of cells of the same type or different types.

In the present invention, the medium used for concentrating T cells is not particularly limited, and a medium used for culturing animal cells can be prepared into a basal medium. Where necessary, the basal medium may contain a medium additive and the like. Examples of the basal medium and medium additive include those similar to the basal medium used in the above-mentioned process (1).

When Vitamin C is used in process (3), Vitamin C may be similar to those described in process (1) and can be added similarly. In one embodiment, the concentration of Vitamin C in the medium or culture medium is preferably 5 µg/ml - 200 µg/ml. In another embodiment, vitamin C is added in an amount corresponding to 5 µg/ml - 500 µg/ml in the culture medium (e.g., amount corresponding to 5 µg/ml, 10 µg/ml, 25 µg/ml, 50 µg/ml, 100 µg/ml, 200 µg/ml, 300 µg/ml, 400 µg/ml, 500 µg/ml).

When hormone is used in process (3), examples of the hormone include corticosteroid. Corticosteroid is glucocorticoid or a derivative thereof, and cortisone acetate, hydrocortisone, fludrocortisone acetate, predonisolone, triamcinolone, methylprednisolone, dexamethasone, betamethasone, and beclometasone dipropionate are recited as examples. Preferred is dexamethasone. When corticosteroid is dexamethasone, its concentration in the medium is 1 nM - 100 nM.

In process (3), the medium may contain a CD3/TCR complex agonist. The CD3/TCR complex agonist is not particularly limited as long as it is a molecule capable of transducing a signal from a CD3/TCR complex to a T cell by specifically binding to the CD3/TCR complex. Examples of the CD3/TCR complex agonist include CD3 agonist and/or TCR agonist. As the CD3 agonist, an anti-CD3 agonist antibody (to be also simply referred to as "anti-CD3 antibody") or a fragment bonded thereto can be mentioned, and as the TCR agonist, at least one selected from the group consisting of an anti-TCR agonist antibody (to be also simply referred to as "anti-TCR antibody") or a fragment bonded thereto, an MHC/antigen peptide complex or a multimer thereof, and an MHC/superantigen complex or a multimer thereof can be mentioned. When an anti-CD3 antibody is used, the anti-CD3 antibody includes both a polyclonal antibody and a monoclonal antibody, preferably a monoclonal antibody. The antibody may belong to any immunoglobulin class of IgG, IgA, IgM, IgD and IgE, preferably IgG. As the anti-CD3 antibody, an antibody produced from OKT3 clone (OKT3), an antibody (UCHT1) produced from UCHT1 clone and the like can be mentioned, preferably UCHT1. The concentration of anti-CD3 antibody in the medium is, for example, 10 ng/ml - 1000 ng/ml, preferably 50 ng/ml - 800 ng/ml, more preferably 250 ng/ml - 600 ng/ml. The above-mentioned CD3/TCR complex agonist may be commercially available, purified from nature, or produced by peptide synthesis or genetic engineering techniques or chemical synthesis methods. For example, OKT3 and UCHT1 can be purchased from ThermoFisher, GeneTex and the like.

When cytokine is used in process (3), IL-2 and IL-7 and the like can be mentioned as the cytokine. When cytokine is IL-2, the concentration thereof in the medium is 10 U/ml - 1000 U/mL, and when it is IL-7, the concentration thereof in the medium is 1 ng/ml - 1000 ng/mL.

In process (3), the culture temperature conditions are not particularly limited. The temperature is, for example, about 37°C to about 42°C, preferably about 37°C to about 39°C. The culture period can be appropriately determined by those skilled in the art by monitoring the number of T cells and the like. The number of days is not limited as long as cells can be concentrated. The culture period is, for example, not less than 1 day, not less than 2 days, not less than 3 days, not less than 4 days, not less than 5 days, preferably not less than 6 days. It is preferably not more than 28 days, more preferably not more than 14 days.

In T cells constituting a cell bank of T cell, the expression of endogenous gene may be appropriately regulated. Specifically, in the T cells constituting a cell bank of T cell, the expression of at least one kind (e.g., one kind, two kinds, three kinds, six kinds, nine kinds) of HLA (Human Leukocyte Antigen) gene is preferably suppressed or the HLA gene is preferably deleted to reduce rejection in allogeneic transplantation. Therefore, in one embodiment of the present invention, the provision system of the present invention contains T cell in which the expression of at least one kind of HLA gene is suppressed. In the present specification, the suppression of the expression of HLA gene also includes deletion of the gene.

As the above-mentioned HLA gene, specifically, one or more kinds of HLA genes selected from the group consisting of class I HLA (i.e., HLA-A, HLA-B, HLA-C, HLA-E, HLA-F and HLA-G) gene and class II HLA (i.e., HLA-DR, HLA-DQ and HLA-DP) gene can be mentioned. Alternatively, the gene expression important for the above-mentioned expression of the HLA gene and the presentation thereof on the cell surface may be suppressed. Examples of the gene include B2M gene encoding B2M, which is an important protein for presenting HLA class 1 HLA on the cell surface, and CIITA gene encoding CIITA, which is an important protein for the expression of class II HLA gene. On the other hand, since a HLA gene expression-suppressed cell is easily targeted by natural killer (NK) cells due to the suppression of HLA gene expression, it is also preferable to avoid being targeted by NK cell by overexpressing CD47 gene, or suppressing the expression of only two genes, HLA-A gene and HLA-B gene and maintaining the expression of HLA-C gene, HLA-E gene, HLA-F gene and HLA-G gene, which are important for avoiding NK cell attacks (in this case, the expression of class II HLA gene may or may not be suppressed, but it is preferable to suppress the gene expression in view of immunocompatibility., In a preferred embodiment, therefore, the combination of HLA genes whose expression is to be suppressed includes (i) combination of HLA-A gene and HLA-B gene, (ii) combination of HLA-A gene, HLA-B gene, HLA-DR gene, HLA-DQ gene and HLA-DP gene, and (iii) combination of HLA-A gene, HLA-B gene, HLA-C gene, HLA-E gene, HLA-F gene, HLA-G gene, HLA-DR gene, HLA-DQ gene and HLA-DP gene. HLA, which is a human major histocompatibility complex (MHC), has been described as an example in the above. Even when a non-human MHC is used, it is preferable to suppress expression of at least one kind of MCH gene in the same manner.

As a method for suppressing the expression of HLA gene, a known method can be appropriately used. For example, the expression of HLA gene can be knocked down by introducing HLA mRNA, B2M mRNA, and/or siRNA, shRNA, miRNA, antisense oligo nucleic acid, ribozyme that targets CIITA mRNA into cells at the stage of differentiation from pluripotent cells into T cells (e.g., pluripotent stem cell, hematopoietic progenitor cell (CD34+/CD43+), ProT cells (CD4-/CD8-), CD3+/CD4+/CD8+T cells, CD3+/CD4-/CD8+T cells, or other cells (e.g., CD3-/CD4+/CD8+ cell, etc.) etc.), or introducing a nucleic acid encoding the molecule into the cells by the below-mentioned method. Alternatively, HLA gene, B2M gene, and/or CIITA gene in the cells may be knocked out by genome editing (e.g., CRISPR system, TALEN, ZFN and the like).

In the T cells constituting a cell bank of T cell, it is preferable to suppress expression of the TCR chain by siRNA, or suppress expression of TCR in the T cells by introducing exogenous TCR known to be harmless for the test subject to be administered with the T cells. When the aforementioned method by siRNA is applied to this method, to avoid the effect of siRNA on exogenous TCR, it is preferable that the nucleotide sequence of the nucleic acid encoding TCR is a sequence (codon conversion type sequence) different from the nucleotide sequence corresponding to the RNA acted on by the siRNA suppressing the expression of the endogenous TCR chain. These methods are described in, for example, WO 2008/153029. The aforementioned base sequence can be produced by introducing a silent mutation into a nucleic acid encoding TCR obtained naturally or chemically synthesizing artificially designed nucleic acid. To avoid mispairing with the endogenous TCR chain, a part or all of the constant regions of the nucleic acid encoding the introduced TCR may also be substituted with a constant region derived from an animal other than the test subject.

In the T cells constituting a cell bank of T cell, moreover, a nucleic acid containing an exogenous gene may be introduced, or the exogenous gene product may be expressed.

The above-mentioned exogenous gene is not particularly limited, and an exogenous gene encoding protein, cytokine, chemokine and the like that can contribute to the regulation of the function of T cells can be used. Examples of the above-mentioned exogenous gene include a gene encoding a chimeric antigen receptor (CAR) (hereinafter to be also referred to as "CAR gene"), a gene encoding an exogenous T cell receptor (TCR) (hereinafter to be also referred to as "TCR gene") and the like. The above-mentioned CAR and TCR expressed from genes introduced into cells can recognize and bind to antigen and/or antigen-HLA complex. In the present specification, the nucleic acid encoding TCR is a nucleic acid containing a base sequence encoding one strand that forms TCR and a base sequence encoding the other strand.

The term "capable of binding" as used herein means "having an ability to bind" and refers to a capability to form a non-covalent complex with one or more other molecules. Various methods and assays to determine binding capability are known in the art. Binding is usually a binding with high affinity, wherein the binding affinity as measured in KD values preferably is less than 1 µM, more preferably less than 100 nM, even more preferably less than 10 nM, even more preferably less than 1 nM, even more preferably less than 100 pM, even more preferably less than 10 pM, even more preferably less than 1 pM. The term "KD" or "KD value" relates to the equilibrium dissociation constant as known in the art and indicates the intermolecular binding affinity. A smaller KD value shows higher binding affinity.

The TCR used in the present invention encompasses not only one in which α chain and β chain of TCR constitute a heterodimer (i.e., αβTCR), or γ chain and δ chain of TCR constitute a heterodimer (i.e., γδTCR), but also one in which they constitute a homodimer. Furthermore, one lacking a part of or whole constant region and one with recombination of an amino acid sequence may also be used.

The constant region of the above-mentioned TCR chain may be the constant region of the TCR chain of the cytotoxic T lymphocyte (CTL) clone, from which it is derived, wherein the region has been subjected to a predetermined modification. Examples of the modification include, but are not limited to, replacing particular amino acid residues in the constant region of the TCR of the CTL clone with cysteine residues, thereby enhancing efficiency of dimer expression by disulfide bond between TCR chains and the like.

The above-mentioned TCR may be a variant. In the following, unless otherwise specified, TCR includes variants thereof. Examples of the variant include variant of the T cell receptor developed by the present inventor and consisting of a combination of two polypeptides containing the constant region of a TCR chain selected from the group consisting of α chain, β chain, γ chain and δ chain and the like. TCR variant is characterized in that it does not include a complementarity determining region (CDR) of TCRα chain and β chain, or a complementarity determining region (CDR) (preferably a part or all of the variable region containing CDR) of the same kind of chain, and that it does not include a complementarity determining region (CDR) of T cell receptor chain from which the constant region derives, or a complementarity determining region (CDR) (preferably a part or all of the variable region containing CDR) of the same kind of chain. Accordingly, the above-mentioned variant does not include a natural type or artificial type (e.g., animal species from which constant region and variable region are derived are different) of αβTCR, yδTCR, or TCR variants in which amino acids are further added to TCR of these. For example, when a polypeptide corresponding to at least one of the chains of the variant of the present invention contains the constant region of the T cell receptor α chain, the polypeptide does not contain the complementarity determining region of the T cell receptor α chain, preferably a part or all of the variable region containing the complementarity determining region. However, it may contain TCR chain other than α chain and β chain, for example, CDR and variable region of γ chain and δ chain. Similarly, when a polypeptide corresponding to at least one of the chains of the above-mentioned variant contains the constant region of the T cell receptor β chain, the polypeptide does not contain the complementarity determining region of the T cell receptor β chain, preferably a part or all of the variable region containing the complementarity determining region. However, it may contain TCR chain other than α chain and β chain, for example, CDR and variable region of γ chain and δ chain. The same applies to γ chain and δ chain.

A membrane transfer signal peptide (hereinafter to be referred to as "signal peptide") may be added to the above-mentioned TCR. As the signal peptide, CD8, Immunoglobulin-H (IGH), CD4, membrane transfer signal peptide derived from a gene encoding various peptides having transmembrane domain and/or an amino acid sequence obtained by deletion, substitution, insertion and/or addition of one or several (e.g., 2, 3, 4, 5) amino acids in the amino acid sequence of the signal peptide, or a signal peptide composed of an amino acid sequence having identity with the amino acid sequence of the signal peptide can be used. When a signal peptide is added, the binding position and the number of signal peptides are not particularly limited.

In the present invention, the "chimeric antigen receptor (CAR)" means a fusion protein containing an antigen-binding domain, a transmembrane domain, and an intracellular signal transduction domain. The antigen-binding domain of CAR includes a short chain antibody (scFv) in which the light chain (VL) and heavy chain (VH) of the variable region of the antibody are linked in tandem via a spacer such as a linker (e.g., linker composed of G and S (GS linker)). The cells expressing CAR recognize the antigen in the scFV region and then transduce the recognition signal thereof into T cells through the intracellular signal transduction domain. Introduction of CAR into the cells makes it possible to impart specificity to the antigen of interest. In addition, since CAR can directly recognize antigen molecules without depending on HLA class I or class II, a high immune response can also be induced against cells with decreased HLA class I or class II gene expression. As the antigen targeted by the aforementioned CAR, the same antigens as the above-mentioned antigens targeted by the aforementioned TCR can be mentioned.

Examples of the transmembrane domain of CAR include, but are not limited to, transmembrane domains derived from one or more proteins selected from the group consisting of α chain, β chain or ζ chain of TCR, CD28, CD3ε chain, CD45, CD4, CD5, CD8, CD9, CD16, CD22, CD33, CD37, CD64, CD80, CD86, CD134, 4-1BB (CD137) and CD154 and the like. The transmembrane domain of the molecule from which the first intracellular signal transduction domain linked to the antigen binding domain is derived may be used. For example, when the molecule from which the first intracellular signal transduction domain linked to the antigen binding domain is derived is CD28, the transmembrane domain may also be derived from CD28. Alternatively, an artificially designed transmembrane domain may also be used.

Examples of the intracellular signal transduction domain of CAR include, but are not limited to, intracellular domains derived from one or more proteins selected from the group consisting of CD3ζ chain (TCRζ chain), FcRy chain, FcRβ chain, CD3γ chain, CD3δ chain, CD3ε chain, CD5, CD22, CD79a, CD79b and CD66d. Of these, an intracellular signal transduction domain derived from CD3ζ chain is preferable. The intracellular signal transduction domain may further contain an intracellular domain of a co-stimulatory molecule. Examples of the co-stimulatory molecule include intracellular domains of one or more kinds of proteins selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3 and CD83. The strength and duration of CAR activity can be controlled by selecting the type and number of co-stimulatory molecule to be bound (e.g., Mol Ther. 2009; 17:1453-1464.).

A spacer may be incorporated between the antigen-binding domain and transmembrane domain of CAR or between the intracellular transduction domain and transmembrane domain of CAR. As the spacer, a peptide generally consisting of not more than 300 amino acids, preferably 10 - 100 amino acids, most preferably 25 - 50 amino acids, can be used. Specific examples thereof include, but are not limited to, peptides containing a hinge region derived from IgG1, and CH2CH3 region of immunoglobulin and a part of CD3 and the like.

Examples of specific CAR include, but are not limited to, a first generation CAR in which scFv and CD3ζ chain are linked via a spacer, a second generation CAR in which a transmembrane domain derived from CD28 and an intracellular domain are incorporated between the scFV and CD3ζ chain of the first generation CAR to enhance the ability to activate T cells, and a third generation CAR in which the intracellular domain of a co-stimulatory molecule (4-1BB or OX40) different from CD28 is incorporated between the intracellular domain of CD28 and CD3ζ chain of the second generation.

More specific examples of CAR used in the present invention include a chimeric antigen receptor containing scFv recognizing CD19 as an antigen binding domain, transmembrane domain of CD8 as a transmembrane domain, intracellular domain derived from CD28 as an intracellular signal transduction domain, an intracellular domain derived from CD30, an intracellular domain derived from 4-1BB, and an intracellular domain derived from CD3ζ chain. The order of the above-mentioned intracellular domains contained in the intracellular signal transduction domain is not particularly limited and, for example, the order of the intracellular domain derived from CD28, the intracellular domain derived from CD30 or the intracellular domain derived from 4-1BB, and the intracellular domain derived from CD3ζ chain is adopted. More specifically, the chimeric antigen receptor in the present invention is composed of, for example, the amino acid sequence shown in SEQ ID NO: 4 or 5, or an amino acid sequence obtained by deletion, substitution, insertion and/or addition of one or two or more (preferably, about 1 - 100, preferably about 1 - 50, further preferably about 1 - 10, particularly preferably 1 - several (2, 3, 4 or 5)) amino acids in the amino acid sequence shown in SEQ ID NO: 4 or 5.

Examples of the intracellular domain derived from CD30 include an amino acid sequence obtained by deletion, substitution, insertion and/or addition of one or two or more (preferably, about 1 - 100, preferably about 1 - 50, further preferably about 1 - 10, particularly preferably 1 - several (2, 3, 4 or 5)) amino acids in the amino acid sequence shown in SEQ ID NO: 7. When the amino acid sequence is deleted, substituted, inserted and/or added as described above, the location of the deletion, substitution, insertion and/or addition is not particularly limited as long as the function of the intracellular domain of CD30 is maintained.

Examples of the antigen targeted by the above-mentioned TCR and CAR include, but are not limited to, tumor antigens. The tumor antigen may be a tumor-specific antigen (TSA) or a tumor-associated antigen (TAA). Specific examples of such tumor antigen include one or more kinds of antigens selected from the group consisting of differentiated antigens such as MART-1/MelanA (MART-I), gp100 (Pmel 17), tyrosinase, TRP-1, TRP-2 and the like, tumor-specific multilineage antigens such as WT1, Glypican-3, MAGE-1, MAGE-3, BAGE, GAGE-1, GAGE-2, p15 and the like, fetal antigens such as CEA and the like, overexpressed tumor genes or mutated tumor suppressive genes such as p53, Ras, HER-2/neu and the like, unique tumor antigens caused by chromosome translocation such as BCR-ABL, E2A-PRL, H4-RET, IGH-IGK, MYL-RAR and the like, and virus antigens such as Epstein Barr virus antigen EBVA, human papilloma virus (HPV) antigens E6 and E7 and the like. As other tumor antigens, TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, β-catenin, CDK4, Mum-1, p 15, p 16, 43-9F, 5T4, 791Tgp72, α-fetoprotein, β-HCG, BCA225, BTAA, CA 125, CA 15-3/CA 27.29/BCAA, CA 195, CA 242, CA-50, CAM43, CD68/P1, CO-029, FGF-5, G250, Ga733/EpCAM, HTgp-175, M344, MA-50, MG7-Ag, MOV18, NB/70K, NY-CO-1, RCAS1, SDCCAG16, TA-90/Mac-2 binding protein/cyclophilin C-associated protein, TAAL6, TAG72, TLP and TPS can be mentioned.

It can be confirmed by a known method that the above-mentioned TCR or CAR (hereinafter sometimes to be abbreviated as "TCR etc.") specifically recognizes antigen and can bind thereto. A suitable method includes, for example, dextramer assay, ELISPOT assay and the like. By performing the ELISPOT assay, it can be confirmed that T cells expressing TCR on the cell surface recognize the target antigen by the TCR etc. and the signal thereof has been transmitted into the cells.

Previously, the present inventors found that cytotoxic activity increases in cells expressing a fusion protein containing IL-15 and IL-15Rα (hereinafter sometimes to be abbreviated as "IL-15/IL-15Rα") together with the above-mentioned CAR, as compared to the cells expressing CAR alone. Therefore, from the aspect of cytotoxic activity, T cells constituting a cell bank of T cell preferably express IL-15/IL-15Rα, and more preferably express the above-mentioned CAR. To obtain T cells expressing IL-15/IL-15Rα, the IL-15/IL-15Rα gene is introduced into cells at the stage of differentiation from pluripotent cells into T cells (e.g., pluripotent stem cell, hematopoietic progenitor cell (CD34+/CD43+), ProT cells (CD4-/CD8-), CD3+/CD4+/CD8+T cells, CD3+/CD4-/CD8+T cells, or other cells (e.g., CD3-/CD4+/CD8+ cell, etc.) etc.). It is preferable to introduce IL-15/IL-15Rα into the cell in the form of a nucleic acid encoding same. The introduction can be performed in the same manner as in the above-mentioned nucleic acid introduction step.

In the IL-15 signal transduction system, IL-15Rα expressed on antigen-presenting cells generally binds to IL-15 and IL-15 is presented to IL-15 receptor consisting of a common γ chain (yc) with IL-15Rβ on CD8-positive and CD4-negative cell (trans-presentation), whereby the cytotoxic activity of CD8 positive CD4 negative cell is maintained. Therefore, when the T cell expressing IL-15/IL-15Rα is CD8 positive CD4 negative, the cell can transmit the IL-15 signal into its own cell via the IL-15 receptor. Alternatively, the T cell expressing IL-15/IL-15Rα can transmit the IL-15 signal into other CD8 positive CD4 negative cells via the IL-15 receptor. As described above, since IL-15/IL-15Rα can maintain cytotoxic activity of CD8 positive CD4 negative cell, it is expected to show a continuous cytotoxic effect on cells targeted by CAR.

IL-15/IL-15Rα may be a transmembrane type protein or a secretor protein. It is known that, in IL-15Rα, the IL-15 binding domain of 1-65 amino acids from the N-terminal of the mature protein is the region responsible for binding to IL-15 (Wei X. et al., J. Immunol., 167: 277-282, 2001). Therefore, the transmembrane type protein may be a protein that retains the IL-15 binding domain and retains the transmembrane domain of IL-15Rα. On the other hand, the secretor protein may be a protein that maintains the IL-15 binding domain and lacks the transmembrane domain of IL-15Rα (e.g., protein consisting of 1-65 amino acid residues, 1-85 amino acid residues, or 1-182 amino acid residues of IL-15Rα, peptide containing an amino acid sequence not less than 85% (e.g., 90%, 95%, 97%, 98%, 99%) identical with the amino acid sequence, and the like).

A spacer may be incorporated between IL-15 and IL-15Rα of IL-15/IL-15Rα. As the spacer, a peptide generally consisting of not more than 300 amino acids, preferably 10 - 100 amino acids, most preferably 20 - 50 amino acids, can be used. Specific examples thereof include, but are not limited to, the aforementioned GS linker and the like.

IL-15/IL-15Rα is not particularly limited as long as it is a protein in which IL-15 and IL-15Rα are fused, and specific examples thereof include a peptide consisting of SEQ ID NO: 7. While IL-15/IL-15R is not particularly limited as long as it can bind to the IL-15 receptor and transmit the IL-15 signal into the cell, for example, a peptide containing an amino acid sequence having a homology or identity of not less than about 90%, preferably not less than about 95%, more preferably not less than about 97%, particularly preferably not less than about 98%, most preferably not less than about 99%, with the amino acid sequence shown in SEQ ID NO: 7 can be mentioned. As used herein, the "homology" or "identity" means the proportion (%) of the same amino acid and similar amino acid residue (same amino acid residues in the case of identity) to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment). The "similar amino acid" means amino acids having similar physicochemical properties and, for example, amino acids classified in the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr), amino acids having a small side chain (Gly, Ala, Ser, Thr, Met) and the like can be mentioned. It is predicted that the substitution with such similar amino acids does not change the phenotype of the protein (that is, conservative amino acid substitution). Specific examples of the conservative amino acid substitution are well known in the technical field and are described in various documents (e.g., Bowie et al., Science, 247: 1306-1310 (1990)). The homology or identity of the amino acid sequence in the present specification can be calculated using homology calculation algorithm NCBI BLAST (National Center for Biotechnology Information Basic Local Alignment Search Tool), and under the following conditions (expectancy =10; accept gap; matrix =BLOSUM62; filtering =OFF).

An exogenous gene can be introduced by a known method. When the introduced nucleic acid is in the form of a DNA, for example, calcium phosphate coprecipitation method, PEG method, electroporation method, microinjection method, lipofection method and the like can be used. For example, the methods described in Cell Engineering additional volume 8, New Cell Engineering experiment protocol, 263-267 (1995) (published by Shujunsha), Virology, vol. 52, 456 (1973), Folia Pharmacol. Jpn., vol. 119 (No. 6), 345-351 (2002) and the like can be used. When a virus vector is used, the nucleic acid is introduced, together with a packaging vector as necessary, into a suitable packaging cell (e.g., Plat-E cell) and complementation cell line (e.g., 293 cell), a virus vector produced in the culture supernatant is recovered, and cells are infected with the vector by an appropriate method suitable for each virus vector, whereby the vector is introduced into the cells. As the virus vector, retrovirus vector (including lentivirus vector and pseudo type vector), adenovirus vector, adeno-associated virus vector, herpes virus vector, Sendaivirus vector, episomal vector and the like can be mentioned. A transposon expression system (PiggyBac system) may also be used. As the plasmid vector, animal cell expression plasmid (e.g., pa1-11, pXT1, pRc/CMV, pRc/RSV, pcDNAI/Neo) and the like can be mentioned. Specifically, when a retrovirus vector is used as the vector, a specific means is disclosed in WO 2007/69666, Cell, 126, 663-676 (2006) and Cell, 131, 861-872 (2007) and the like. Particularly, when a retrovirus vector is used, highly efficient transfection into various cells is possible by using a recombinant fibronectin fragment CH-296 (manufactured by Takara Bio Inc.). When the introduced nucleic acid is in the form of DNA and a viral vector is used, the kind of the viral vector can be appropriately selected depending on the type of cell to be introduced. When the target cell is a T cell, a retrovirus vector is preferably used, and a gamma retrovirus vector is more preferably used. When the target cell is an iPS cell, a lentivirus vector is preferably used.

The introduction nucleic acid may also be directly introduced into cells in the form of RNA and used to express the introduced gene in the cells. As a method for introducing RNA, a known method can be used and, for example, a lipofection method, an electroporation method, or the like can be preferably used. When the reprogramming factor is in the form of a protein, it can be introduced into a cell by a method such as lipofection, fusion with cellular membrane-penetrating peptide (e.g., HIV-derived TAT and polyarginine), microinjection and the like, and the like.

The above-mentioned TCR etc. are introduced into the cells in the form of a nucleic acid encoding TCR etc. In addition, a fusion protein containing IL-15 and IL-15Rα is also introduced into a cell in the form of a nucleic acid encoding the fusion protein. The nucleic acid and a nucleic acid for suppressing the expression of the above-mentioned HLA gene may be DNA or RNA, or DNA/RNA chimera, and preferably DNA. In addition, the nucleic acid may be double-stranded or single-stranded. In the case of double strands, double-stranded DNA, double-stranded RNA or DNA:RNA hybrid may be used. When the nucleic acid is RNA, T is to be read as U as regards the RNA sequence. In addition, the nucleic acid may contain natural nucleotide, modified nucleotide, nucleotide analogue, or a mixture of these as long as it can express polypeptide in vitro or in a cell.

The above-mentioned nucleic acid can be constructed by a method known per se. For example, based on the amino acid sequence or nucleic acid sequence of known TCR or CAR, a DNA strand is chemically synthesized, or synthesized partially overlapping oligo DNA short chains are connected using PCR method or Gibson Assembly method, whereby a DNA encoding the full length or a part of the TCR or CAR can be constructed. The nucleic acid for suppressing the expression of HLA gene, and the nucleic acid encoding a fusion protein containing IL-15 and IL-15Rα can also be constructed in the same manner.

The above-mentioned nucleic acid can be incorporated into an expression vector. The vector may be a vector that integrates or does not integrate into the genome of the target cell. In one embodiment the vector that does not integrate into the genome is capable of replicating outside the genome of the target cell. The vector may be present in multiple copies outside the genome of the target cell. In another embodiment of the invention, the vector integrates into the genome of the target cell. Alternatively, the introduced nucleic acid can also be integrated into the genome by homologous recombination using genome editing (e.g., CRISPR system, TALEN, ZFN etc.) and the like. In preferable embodiments, nucleic acid such as vector and the like are integrated at a pre-defined location of the genome of the target cell.

Examples of the promoter to be used in the above-mentioned vector include EF1α promoter, CAG promoter, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter, TCR V α gene promoter, TCR V β gene promoter and the like. Of these, EF1α promoter, CAG promoter, MoMuLV LTR, CMV promoter, SRα promoter and the like are preferable.

The above-mentioned vector may contain transcription and translation regulatory sequence, ribosome binding site, enhancer, replication origin, polyA addition signal, selection marker gene and the like on demand besides the above-mentioned promoters. Examples of the selection marker gene include dihydrofolate reductase gene, neomycin resistance gene, puromycin resistance gene and the like.

In one embodiment of the present invention, heterodimers of α chain and β chain of TCR can be constructed in the target cell and on the cell surface by introducing an expression vector containing a nucleic acid encoding the α chain of TCR and a nucleic acid encoding β chain of TCR into the target cell. In this case, the nucleic acid encoding the α chain of TCR and the nucleic acid encoding β chain of TCR may be incorporated into separate expression vectors or a single expression vector. When they are incorporated into a single expression vector, these two kinds of nucleic acids are preferably incorporated via a sequence enabling polycistronic expression. Using a sequence enabling polycistronic expression, plural genes incorporated in one kind of expression vector can be more efficiently expressed. Examples of the sequence enabling polycistronic expression include 2A sequence (e.g., foot-and-mouth disease virus (FMDV)-derived 2A sequence (F2A), horse rhinitis A virus (ERAV)-derived 2A sequence (E2A), Porcine teschovirus (PTV-1)-derived 2A sequence (P2A), Thosea asigna virus (TaV)-derived 2A sequence (T2A sequence) (PLoS ONE3, e2532, 2008, Stem Cells 25, 1707, 2007), internal ribosome entry site (IRES) (U.S. Patent No. 4,937,190) and the like. From the aspect of uniform expression levels, P2A sequence and T2A sequence are preferable. The same applies to the case of using an expression vector containing a nucleic acid encoding the γ chain of TCR and a nucleic acid encoding the δ chain of TCR.

An exogenous gene to be introduced into T cells constituting a cell bank of T cell can be introduced into a cell that occurs during the process of differentiating stem cell (e.g., iPS cell or ES cell) into T cell. As used herein, the cell that occurs during the process of differentiating stem cell (e.g., iPS cell or ES cell) into T cell also includes a stem cell (e.g., iPS cell or ES cell) and T cell.

In one embodiment of the present invention, the exogenous gene to be introduced into T cell constituting the cell bank of T cell is introduced into iPS cell, hematopoietic progenitor cell and/or T cell.

In one embodiment of the present invention, before constituting the cell bank of T cell, the T cell obtained above can be expansion cultured as necessary by the method shown in the below-mentioned "1-5. Expansion culture".

A master cell bank of T cell can be constructed by dispensing the T cells obtained by the above-mentioned method into plural preservation containers (aseptic vial and the like), and stocking them. The stocked T cells are preferably frozen. One container of T cells in the master cell bank of T cell can be thawed as necessary and subjected to a property analysis test as appropriate. A working cell bank of T cell can be constructed as necessary by expansion culturing T cells prepared from the same container or from another container by the method shown in "1-5. Expansion culture" described later, dispensing the obtained T cells in a plurality of containers for each expansion culture, and stocking them. The stocked T cells are preferably frozen.

Therefore, the provision system of the present invention may include a step of constructing a master cell bank of T cell and/or a working cell bank of T cell (hereinafter to be also referred to as "cell bank of T cell construction step"). In the following, the part constituted to execute the construction step of a cell bank of T cell is sometimes referred to as a "cell bank of T cell-construction part".

### 1-2. Freezing T cells

As described above, the T cells in the cell bank of T cell are preferably frozen. T cells can also be frozen by a known method. Examples of the method include, but are not limited to, placing the container containing the T cells in a freezer (e.g., ultra-low temperature freezer), and bringing the cells into contact with a low temperature medium (e.g., liquid nitrogen, etc.) and preserving same in a freezer or cryopreservation system (e.g., locator, etc.). The freezing temperature is typically not more than 0°C, preferably not more than -20°C, more preferably not more than -40°C, further preferably not more than -80°C. The cooling rate in the freezing operation is typically a cooling rate of 1 - 5 hr, preferably 2 - 4 hr, particularly about 3 hr, from the start of cooling at 4°C to reach -80°C. Such cooling rate can be achieved by providing a freezing means set to a desired temperature in contact with a container containing T cells directly or after accommodating the container in a freezing treatment container. The freezing treatment container may have a function of controlling the rate of temperature decrease inside the container to a predetermined speed. As the freezing treatment container, BICELL (registered trade mark) (Japan) and the like can be used. The above-mentioned cooling rate can be achieved by using a freezer whose cooling rate can be controlled by setting a program or the like. As such freezer, any known freezer, for example, a program freezer (e.g., KryoMed (Thermo Fisher), PDF-2000G (Strex), KRYO-560-16 (Asahi Life Science)), and the like can be used.

The freezing operation can be performed using a culture medium in which colonies were immersed, a physiological buffer solution, or the like as a cryoprotective solution, and treating the solution by adding a cryoprotective agent, or replacing the culture medium with a cryoprotective solution containing a cryoprotective agent. When replacing the culture medium with a cryoprotective solution, the cryoprotective solution may be added after substantially removing all the culture medium, or the cryoprotective solution may be added while leaving a part of the culture medium. The cryoprotective solution may be commercially available and, for example, CryoStor (registered trade mark) CS10, and STEM-CELLBANKER (registered trade mark) (ZENOAQ) can be mentioned.

The cryoprotective agent is not particularly limited and examples thereof include dimethyl sulfoxide (DMSO), ethylene glycol (EG), propylene glycol (PG), 1,2-propanediol (1,2-PD), 1,3-propanediol (1,3-PD), butyleneglycol (BG), isopreneglycol (IPG), dipropylene glycol (DPG), glycerol and the like. The cryoprotective agent may be used alone or two kinds or three kinds or more may be used in combination. Moreover, the cryoprotective agent may be used in combination with an extracellular cryoprotective agent. Examples of the extracellular cryoprotective agent include polyethylene glycol, carboxymethylcellulose sodium, polyvinylpyrrolidone, hydroxyethylstarch (HES), dextran, albumin and the like.

The concentration of the cryoprotective agent added to the culture medium, or the concentration of the cryoprotective agent in the cryoprotective solution, is typically 2 - 20%(v/v), preferably 5 - 15%, more preferably 8 - 13%, of the total culture medium or cryoprotective solution. The above-mentioned concentration can be appropriately adjusted by the kind of the cryoprotective agent. For example, when DMSO is used as the cryoprotective agent, concentration of DMSO is typically 2 - 20%(v/v), preferably 2.5 - 12.5%, more preferably 5 - 10%, of the total culture medium or cryoprotective solution.

Specific examples of the cryoprotective agent and the freezer are as mentioned above. As the container containing T cells, a cryopreservation container can be used. Specific examples of the cryopreservation container include aseptic vial (e.g., glass ampoule, polymer vial (AT-closed vial) etc.) and the like.

### 1-3. Preparation of T cell

In the present invention, a cell bank of T cell generally contains one or more containers in which T cells are dispensed and frozen. Thus, the preparation of T cell also includes a process of collecting one or more containers from a cell bank of T cell and thawing the T cells, a process of washing the thawed cells as necessary, a process of preculturing and cultivating the cells, a process of maintenance culturing the cells, and the like. Each of the above-mentioned processes may be performed by the same entity or by different entities.

The aforementioned method for thawing T cells can be performed by a known method. For example, a container containing frozen T cells collected from a cell bank of T cell is contacted with a solid, liquid or gaseous medium (e.g., water, culture medium) having a temperature higher than the freezing temperature by using a water bath, an incubator, or the like. The method is not limited to this method. The temperature of the medium is typically 4°C - 50°C, preferably 30°C - 40°C, more preferably 36°C - 38°C. The thawing time is typically within 2 minutes, particularly 20 seconds, whereby a decrease in the survival rate of the cell can be drastically suppressed. The thawing time can be adjusted by, for example, changing the thawing means, temperature of immersion medium, volume or composition of the culture medium or cryoprotective solution during freezing, and the like.

The cells can be washed by a known method. For example, the method includes, but is not limited to, suspending the cells in a cell washing (e.g., culture medium or biological buffer etc. containing serum, serum components (serum albumin and the like)), centrifuging same, discarding the supernatant, and collecting the precipitated cells. In the process of washing the cells, the cycle of suspending, centrifugation, and recovery may be performed once or plural times (e.g., 2, 3, 4, 5 or more). In one embodiment of the present invention, the process of washing the cells is performed immediately after the process of thawing the T cells collected from a cell bank of T cell. Examples of commercially available cell washing that can be used in the present invention include CELLOTION (Japan ZENYAKU KOGYO) and the like.

In the present invention, a medium for the aforementioned preculture and maintenance culture is not particularly limited, and a medium used for culturing animal cells can be prepared into a basal medium. Where necessary, the basal medium may contain a medium additive and the like. As the basal medium and medium additive, those recited in the above-mentioned 1-1., process (1), can be mentioned.

The preculture and maintenance culture can be performed for an appropriate culture period according to the purpose. The culture period is preferably not less than 1 day (e.g., 5, 6, 7 days) and not more than 10 days (e.g., 7, 8, 9, 10 days). The culture temperature is not particularly limited and is 30°C - 40°C, preferably 37°C. Culture is performed in the presence of CO₂-containing air and the CO₂ concentration is preferably 2 - 5%.

### 1-4. Introduction of nucleic acid into T cell prepared from a cell bank of T cell

The provision system of the present invention may include a step of introducing a nucleic acid containing an exogenous gene into T cells prepared from a cell bank of T cell (hereinafter to be also referred to as "nucleic acid introduction step"). In the present specification, "include a step" or "include a process" means that a part that is constituted to perform the step or process is contained. In the following, a part that is constituted to perform the nucleic acid introduction step is sometimes referred to as a "nucleic acid introduction part".

A method for introducing a nucleic acid in the nucleic acid introduction step, an exogenous gene and a nucleic acid containing same are as described above.

The exogenous gene to be introduced in the nucleic acid introduction step may be the same as or different from the exogenous gene already introduced into the T cells constituting a cell bank of T cell.

The nucleic acid introduction part may be provided with a basal medium, a culture container, and a sterile space, and may be configured so that a nucleic acid containing an exogenous gene can be introduced into the above-mentioned prepared T cells. In the present specification, the sterile space means a sterile internal space defined by a clean chamber, a clean bench, a sterile room, and the like, and also includes a sterile internal space defined by the entire nucleic acid introduction part and a sterile space that is a part of the internal space of the nucleic acid introduction part. The device, medium, etc. provided in the nucleic acid introduction part are appropriately selected according to the property of the device, the method of introducing nucleic acid, and the like. The nucleic acid introduction part may be provided as necessary with one or more kinds of medium additive, clean bench, incubator, bioreactor for cell culture, microscope, centrifuge, aspirator, nucleic acid to be introduced, nucleic acid encoding protein for genome editing, and the like, pipette, tube, buffering agent (e.g., acetate buffer, phosphate buffer, citrate buffer, citric acid phosphate buffer, boric acid buffer, tartaric acid buffer, tris buffer, phosphate buffered saline, McIlvaine buffer etc.), gene transfer adjuvant and the like, and further provided with other reagents, device and the like according to the kind of the nucleic acid introduction method.

Specific examples of the above-mentioned basal medium and medium additive provided to the nucleic acid introduction part are as mentioned above. Examples of the culture container include petri dish, flask, plastic bag, Teflon (registered trade mark) bag, dish, petri dish, tissue culture dish, multidish, microplate, microwell plate, Multiplate, multiwell plate, chamber slide, cell culture flask, spinner flask, tube, tray, culture bag, roller bottle and the like that are generally used for cell culture. The material of these culture containers is not particularly limited and, for example, glass, polyvinyl chloride, cellulose type polymers such as ethylcellulose, acetylcellulose and the like, plastic such as polystyrene, polymethyl methacrylate, polycarbonate, polysulfone, polyurethane, polyester, polyamide, polystyrene, polypropylene, polyethylene, polybutadiene, poly(ethylenevinylacetate) copolymer, poly(butadiene-styrene) copolymer, poly(butadiene-acrylonitrile) copolymer, poly(ethylene-ethylacrylate) copolymer, poly(ethylene-methacrylate) copolymer, polychloroprene, styrole resin, chlorosulfonated polyethylene, ethylenevinyl acetate, acrylic block copolymer and the like, and the like can be mentioned.

As the above-mentioned other reagent and device, when, for example, nucleic acid introduction is performed by a calcium phosphate coprecipitation method, the nucleic acid introduction part may be provided with one or more kinds of various reagents and solutions (e.g., CaCl₂ and its solution, HCl and its solution, NaCl and its solution, MgCl₂ and its solution, Na₂HPO₄ and its solution, KH₂PO₄ and its solution, NP-40 and its solution, HEPES and its solution, DMSO and its solution etc.) and the like. For example, when an electroporation method is used, the nucleic acid introduction part may be provided with one or more kinds of electroporator, electrode (e.g., platinum electrode, cuvette electrode, petri dish platinum plate electrode etc.), electroporation plate, chamber (e.g., electrode chamber, plate chamber etc.), and the like. For example, when a lipofection method is used, it may be provided with one or more kinds of transfection reagents (e.g., Lipofectamine (invitrogen), jetPEI (Polyplus-transfection), XfectTM (Clontech TaKaRa cellartis), GenomONETM (ISHIHARA SANGYO KAISHA)), Trans IT (Mirus Bio LLC), RmesFect/RmesFect Stem (OZ BIOSCIENCES) etc.) and the like. For example, when nucleic acid introduction is performed using a virus vector, the nucleic acid introduction part may be provided with one or more kinds of various virus vectors, packaging vector plasmid, packaging (e.g., Plat-E cell), complementary cell line, laboratory of biosafety level 2 and the like recited above.

### 1-5. Expansion culture

The provision system of the present invention may include a step of expansion culturing T cells prepared from a master cell bank of T cell or T cells obtained by introducing a nucleic acid containing an exogenous gene into the T cells (hereinafter to be also referred to as "expansion culture step"). In the following, the part constituted to execute the expansion culture step is sometimes referred to as a "expansion culture part".

In the present specification, the "expansion culture" means culturing for the purpose of proliferating a desired cell population and increasing the cell number. The increase in cell number may be achieved by increasing the number of cells by proliferation to exceed the decrease in number by death, and it does not require proliferation of all cells in the cell population. The increase in the cell number may be 1.1 times, 1.2 times, 1.5 times, 2 times, 3 times, 4 times, 5 times, 6 times, 7 times, 8 times, 9 times, 10 times, 15 times, 20 times, 30 times, 40 times, 50 times, 100 times, 300 times, 500 times, 1,000 times, 3,000 times, 5,000 times, 10,000 times, 100,000 times, or not less than 1,000,000 times, compared to that before the start of expansion culture.

The expansion culture step can be performed by a known method. However, from the aspect of cell proliferation efficiency, it is preferable to perform the expansion culture step by a method including a process of stimulating T cells with a CD30 agonist, which was found by the present inventors. The T cells can be stimulated with a CD30 agonist by culturing the T cells in the presence of a CD30 agonist. Thus, a method including a process of stimulating T cells in the presence of a CD30 agonist is preferable. Therefore, in one embodiment of the present invention, the expansion culture step may include a process of culturing T cells in the presence of a CD30 agonist. When the expansion culture is performed in the presence of a CD30 agonist, the culture medium preferably contains a CD3/TCR complex agonist, and fibronectin or a variant thereof as well. CD3/TCR complex agonist and CD30 agonist can each stimulate CD3/TCR complex and CD30.

The proliferative capacity of T cell differentiated from iPS cell is lower than that of iPS cell. Therefore, even if a master cell bank and/or working cell bank are/is constructed in the stage of T cell, it has been difficult to supply T cells and T cell products in an amount sufficient to achieve the expected therapeutic effect by administering to one or more humans. The present invention overcomes such difficulty. Furthermore, using a method including the above-mentioned process of stimulating T cells with a CD30 agonist in the expansion culture step, it becomes possible to supply T cell products more stably by constructing a master cell bank and/or a working cell bank at the stage of T cell.

In the present specification, "stimulation" means that a certain substance binds to various receptors and the like to activate a signal pathway at the downstream thereof.

In the present invention, a medium used for expansion culture step of T cells is not particularly limited, and a medium used for culturing animal cells can be prepared into a basal medium. Where necessary, the basal medium may contain a medium additive and the like. As the basal medium and medium additive, those recited in the above-mentioned 1-1., process (1), can be mentioned. The culture can be performed, for example, in a CO₂ incubator under a CO₂ concentration atmosphere of about 1 - about 10%, preferably about 2 - about 5% at about 30 - about 40°C, preferably about 37°C. The culture period can be appropriately determined by those skilled in the art by monitoring the number of T cells and the like. The number of days is not limited as long as T cells can be obtained. The culture period is, for example, not less than 3 days, not less than 5 days, not less than 7 days, not less than 10 days, not less than 14 days, not less than 21 days, preferably not less than 5 days and not more than 15 days. It is preferably not more than 30 days, more preferably not more than 21 days. The culture period is, for example, 3 - 30 days, 5 - 30 days, 7 - 30 days, 10 - 30 days, 14 - 30 days, 21 - 30 days, 3 - 21 days, 5 - 21 days, 7 - 21 days, 10 - 21 days, 14 - 21 days, 3 - 15 days, 5 - 15 days, 7 - 15 days, 10 - 15 days, 14 - 15 days, or the like.

When vitamin C is used in the expansion culture step, Vitamin C may be the same as that described in process (2-1) and can be added similarly. In one embodiment, the concentration of vitamin C in the medium or culture medium is preferably 5 µg/ml - 200 µg/ml. In another embodiment, vitamin C is added to the culture medium at an amount corresponding to 5 µg/ml - 500 µg/ml (e.g., amount corresponding to 5 µg/ml, 10 µg/ml, 25 µg/ml, 50 µg/ml, 100 µg/ml, 200 µg/ml, 300 µg/ml, 400 µg/ml, 500 µg/ml).

When a CD3/TCR complex agonist is used in the expansion culture step, CD3/TCR complex agonist used in this step may be the same as that described in the above-mentioned 1-1., process (3). The concentration of the CD3/TCR complex agonist in the medium is also the same as that described in the above-mentioned 1-1., process (3).

Fibronectin or a variant thereof is preferably present in the medium in the expansion culture step. Such fibronectin is not particularly limited as long as it is a molecule capable of binding to T cells. The variant of fibronectin is not particularly limited as long as it is a molecule capable of binding to VLA-5 and VLA-4 on the surface of T cells, and examples thereof include RetroNectin. Fibronectin and a variant thereof may be present in any form in the medium. For example, they may be contained in the medium during culture, or may be immobilized on a culture container, and are preferably immobilized on a culture container.

When fibronectin or a variant thereof is contained in a medium, the medium may be the same as that containing a CD3/TCR complex agonist. The presence or absence of serum, additive and the like may be the same as that in the medium containing a CD3/TCR complex agonist. When fibronectin or a variant thereof is contained in a medium, the lower limit of the concentration of fibronectin or a variant thereof may be not less than 10 ng/ml, preferably not less than 100 ng/ml, and the upper limit may be not more than 10000 µg/ml, preferably not more than 1000 µg/ml.

In the expansion culture step, the medium also preferably contains a CD30 agonist. The CD30 agonist is not particularly limited as long as it is a molecule capable of transducing a signal from a CD30 into a cell by specifically binding to CD30. Examples of the CD30 agonist include at least one selected from the group consisting of anti-CD30 agonist antibody (to be also simply referred to as "anti-CD30 antibody") or a fragment bonded thereto and CD30 ligand or a fragment bonded thereto.

Like the CD3/TCR complex agonist, the CD30 agonist used in the expansion culture step may be present in any form as long as it can be present in contact with CD30 during culturing. For example, it may be contained in the medium during culture, or may be immobilized on a culture container, and is preferably contained in the medium.

When a CD30 agonist is contained in a medium, the medium may be the same as that containing a CD3/TCR complex agonist. The presence or absence of serum, additive and the like may be the same as that in the medium containing a CD3/TCR complex agonist. When a CD30 agonist is contained in a medium, the concentration of the CD30 agonist in the medium can be appropriately determined by those of ordinary skill in the art according to the CD30 agonist. For example, when the CD30 agonist is an anti-CD30 agonist antibody or a bond fragment thereof, the concentration of the anti-CD30 agonist antibody or a bond fragment thereof in the medium is generally 1 ng/ml - 10000 ng/ml, preferably 30 ng/ml - 300 ng/ml.

When the CD30 agonist is immobilized on a culture container, the culture container may be the same as that on which the CD3/TCR complex agonist is immobilized. In addition, a method of immobilizing the CD30 agonist on the culture container may be the same as that of immobilizing the CD3/TCR complex agonist. The lower limit of the concentration of a CD30 agonist solution when the CD30 agonist is immobilized on a culture container may be not less than 0.1 ng/ml, preferably not less than 1 ng/ml, and the upper limit may be not more than 10000 ng/ml, preferably not more than 1000 ng/ml. The concentration is, for example, 0.1 - 10000 ng/ml, 1 - 10000 ng/ml, 0.1 - 1000 ng/ml, 1 - 1000 ng/ml, 30 ng/ml - 300 ng/ml, or the like.

When cytokine is used in the expansion culture step, cytokine may be IL-2, IL-7, IL-12, IL-15, IL-18, IL-21 or the like. Only one kind of these may be used or plural kinds (preferably all kinds) thereof may be used. When cytokine is IL-2, the concentration thereof in the medium may be 10 U/ml - 1000 U/ml or 0.01 ng/mL - 1000000 ng/mL, when cytokine is IL-7, the concentration thereof in the medium may be 0.1 ng/ml - 1000 ng/ml, 1 ng/ml - 500 ng/ml, 5 ng/ml - 100 ng/ml (e.g., 10 ng/ml). The concentration of IL-12 in the medium may be 0.1 ng/ml - 1000 ng/ml, 1 ng/ml - 500 ng/ml, 5 ng/ml - 100 ng/ml (e.g., 50 ng/ml), the concentration of IL-15 in the medium may be 0.1 ng/ml - 1000 ng/ml, 1 ng/ml - 500 ng/ml, 5 ng/ml - 100 ng/ml (e.g., 10 ng/ml), the concentration of IL-18 in the medium may be 0.1 ng/ml - 1000 ng/ml, 1 ng/ml - 500 ng/ml, 5 ng/ml - 100 ng/ml (e.g., 50 ng/ml), and the concentration of IL-21 in the medium may be 0.1 ng/ml - 1000 ng/ml, 1 ng/ml - 500 ng/ml, 5 ng/ml - 100 ng/ml (e.g., 20 ng/ml).

In the expansion culture step, a TNF family cytokine may be contained as cytokine in the medium. Examples of the TNF family cytokine include TNF-α, TNF-β, lymphotoxin α, Fas ligand, TRAIL, TWEAK, TL1A, RANK ligand, OX40 ligand, APRIL, AITRL, BAFF, 4-1BBL and CD40 ligand and the like, and TL1A is preferable. When TL1A is used, the concentration thereof in the medium may be 5 ng/ml - 500 ng/ml, preferably 10 ng/ml - 300 ng/ml, more preferably 20 ng/ml - 200 ng/ml (e.g., 50 ng/ml).

In the expansion culture step, moreover, an apoptosis inhibitor may be further contained in the medium. As the apoptosis inhibitor, a protease inhibitor can be mentioned, for example, caspase inhibitor. As the caspase inhibitor, Pan Caspase FMK inhibitor Z-VAD (N-benzyloxycarbonyl-Val-Ala-Asp(O-Me) fluoromethylketone) (hereinafter sometimes referred to as "Z-VAD-FMK") is preferable, and the concentration thereof in the medium may be 1 µM - 1000 µM, preferably 1 µM - 500 µM, more preferably 1 µM - 200 µM, particularly preferably 1 µM - 50 µM (e.g., 10 µM).

In the present invention, the obtained T cells may be used after isolation, or may be used as it is (namely, as a cell population possibly containing other cell type). When isolated, isolation can be performed using at least one molecule selected from the group consisting of αTCR, βTCP and CD3 as an index, and the isolation method used may be a method well known to those of ordinary skill in the art. Examples thereof include, but are not limited to, a method using an antibody for αTCR, βTCR and CD3 (bound with magnetic beads and the like as necessary) and isolation by flow cytometry or magnetic cell separation method, a purification method using an affinity column on which a desired antigen is immobilized and the like.

When used as it is, the ratio of the T cells in the cell population may be increased by using a method well known to those of ordinary skill in the art. Examples of the method for increasing the ratio of the T cells in the cell population include, but are not limited to, the methods of Front. Immunol., 5:636 (2014), National Publication of International Patent Application No. 2017-537625, National Publication of International Patent Application No. 2003-529363 and the like.

Therefore, the expansion culture part is provided with a basal medium, a culture container, a sterile space, and preferably a CD3 agonist, and constituted to perform expansion culture of T cells. The device, medium and the like to be provided to the nucleic acid introduction part are appropriately selected according to the property of the device, method of expansion culture, and the like. When a CD3 agonist is used, the culture container and CD30 agonist may be provided on the expansion culture part such that CD30 is immobilized on the culture container. The expansion culture part may be provided with, as necessary, one or more kinds of CD3/TCR complex agonist, fibronectin or a variant thereof, apoptosis inhibitor, medium additive, clean bench, incubator, bioreactor for cell culture, microscope, centrifuge, aspirator, pipette, tube, buffering agent (e.g., acetate buffer, phosphate buffer, citrate buffer, citric acid phosphate buffer, boric acid buffer, tartaric acid buffer, tris buffer, phosphate buffered saline, McIlvaine buffer etc.) and the like, and further provided with other reagents, device and the like according to the kind of the culture method. When cytokine is used as a medium additive, preferable cytokine is, for example, IL-2, IL-7, IL-12, IL-15, IL-18, IL-21, TNF family cytokine or the like.

Specific examples of the above-mentioned basal medium, medium additive, CD3 agonist, CD3/TCR complex agonist, fibronectin or a variant thereof and apoptosis inhibitor to be provided to the expansion culture part are as mentioned above. Specific examples of the culture container include the same one described in 1-4.. A T cell product can be produced by filling the expansion cultured T cells in an appropriate storage container (sterile vial, blood transfusion bag, etc.), labeling the container as necessary, and packaging the container.

### 1-6. Production of frozen T cell product

The provision system of the present invention may include a step of producing a frozen T cell product from the aforementioned expansion cultured T cells (hereinafter to be also referred to as "frozen T cell product production step"). In the following, the part constituted to execute the frozen T cell product production step is sometimes referred to as a "frozen T cell product production part".

The frozen T cell product production step can be performed by a method similar to 1-2.

Therefore, the frozen T cell product production part may be provided with at least a cryoprotective agent, a cryopreservation container, a freezer and/or a cryoprotective system (e.g., Locator etc.), and constituted to produce a frozen stock of expansion cultured T cells. The frozen stock production part may be provided with, as necessary, one or more kinds of low temperature medium (e.g., liquid nitrogen etc.), pipette, tube, buffering agent (e.g., acetate buffer, phosphate buffer, citrate buffer, citric acid phosphate buffer, boric acid buffer, tartaric acid buffer, tris buffer, phosphate buffered saline, McIlvaine buffer etc.) and the like.

Specific examples of the cryoprotective agent and freezer to be provided to the frozen T cell product production part are as mentioned above. Specific examples of the cryopreservation container include, vial (e.g., glass ampoule, plastic tube etc.) and the like.

### 1-7. Construction of T cell product collection

The provision system of the present invention may include a step of constructing a T cell product collection including two or more kinds of T cell products by collecting T cell products (hereinafter to be also referred to as "T cell product collection construction step"). In the following, the part constituted to execute the T cell product collection construction step is sometimes referred to as a "T cell product collection construction part".

The T cell products constituting the T cell product collection may be frozen T cell products. From the aspect of the stability of T cells, a frozen T cell product is preferable for long-term storage.

The T cell product collection construction step can also be performed by a known method. Such method includes construction by producing two or more kinds of T cell products by the above-mentioned method, attaching a label or the like so that the kind of the T cell products can be distinguished, and storing the products. The kind of the collection can also be increased by further adding different kinds of T cell products to the constructed T cell product collection. For easy storage, it is preferable to store in the same space (e.g., in the same freezer, in the same room, in the same building, on the same site, etc.); however, it may be stored in a physically separate space. Any method may be used to distinguish the kind of a T cell product as long as the kind can be distinguished. For example, a label and the like may be attached to the container, or when stored in other freezer or cryoprotective system, a label and the like may be attached to the freezer or cryoprotective system, or management software and the like may be used to distinguish the products and the like.

Therefore, the T cell product collection construction part may be provided with at least T cell products to be collected, and constituted to construct a T cell product collection including two or more kinds of T cell products by collecting T cell products constructed as mentioned above. In addition, the T cell product collection construction part may be provided, as necessary, with one or more kinds of label, freezer, cryoprotective system, management software and the like. Specific examples of the freezer are as described above.

### 1-8. Identification of antigen

The provision system of the present invention may include a step of identifying a tumor-specific antigen or a tumor-associated antigen (hereinafter to be simply referred to as "antigen") expressed in a tumor of the test subject (hereinafter to be also referred to as "antigen identification step"). In the following, the part constituted to execute the antigen identification step is sometimes referred to as an "antigen identification part".

The antigen identification step can also be performed by a known method. Examples of the method include collecting a biological sample such as tumor tissue section, body fluid (e.g., blood, serum, plasma etc.) containing tumor cells, and the like from a test subject, extracting mRNA from the sample, synthesizing cDNA from the mRNA as necessary, and identifying the nucleic acid by a nucleic acid amplification method and/or nucleic acid detection method such as digital PCR (e.g., ddPCR), RT-PCR, biochip (e.g., microarray), RNAseq and the like. PCR can be performed using, for example, a primer set capable of amplifying mRNA of a tumor-specific antigen or a tumor-associated antigen, and a PCR device. When RNAseq is used, PCR can be performed using a next-generation sequencer.

Therefore, the antigen identification part may be constituted to be able to identify a tumor-specific antigen or tumor-associated antigen expressed in the tumor of the subject. For example, the antigen identification part is provided with at least the above-mentioned primer set and the PCR device, at least the above-mentioned biochip, or at least the above-mentioned next-generation sequencer.

Examples of the above-mentioned next generation sequencer include, but are not limited to, the device manufactured by illumina (e.g., MiSeq, HiSeq2500), the device manufactured by Thermo Fisher Scientific (e.g., Ion Proton, Ion PGM), the device manufactured by Roche Diagnostic (e.g., GS FLX+, GS Junior) and the like.

### 1-9. Selection of T cell product

In the provision system of the present invention, information of the test subject is obtained, and an appropriate T cell product can be selected based on the information. Therefore, the provision system of the present invention may include a step of obtaining test subject information and/or a step of selecting a T cell product suitable for the test subject. In the following, the part constituted to execute the test subject information obtaining step is sometimes referred to as a "test subject information obtaining part".

The information of the test subject is not particularly limited and includes, for example, genetic information (e.g., HLA gene-related information, abnormal gene information, etc.) of the test subject, diagnosis result, history, drug administration history, age, gender, blood type, height, body weight, and when the test subject has a tumor, information of tumor-specific or tumor-associated antigen expressed in the tumor.

One embodiment of the present invention may include a step of selecting a T cell product expressing a CAR or an exogenous TCR that recognizes and binds to the antigen identified from the information of the test subject from a T cell product collection containing two or more kinds of T cell products (hereinafter to be also referred to as "T cell product selection step"). In the following, the part constituted to execute the T cell product selection step is sometimes referred to as a "T cell product selection part". The T cell product in the T cell product selection step may be a frozen T cell product. From the aspect of the stability of T cells, a frozen T cell product is preferable.

The step of selecting a T cell product expressing a CAR or an exogenous TCR that recognizes and binds to the identified antigen from a T cell product collection containing T cell products is encompassed in concept in the step of selecting a T cell product suitable for the test subject based on the obtained information of the test subject. The test subject information obtaining part includes an antigen identification part. In one embodiment of the present invention, the T cell product selection step can select a cell bank of T cell containing T cells that express appropriate CAR, exogenous TCR, cytokine, chemokine, and the like, based on, for example, the content of the production plan of the T cell product to be provided.

T cell product selection step can be performed by a known method. The method includes selecting, based on the material (paper medium or electronic data medium) showing the identification results by the above-mentioned antigen identification step, a T cell product containing T cells that express a CAR or an exogenous TCR that recognize and bind to the identified antigen from a list of T cell products prepared in advance. When multiple kinds of tumor-specific antigens or tumor-associated antigens are expressed in the antigen identification step, a T cell product containing T cells can be selected based on the evaluation criteria such as one having the highest expression level or one expected to be most effective with the T cell product. Only one kind of T cell product may be selected, or two or more kinds may be selected. These steps may use software designed for the selection of the above-mentioned T cell product. T cell product can be selected according to instructions (prescription, etc.) from a doctor or medical institution.

Therefore, the T cell product selection part is provided with at least a material showing the identification results in the antigen identification step, and may be constituted to select a T cell product expressing a CAR or an exogenous TCR that recognizes and binds to the identified antigen from a T cell product collection containing T cell products. The T cell product selection part may be provided, as necessary, with one or more kinds of a list of T cell products, the above-mentioned software designed for selection of the T cell product and the like.

The construction part of a cell bank of T cell, construction part of a cell bank collection of T cell (described later), cell bank of T cell selection part (described later), frozen T cell product production part, T cell product collection construction part and T cell product selection part manage information relating to T cells constituting the cell bank of T cell and T cell product (hereinafter to be referred to as "T cell-related information"). The T cell-related information includes production records, property analysis results, quality evaluation results, storage temperature control records, production plans, and the like relating to the T cells. When the T cell is an iPS cell-derived T cell, the production records, property analysis results, quality evaluation results, storage temperature records, specific gene information, and the like relating to the iPS cell are included. When the T cell is a T cell into which a nucleic acid encoding an exogenous gene has been introduced, information on the exogenous gene (e.g., kind of protein and cytokine encoded by the exogenous gene, etc.) and the like are included. In the cell bank of T cell selection part and T cell product selection part, a cell bank of T cell and a T cell product are each selected based on the contents of the production plan of the T cell product to be provided. In the cell bank of T cell selection part and T cell product selection part, a cell bank of T cell and a T cell product may be appropriately selected based on the information of the test subject obtained in the test subject information obtaining part. Therefore, the cell bank of T cell selection part and the T cell product selection part can be used even when only one kind of cell bank of T cell and/or T cell product is involved.

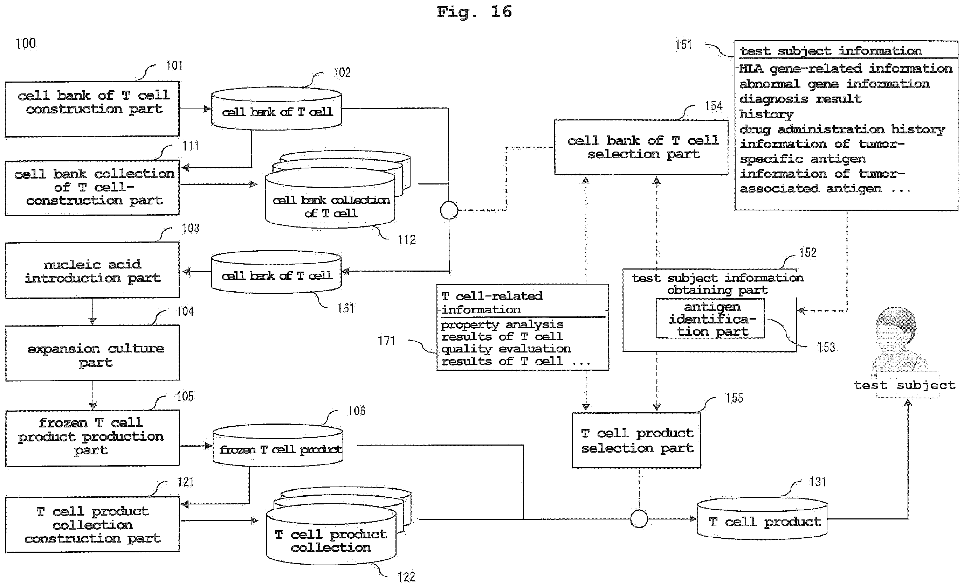

One aspect of the present invention is described by reference to Fig. 16. In the present specification and the drawings, the constituent elements having substantially the same functional constituent are designated by the same symbols to omit duplicate description.

As shown in Fig. 16, the T cell product provide system 100 prepares T cells from a cell bank of T cell 102 to provide a T cell product 131. The cell bank of T cell 102 may be constructed by the cell bank of T cell construction part 101. When two or more kinds of cell banks of T cell are constructed, they may be collected by the cell bank collection of T cell-construction part 111 to construct the cell bank collection of T cell 112.

The prepared T cells may be introduced with a desired exogenous gene into the nucleic acid introduction part 103 as necessary. Further, the T cells may be expansion cultured in the expansion culture part 104 if necessary. Further, the T cells may be frozen in the frozen T cell product production part 105 to provide a frozen T cell product 106 if necessary. When two or more kinds of frozen T cell products are produced, they may be collected by the T cell product collection construction part 121 to construct a T cell product collection 122.

In the cell bank of T cell selection part 154 and the T cell product selection part 155, an appropriate cell bank of T cell and T cell product can be selected as the T cell product 131 to be provided based on the T cell-related information 171. Such T cell-related information is managed by the cell bank of T cell construction part 101, the cell bank collection of T cell-construction part 111, the cell bank of T cell selection part 154, the T cell product collection construction part 121, and the T cell product selection part 155.

Furthermore, to provide a T cell product more suitable for the treatment and/or prevention of the subject, the test subject information 151 is obtained by the test subject information obtaining part 152, and an appropriate T cell product 131 can be selected by the T cell product selection part 155 based on the information of the test subject. The T cell product 131 is the same as the T cell product 106 when the T cell product is one kind, and one or more kinds of T cell products contained in the T cell product collection 122 when selected from the T cell product collection 122. The test subject information obtaining part may include an antigen identification part 153. An appropriate cell bank of T cell 161 may be selected by the cell bank of T cell selection part 154 based on the information obtained in the test subject information obtaining part 152. The cell bank of T cell 161 is the same as the cell bank of T cell 102 when the cell bank of T cell is one kind, and when selected from the cell bank collection of T cell 112, the cell bank of T cell 161 may be one or more kinds included in the cell bank collection of T cell 112. T cells can be prepared from the cell bank of T cell 161 and the T cell product can be provided in the same manner as described above.

Next, the constitution of hardware that can be included in the test subject information obtaining part, the cell bank of T cell construction part, the cell bank of T cell selection part and/or the T cell product selection part is described. Since the cell bank of T cell selection part and the T cell product selection part have the same hardware constitution, the hardware constitution that can be included in the T cell product selection part is described here.

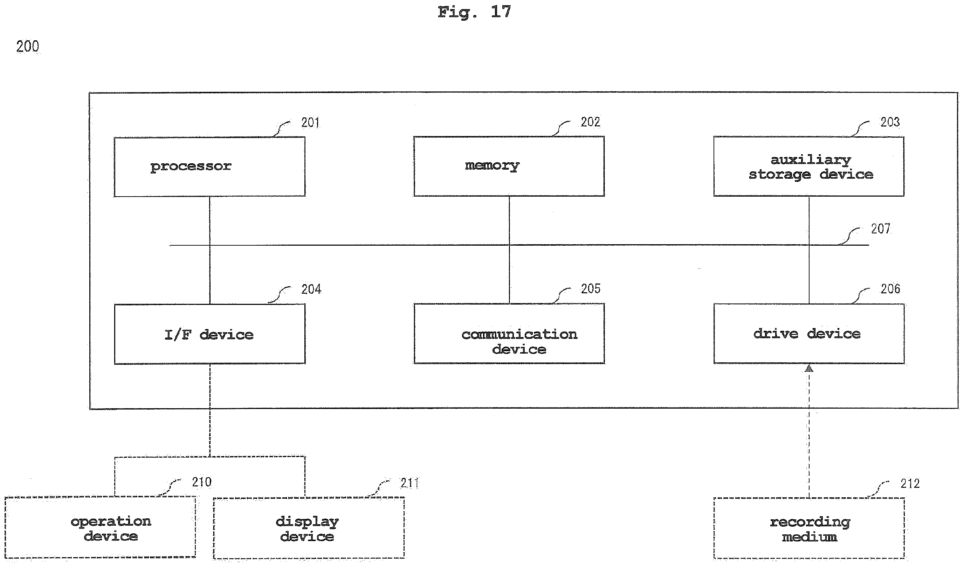

Fig. 17 shows one embodiment of a hardware constitution that can be included in the T cell product selection part 200. As shown in Fig. 17, the T cell product selection part 200 includes processor 201, memory 202, auxiliary storage device 203, I/F (Interface) device 204, communication device 205, and drive device 206. The hardware of the T cell product selection part 200 are connected to each other via bus 207.

The processor 201 has various arithmetic units such as CPU (Central Processing Unit) and GPU (Graphics Processing Unit). The processor 201 reads and executes programs such as various software installed in the auxiliary storage device 203 on the memory 202.

The memory 202 has a main storage device such as ROM (Read Only Memory) and RAM (Random Access Memory). The processor 201 and memory 202 form a computer, and the processor 201 realizes various functions by executing programs such as various software read on the memory 202.

The auxiliary storage device 203 stores programs such as various software, various information (e.g., T cell-related information, test subject information, etc.) and data used when the programs such as various software are executed by the processor 201.

The I/F device 204 is a connection device that connects an operation device 210 and a display device 211 to the T cell product selection part 200. The I/F device 204 receives various instructions to the T cell product selection part 200 via the operating device 210. In addition, the I/F device 204 outputs the processing result by the T cell product selection part 200 via the display device 211.

The communication device 205 is a communication device for communicating with other device via the network.

The drive device 206 is a device for setting the recording medium 212. The recording medium 212 here includes a medium such as CD-ROM, flexible disk, magneto-optical disk, or the like that optically, electrically, or magnetically records information. In addition, the recording medium 212 may include a semiconductor memory or the like for electrically recording information such as ROM or flash memory.

The programs such as various software to be installed in the auxiliary storage device 203 are installed by, for example, setting the distributed recording medium 212 in the drive device 206, and reading the programs such as various software recorded in the recording medium 212 by the drive device 206. Alternatively, programs such as various software installed in the auxiliary storage device 203 may be installed by being downloaded from the network via the communication device 205.

### 1-10. Use of T cell product

The T cell product provided by the provision system of the present invention may exhibit cytotoxic activity against cancer cell, cancer stem cell, tumor cell and the like, and can therefore be used for the prevention or treatment of cancer or tumor and can be administered to the test subject. In the present specification, the test subject means a mammal (e.g., mouse, rat, hamster, rabbit, cat, dog, bovine, sheep, monkey, human) to which a T cell product is administered in a clinical trial or the like. The test subject is preferably human.

Cancers and tumors that are prevented or treated by the above-mentioned T cell products are described, for example, in "Daniel Baumhoer et al., Am J. Clin Pathol, 2008, 129, 899-906" and the like. Specific examples thereof include, but are not limited to, liver cancer (e.g., hepatoma), ovarian cancer (e.g., ovary clear cell adenocarcinoma), childhood cancer, lung cancer (e.g., squamous cell carcinoma, small cell lung cancer), testis cancer (e.g., nonseminomas germ cell tumor), soft tissue tumor (e.g., liposarcoma, malignant fibrous histiocytoma), uterine cancer (e.g., cervix intraepithelial tumor, cervix squamous cell carcinoma), melanoma, adrenal gland tumor (e.g., adrenal gland adenoma), neurotic tumor (e.g., schwannoma), gastric cancer (e.g., adenocarcinoma of stomach), renal cancer (e.g., Grawitz tumor), breast cancer (e.g., invasive lobular carcinoma, mucous cancer), thyroid cancer (e.g., medullar cancer), laryngeal cancer (e.g., squamous cell carcinoma), urinary bladder cancer (e.g., invasive transitional cell carcinoma) and the like.

The T cells to be contained in the above-mentioned T cell product may be cultured and/or stimulated using an appropriate medium and/or a stimulating molecule before administration to a test subject. Examples of the stimulating molecule include, but are not limited to, cytokines, suitable protein, other components and the like. Examples of the cytokines include IL-2, IL-7, IL-12, IL-15, IFN-y and the like, and IL-2 can be preferably used. While the concentration of IL-2 in the medium is not particularly limited, for example, it is preferably 0.01 - 1×10⁵ U/mL, more preferably 1 - 1×10⁴ U/mL. Examples of the suitable protein include CD3 ligand, CD28 ligand, anti-CD3 antibody, anti-CD30 antibody, and anti-IL-4 antibody. Besides these, a lymphocyte stimulating factor such as lectin and the like can also be added. Furthermore, serum or plasma may be added to the medium. While the amount of addition to these media is not particularly limited, 0% by volume - 20% by volume can be mentioned. In addition, the amount of serum or plasma to be used can be changed according to the culturing stage. For example, serum or plasma concentration can be reduced stepwise. The origin of serum or plasma may be either autologous or allogeneic, and autologous one is preferable from the aspect of safety.

The above-mentioned T cells are preferably used by parenteral administration to the test subject. Examples of the method for parenteral administration include intravenous, intraarterial, intramuscular, intraperitoneal, and subcutaneous administration and the like. While the dose is appropriately selected according to the condition, body weight, age and the like of the test subject, the medicament is generally administered such that the cell number is generally 1×10⁶ - 1×10¹⁰ cells, preferably 1×10⁷ - 1×10⁹ cells, more preferably 5×10⁷ - 5×10⁸ cells, per dose to a test subject with body weight 60 kg. The T cells may be administered once or in plural portions.

### 2. Construction method of master cell bank of T cell and/or working cell bank of T cell

The present invention also provides a method for constructing the above-mentioned master cell bank of T cell and/or working cell bank of T cell (hereinafter sometimes referred to as "the cell bank construction method of the present invention"). In one embodiment of the present invention, when the above-mentioned exogenous gene is CAR gene, the cell bank construction method of the present invention includes (I) a process of differentiating an induced pluripotent stem cell free of a chimeric antigen receptor (CAR) gene into a T cell for CAR-T therapy, (II) a process of stocking the differentiated T cells, and (III) a process of characterisation of the differentiated T cells. While the above-mentioned processes (II) and (III) are generally performed in this order, process (II) may be performed after process (III). In another embodiment, when the above-mentioned exogenous gene is TCR gene, the cell bank construction method of the present invention includes (i) a process of differentiating an induced pluripotent stem cell free of a TCR gene into a T cell for TCR-T therapy, (ii) a process of stocking the differentiated T cells, and (iii) a process of characterisation of the differentiated T cells. While the above-mentioned processes (ii) and (iii) are generally performed in this order, process (ii) may be performed after process (iii). Each of the above-mentioned processes may be performed by the same entity or different entities.

In another embodiment of the present invention, a master cell bank of T cell and/or a working cell bank of T cell constructed by the cell bank construction method of the present invention (hereinafter sometimes referred to as "the cell bank of T cell of the present invention") is provided.

### 2-1. Differentiation induction of induced pluripotent stem cell free of CAR gene or exogenous TCR gene into T cell

The processes (I) and (i) of the above-mentioned 2. can be performed using induced pluripotent stem cell free of CAR gene and/or exogenous TCR gene and according to the method described in processes (1) and (2) of the above-mentioned 1-1. For CAR-T therapy in process (I) means use for the treatment or prophylaxis with CAR expressed in T cells, and excludes treatment or prophylaxis with exogenous TCR and endogenous TCR expressed in T cells. The T cell may contain an exogenous TCR gene introduced for purposes other than therapeutic or preventive purposes, for example, the purpose of suppressing the expression of endogenous TCR gene and for the purpose of efficiently producing uniform T cells, described in the above-mentioned 1-1. Similarly, for TCR-T therapy in process (i) means use for the treatment or prophylaxis with exogenous TCR expressed in T cells, and excludes treatment or prophylaxis with exogenous CAR and endogenous TCR expressed in T cells. The T cell may contain an exogenous TCR gene introduced for purposes other than therapeutic or preventive purposes, for example, the purpose of suppressing the expression of endogenous TCR gene and the like, described in the above-mentioned 1-1.

The description of CAR and TCR in the above-mentioned processes (I) and (i), specific examples of the antigen targeted by TCR and CAR, definition of each term such as T cell and the like, and the like are as described in the above-mentioned 1. and 1-1. Specific examples of the T cell obtained by differentiation induction in the above-mentioned process are as described in the above-mentioned 1.. The T cell is preferably cytotoxic T cell, more preferably CD8αβ-positive cytotoxic T cell that expresses CD8αβ.

### 2-2. Stock of differentiated T cells

The processes (III) and (iii) in the above-mentioned 2. can be performed using T cells obtained in the above-mentioned 2-1., and according to the method described in the above-mentioned 1-1. and 1-2.

The induced pluripotent stem cells to be used for the cell bank construction method of the present invention and the cell bank of T cell of the present invention can be established by the method described in the above-mentioned 1-1. The T cell to be used for the aforementioned induced pluripotent stem cell or the processes (II) and (ii) of the above-mentioned 2. is preferably a T cell showing a suppressed expression of at least one kind of HLA gene as in the cell described in the above-mentioned 1-1. Specific HLA gene and combination, the definition of suppression of the gene expression and a suppression method, and the like are as described in the above-mentioned 1-1.

### 2-3. Characterisation of T cell

The test items for characterisation in processes (III) and (iii) in the above-mentioned 2. vary depending on the biological properties (e.g., auxotrophy), culture history, and test feasibility of the cells to be the subject of the cell bank, and are appropriately determined by those of ordinary skill in the art, or based on the content of discussions between those of ordinary skill in the art and regulatory authorities, and the like. Information relating to the construction method thereof and the results of characterisation and quality evaluation are presented to the pharmaceutical authorities when applying for the manufacturing and marketing approval in each country. The characterisation of a cell bank of T cell mainly evaluates the absence of contamination with an exogenous infectious factor. In particular, since contamination with exogenous virus can lead to serious consequences in clinical use, it is thoroughly evaluated according to ICH Q5A(R1). Other tests include identity test to detect cross contamination with other cell line. In addition, karyotype analysis and tumorigenicity test may also be performed.

The test items of quality evaluation include confirmation test using appropriate cell phenotype as T cell, purity test, production process-derived impurity test, and quantitative tests such as cell number, cell survival rate and the like.

### 2-4. Construction of cell bank collection of T cell

The provision system of the present invention may include a step of constructing a master cell bank collection of T cell and/or a working cell bank collection of T cell containing two or more kinds of master cell banks of T cell and/or working cell banks of T cell (hereinafter to be also referred to as "a cell bank collection of T cell") by collecting master cell banks of T cell and/or a working cell banks of T cell (hereinafter to be also referred to as "a cell bank collection of T cell construction step"). In the present specification, the part constituted to execute the cell bank collection of T cell construction step is sometimes referred to as a "cell bank collection of T cell construction part".

When a cell bank of T cell that is used for providing one or more T cell products may not be sufficient, or when it is appropriate to use a T cell product from a different type of cell bank of T cell depending on the characteristics of the test subject of administration, a separately-constructed cell bank of T cell can be added to an existing cell bank of T cell. By constructing a cell bank collection of T cell by collecting from plural cell banks of T cell, a more stable and robust T cell product provision system can be provided.

The cell bank collection of T cell construction step can also be performed by a known method. Such method includes construction by producing two or more kinds of the cell banks of T cell by the above-mentioned cell bank construction method of the present invention, attaching a label or the like so that the kind of the cell bank of T cell can be distinguished, and storing the products. The kind of the collection can also be increased by further adding a different kind of a cell bank of T cell to the constructed cell bank of T cell. For easy storage, it is preferable to store in the same space (e.g., in the same freezer, in the same room, in the same building, on the same site, etc.); however, it may be stored in a physically separate space. Any method may be used to distinguish the kind of a cell bank of T cell as long as the kind can be distinguished. For example, a label and the like may be attached to the cryopreservation container, or when stored in other freezer or cryoprotective system, a label and the like may be attached to the freezer or cryoprotective system, or management software and the like may be used to distinguish the products and the like.

Therefore, the cell bank collection of T cell-construction part may be provided with at least cell bank of T cell to be collected, and constituted to construct a cell bank collection of T cell including two or more kinds of cell banks of T cell by collecting cell banks of T cell constructed as mentioned above. In addition, the cell bank collection of T cell-construction part may be provided, as necessary, with one or more kinds of label, freezer, cryoprotective system, management software and the like. Specific examples of the freezer are as described above. The construction of a cell bank collection of T cell may be performed by the same entity as the entity constructing a cell bank of T cell or different entities. The cell bank collection of T cell may contain a cell bank of T cell constructed by a different entity.

### 2-4-1. Selection of cell bank of T cell

The provision system of the present invention may include a step of selecting a cell bank of T cell for producing a T cell product to be provided from a cell bank collection of T cell containing cell banks of T cell (hereinafter to be also referred to as "a cell bank of T cell selection step"). In the present specification, the part constituted to execute the selection step of a cell bank of T cell is sometimes referred to as a "cell bank of T cell selection part".

The selection step of the cell bank of T cell can be performed by a known method. According to the method, a cell bank of T cell which contains T cell that expresses an appropriate T cell receptor can be selected based on the contents of the production plan of the T cell product to be provided.

In one embodiment of the present invention, information of the test subject is obtained, and an appropriate cell bank of T cell can be selected based on the information. Therefore, the provision system of the present invention may include a step of obtaining information of the test subject (hereinafter to be also referred to as "test subject information obtaining step") and/or a step of selecting a cell bank of T cell suitable for the test subject. In the following, the part constituted to execute the test subject information obtaining step is sometimes referred to as a "test subject information obtaining part".

The information of the test subject is not particularly limited and includes, for example, genetic information (e.g., HLA gene-related information, abnormal gene information, etc.), diagnosis result, history, drug administration history, age, gender, blood type, height, body weight, and when the test subject has a tumor, information of tumor-specific or tumor-associated antigen expressed in the tumor, of the test subject.

### 3. Method for producing T cell product

In another embodiment, the present invention provides a method for producing a T cell product (hereinafter sometimes referred to as "the production method of T cell product of the present invention"). The method includes (A) a process of preparing a T cell from the cell bank of T cell of the present invention, (B) a process of introducing CAR gene or exogenous TCR gene into the prepared T cell, and (C) a process of expansion culturing the T cell into which the CAR gene or exogenous TCR gene has been introduced. The production method of a T cell product of the present invention may further include (D) a process of freezing the expansion cultured T cells. In addition, a T cell product produced by the production method of a T cell product of the present invention (hereinafter sometimes referred to as "the T cell product of the present invention") is also provided. Each of the above-mentioned processes may be performed by the same entity or by different entities.

### 3-1. Preparation of T cell

Process (A) of the above-mentioned 3. can be performed using the cell bank of T cell of the present invention and according to the method described in the above-mentioned 1-3.

### 3-2. Introduction of nucleic acid

The process (B) of the above-mentioned 3. can be performed using the T cells prepared by the method of the above-mentioned 3-1. and according to the method described in the above-mentioned 1-4. T cells used in process (B) preferably express IL-15/IL-15Rα. The kind of the nucleic acid, explanations on CAR, TCR and IL-15/IL-15Rα, definition of each term, specific examples of the antigen targeted by TCR and CAR, and the like are as described in the above-mentioned 1-1. Thus, in one embodiment, T cells contained in the production method of the frozen stock of the present invention, or T cells contained in the T cell frozen stock of the present invention may be characterized in that the aforementioned CAR or exogenous TCR recognizes and binds to tumor-specific antigen or tumor-associated antigen of the above-mentioned 1-1.

### 3-3. Expansion culture

The process (C) of the above-mentioned 3. can be performed using the T cells into which a nucleic acid has been introduced by the method of the above-mentioned 3-2. and according to the method described in the above-mentioned 1-5. The method of expansion culture, specific examples of the compound used in the expansion culture, concentration of the compound in the medium, definition of each term and the like are as described in the above-mentioned 1-5. In one embodiment, therefore, the production method of the frozen stock of the present invention may include a process of culturing the aforementioned T cells in the presence of a CD30 agonist.

### 3-4. Freezing of T cell

The process (D) of the above-mentioned 3. can be performed using the T cells expansion cultured by the method the above-mentioned 3-3. and according to the method described in the above-mentioned 1-2.

### 4. Construction method of T cell product collection

In another embodiment, the present invention provides a method for constructing a T cell product collection containing two or more kinds of T cell products (hereinafter sometimes referred to as "construction method of T cell product collection of the present invention"), and the method may include a process of collecting the T cell product of the present invention. In addition, a T cell product collection constructed by the construction method of the T cell product collection of the present invention (hereinafter sometimes referred to as "the T cell product collection of the present invention") is also provided. The T cell product constituting the T cell product collection of the present invention may be a frozen T cell product. From the aspect of the stability of T cells, a frozen T cell product is preferable.

The process of collecting the T cell product of the present invention can be performed using the T cell product of the present invention and according to the method described in the above-mentioned 1-7. The T cell product contained in the T cell product collection may be produced by the same entity as the entity that constructs the T cell product collection, or produced by a different entity. Therefore, the T cell product collection may contain a T cell product produced by a different entity.

### 5. Production method of T cell product

The present invention also provides a method for producing a T cell product (hereinafter sometimes referred to as "the production method of the T cell product of the present invention"), and the method may include a process of constructing a master cell bank of T cell and/or a working cell bank of T cell.

Specific examples of T cell, T cell product, master cell bank, working cell bank and the like and the definition of the terms are as described in the above-mentioned 1-1. In addition, the above-mentioned pluripotent stem cell, T cell, T cell product, master cell bank and working cell bank can be produced and constructed by, for example, the method described in the above-mentioned 1. and 2., and the like. The production method of the T cell product of the present invention may include (a) a process of differentiating pluripotent stem cell into T cell, (b) a process of stocking the differentiated T cell, (c) a process of characterisation of the differentiated T cell, (d) a process of preparing T cell from the cell stock, (e) a process of introducing a nucleic acid containing the exogenous gene into a T cell, and/or (f) a process of expansion culturing a T cell. In addition, the T cell to be used in the production method of the T cell product of the present invention may be a T cell in which the expression of at least one kind of HLA gene is suppressed. Specific HLA gene and combination, the definition of suppression of the gene expression and a suppression method and the like are as described in the above-mentioned 1-1. Each of the above-mentioned processes may be performed by the same entity or by different entities.

The process (a) of the above-mentioned 5. can be performed using the pluripotent stem cell described in the above-mentioned 1-1. and according to the method described in the processes (1) and (2) of the above-mentioned 1-1. The above-mentioned pluripotent stem cell is preferably an induced pluripotent stem cell, more preferably a human induced pluripotent stem cell. The induced pluripotent stem cells to be used for the cell bank construction method of the present invention and the cell bank of T cell of the present invention can be established by the method described in the above-mentioned 1-1. The process (b) of the above-mentioned 5. can be performed using the T cells differentiated in the above-mentioned process (1) and according to the method described in the above-mentioned 1-1. and 1-2. The process (c) of the above-mentioned 5. can be performed using the T cells differentiated by the above-mentioned process (1) and according to the method described in the above-mentioned 2-2. The above-mentioned process (d) can be performed using the cell stock produced in the above-mentioned process (c) and according to the method described in the above-mentioned 1-3.

The process (e) of the above-mentioned 5. can be performed using the T cells differentiated by the aforementioned process (a), the cells stocked in the aforementioned process (b), the T cells subjected to the property analysis in the aforementioned process (c), or T cells prepared by the aforementioned process (d) and according to the method described in the above-mentioned 1-4. T cells used in process (e) preferably express IL-15/IL-15Rα. The kind of the nucleic acid, explanations on CAR, TCR and IL-15/IL-15Rα, definition of each term, specific examples of the antigen targeted by TCR and CAR, and the like are as described in the above-mentioned 1-1. Thus, in one embodiment, T cells contained in the production method of the frozen stock of the present invention, or T cells contained in the T cell frozen stock of the present invention may be characterized in that the aforementioned CAR or exogenous TCR recognizes and binds to tumor-specific antigen or tumor-associated antigen of the above-mentioned 1-1.

The process (f) of the above-mentioned 5. can be performed using the T cells into which a nucleic acid has been introduced by the aforementioned process (e) and according to the method described in the above-mentioned 1-5. The method of expansion culture, specific examples of the compound used in the expansion culture, concentration of the compound in the medium, definition of each term and the like are as described in the above-mentioned 1-5. In one embodiment, therefore, the production method of the T cell product of the present invention may include a process of stimulating a T cell by culturing the cell in the presence of a CD30 agonist.

### 6. Provision method of T cell product

Furthermore, the present invention provides a method for providing a T cell product suitable for a test subject (hereinafter sometimes referred to as "the provision method of the present invention"). To provide a T cell product suitable for a test subject, information of the test subject is obtained and an appropriate cell bank of T cell and/or an appropriate T cell product can be selected based on the information. Therefore, the provision method of the present invention includes (p) a process of obtaining information of a test subject, and (q) a process of selecting an appropriate cell bank of T cell and/or an appropriate T cell product based on the obtained information of the test subject. Using the selected cell bank of T cell, process (e) and/or process (f) in the above-mentioned 5. are/is performed to produce a T cell product and the T cell product can be provided to the test subject. According to the provision method of the present invention, when a test subject has a tumor, information relating to a tumor-specific antigen or tumor-associated antigen expressed in the tumor is obtained, and an appropriate T cell product can be selected based thereon. Therefore, the provision method of the present invention includes (x) a process of identifying a tumor-specific antigen or a tumor-associated antigen expressed in the tumor of a test subject, and (y) a process of selecting a T cell product that expresses a CAR or an exogenous TCR that recognizes and binds to the identified antigen from the T cell product collection of the present invention. The (x) a process of identifying a tumor-specific antigen or a tumor-associated antigen expressed in the tumor of a test subject, and (y) a process of selecting a T cell product that expresses a CAR or an exogenous TCR that recognizes and binds to the identified antigen from the T cell product collection of the present invention are conceptually encompassed in (p) a process of obtaining information of a test subject, and (q) a process of selecting an appropriate T cell product based on the obtained information of the test subject. The kind and definition of the terms such as T cell, T cell product, test subject and the like are as described in the above-mentioned 1-1. to 1-10. Each of the above-mentioned processes may be performed by the same entity or by different entities.

The process (x) of the above-mentioned 6. can be performed using the biological sample of the test subject and according to the method described in the above-mentioned 1-8. Specific examples of the biological sample of the test subject are as described in the above-mentioned 1-8.

The process (y) of the above-mentioned 6. can be performed based on the identification results of the aforementioned process (x) and according to the method described in the above-mentioned 1-9. The explanation and definition of CAR and TCR, specific examples of tumor-specific antigen and tumor-associated antigen, specific examples of the evaluation criteria and the like are as described in the above-mentioned 1-1. and 1-9.

Specific examples of the cancer and tumor to be prevented or treated with a T cell product provided by the provision method of the present invention are as described in the above-mentioned 1-10. In addition, the culture and/or stimulation method of T cells contained in the above-mentioned T cell product, administration route, dose, the type of test subject and the like are also as described in the above-mentioned 1-1-10.

The present invention is more specifically explained by the following Examples. The scope of the present invention is not limited by the Examples.

### [Example]

### [Example 1]

### 1. Preparation of iPS cell

As the iPS cell, iPS cells (Ff-I01s04 strain: derived from peripheral blood mononuclear cell of healthy individual) provided by the Center for iPS Cell Research and Application (CiRA), Kyoto University, was used. iPS cell culture was performed according to the protocol distributed by CiRA, "Human iPS cell culture under feeder-free conditions".

### 2. Introduction of T cell receptor (TCR) gene into iPS cell

### 2-1. Introduction of WT1-TCR gene

A gene in which TAK1-derived TRB gene and TRA gene encoding HLA-A*24:02-restricted WT1 specific TCR supplied by Professor Masataka Yasukawa, Graduate School of Medicine, Ehime University, are linked by a P2A sequence (WT1-TCR) was introduced into iPS cells. Gene transfer into iPS cells was performed by incorporation into CS-UbC-RfA-IRES2-hKO1 lentivirus vector supplied by Inst. of Physical and Chemical Research and infecting the iPS cells. In the following, iPS cells into which the WT1-TCR gene has been introduced are sometimes referred to as "WT1αβ-iPSC".

### 2-2. Introduction of Vγ9Vδ2-TCR gene

A gene (Vγ9Vδ2TCR G115) in which the TRG gene and TRD gene encoding G115 γδ T cell clone-derived Vγ9Vδ2 T cell receptor were linked by a P2A sequence was introduced into iPS cells. Vγ9Vδ2TCR G115 is an artificially-synthesized oligo DNA encoding a polypeptide (SEQ ID NO: 1) designed to align in the order shown in Table 1 from the N-terminal.

**[Table 1]**

| order from N-terminal | gene | amino acid number |
|---|---|---|
| 1 | (G115derived from) TRG | 315 |
| 2 | P2A | 22 |
| 3 | (G115derived from) TRD | 292 |

A sequence encoding neomycin resistance gene was removed from pLVSIN-CMV Neo (Clontech), and a lentiviral vector using pLVSIN-Ub in which CMV promoter was replaced with human ubiquitin promoter was prepared. An artificial oligo DNA encoding Vγ9Vδ2TCR G115 synthesized above was incorporated into the multicloning site of pLVSIN-Ub lentivirus vector. Using the plasmid and Lenti-X^{™} 293T cell line (Clontech) and Lenti-X^{™} Packaging Single Shots (VSV-G), a lentiviral vector was prepared. The prepared lentivirus vector was used to infect the iPS cells prepared in [Example 1], 1, whereby the Vγ9Vδ2-TCR gene was introduced into the iPS cells. In the following, iPS cells into which the Vγ9Vδ2TCR G115 gene has been introduced are sometimes referred to as "Vγ9Vδ2-iPSC".

### [Example 2]

### 1. Differentiation of iPS cell into hematopoietic progenitor cell (HPC)

For differentiation of iPS cell into hematopoietic progenitor cell (HPC), a suspended cell population differentiated according to a known method (e.g., methods described in Cell Reports 2(2012)1722-1735 and WO 2017/221975) was used. Specifically, iPS cell, WT1αβ -iPSC and Vγ9Vδ2-iPSC obtained in [Example 1] 1., [Example 1] 2-1. and [Example 1] 2-2. were respectively seeded at 3 × 10⁵ cells/well in an ultra-low adhesion-treated 6 well plate, 10 ng/ml BMP4, 50 ng/ml bFGF, 15 ng/ml VEGF, 2 µM SB431542 were added to EB medium (StemPro34 added with 10 µg/ml human insulin, 5.5 µg/ml human transferrin, 5 ng/ml sodium selenit, 2 mM L-glutamine, 45 mM α-monothioglycerol, and 50 µg/ml ascorbic acid 2-phosphate), and the cells were cultured for 5 days under low-oxygen conditions (5% O₂). Then, 50 ng/ml SCF, 30 ng/ml TPO, 10 ng/ml Flt3L were added, and the cells were cultured for 5 - 9 days (- Day14) to give a suspended cell population. The medium was changed every two or three days during the culture period. The above-mentioned suspended cell population containing HPC was stained using the antibody set of Table 2. The cell populations that underwent the above-mentioned staining were subjected to sorting by FACSAria.

**[Table 2]**

| | |
|---|---|
| anti-CD34 antibody | Abcam PE/Cy7 |
| anti-CD43 antibody | BD APC |
| anti-CD45 antibody | BioLegend BV510 |
| anti-CD14 antibody | BioLegend APC/eFluor780 |
| anti-CD235a antibody | BD FITC |

### 2. Introduction of Vγ9Vδ2-TCR gene into HPC

A gene (Vγ9Vδ2TCR G115) in which the TRG gene and TRD gene encoding G115 γδ T cell clone-derived Vγ9Vδ2 T cell receptor were linked by a P2A sequence was introduced into the cell fraction obtained in [Example 2] 1. The gene was introduced in the same manner as in [Example 1] 2-2. In the following, HPC cell in which Vγ9Vδ2TCR G115 gene has been introduced into HPC is sometimes referred to as "Vγ9Vδ2-iHPC".

### [Example 3]

### 1. Differentiation of HPC into T cell

The cell fraction obtained in [Example 2] 1. and Vγ9Vδ2-iHPC obtained in [Example 2] 2. were differentiated into lymphoid cells according to a known method (e.g., the methods described in Journal of Leukocyte Biology 96(2016)1165-1175 and WO 2017/221975). To be specific, the hematopoietic progenitor cell population was seeded at 2000 cells/well in a 48-well-plate coated with Recombinant h-DLL4/Fc chimeric protein (Sino Biological) and Retronectin (Takara Bio Inc.) and cultured under 5% CO₂, 37°C conditions. The medium was changed every two or three days during the culture period. As the medium, αMEM medium added with 15% FBS, 2 mM L-glutamine, 100 U/ml penicillin, 100 ng/ml streptomycin, 55 µM 2-mercaptoethanol, 50 µg/ml ascorbic acid 2-phosphate, 10 µg/ml human insulin, 5.5 µg/ml human transferrin, 5 ng/ml sodium selenite, 50 ng/ml SCF, 50 ng/ml IL-7, 50 ng/ml Flt3L, 100 ng/ml TPO, 15 µM SB203580, 30 ng/ml SDF-1α was used. The cells were passaged to a similarly-coated 48-well plate on day 7 and day 14 from the start of the culturing. All cells were recovered on day 21 from the start of the culturing and the presence of CD45(+), CD3(+) fractions was confirmed by a flow cytometer. The obtained cells were seeded in a 24-well plate and cultured under 5% CO₂, 37°C conditions. As the medium, αMEM medium added with 15% FBS, 2 mM L-glutamine, 100 U/ml penicillin, 100 ng/ml streptomycin, 50 ng/ml ascorbic acid 2-phosphate, 10 µg/ml human insulin, 5.5 µg/ml human transferrin, 5 ng/mL sodium selenite, 500 ng/mL anti-CD3 antibody (UCHT1 or OKT3 clone), 10 nM dexamethasone (Fuji Pharma: 10171-H02H), 100 U/ml IL-2, 10 ng/mL IL-7. All cells were recovered on day 27 from the start of the culturing (Day41). In the following, a T cell differentiated from the iPS cell obtained in [Example 1] 1. is sometimes referred to as "iγδTC", a T cell differentiated from WT1αβ-iPSC obtained in [Example 1] 2-1., is sometimes referred to as "iWT1αβPTC", a T cell differentiated from Vγ9Vδ2-iPSC obtained in [Example 1] 2-2., is sometimes referred to as "Vγ9Vδ2-iTC", and a T cell differentiated from Vγ9Vδ2-iHPC obtained in [Example 1] 2., is sometimes referred to as "Vγ9Vδ2-iHTC".

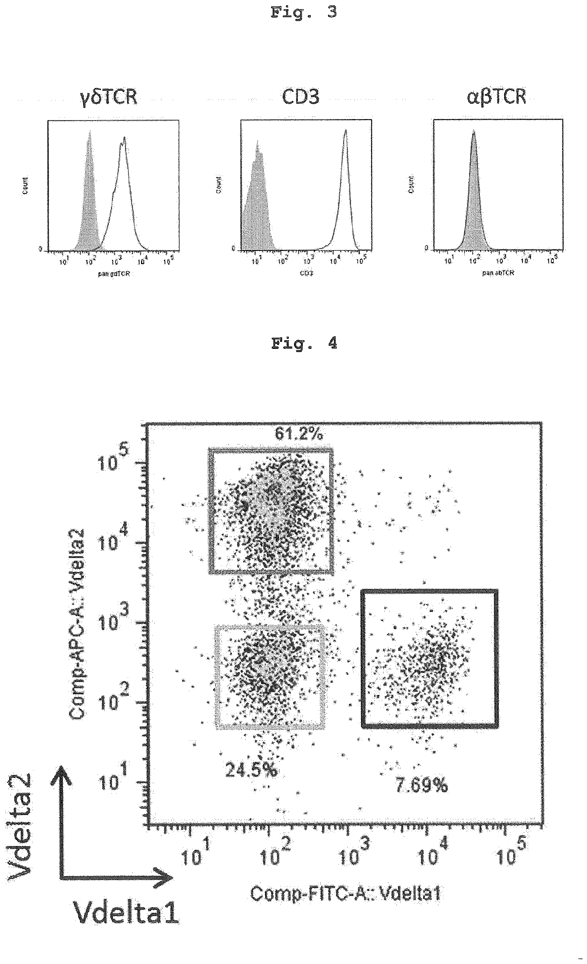

iγδTC was stained using the antibody set shown in Table 3 (Figs. 3 and 4).

**[Table 3]**

| |
|---|
| V*δ*1 Myltenyi FITC |
| V*δ*2 Myltenyi APC |
| *γ δ* TCR BD BV510 |
| CD3 BioLegend APC/Cy7 |
| *αβ*TCR eBioscience FITC |

As regards iγδTC, the expression of CD3, VδTCR, αβTCR, TCR-Vδ1 chain, and TCR-Vδ2 chain on the surface of cell membrane was measure by a flow cytometer (Figs. 3 and 4).

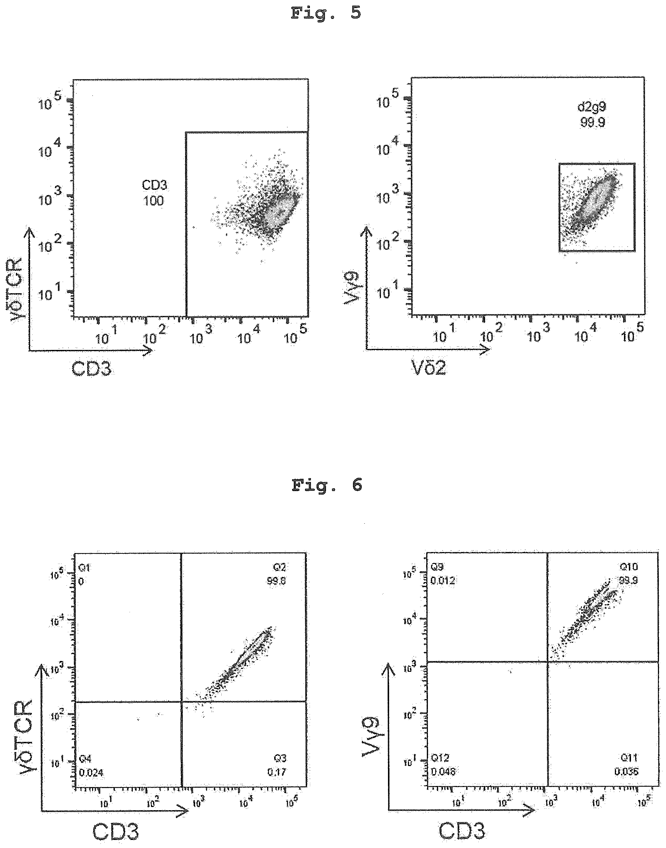

As regards Vγ9Vδ2-iTC (Fig. 5) and Vγ9Vδ2-iHTC (Fig. 6), the expression of CD3, VδTCR, TCR-Vδ1 chain and TCR-Vδ2 chain on the surface of cell membrane was measure by a flow cytometer.

### [Example 4]

### Expansion culture of T cell

### 1. Expansion culture of iγδTC

### 1-1. Expansion culture

iγδTC obtained in [Example 3] was suspended at 2,000,000 cells/mL in a medium obtained by adding cytokine shown in Table 4 to α-MEM medium containing 15% FBS, and the suspension was seeded in a plate containing anti-CD3 antibody (UCHT1) and RetroNectin immobilized thereon and cultured at 5% CO₂/37°C for 3 days. The cells were harvested from the plate on day 3 of culture and the number of the cells was measured using NucleoCounter (registered trade mark) NC-200 (ChemoMetec). The cells were suspended in an appropriate amount of a medium obtained by adding cytokine shown in Table 5 to α-MEM medium containing 15% FBS, added to a non-solidified G-Rex (registered trade mark) 6-well plate (WILSONWOLF) and cultured at 5%CO₂/37°C. Thereafter, a part of the cells were collected from the plate 4 to 6 times on any of days 5, 6, 7, 8, 9, 10, 11, 14 of culture, and the number of cells was measured using NucleoCounter (registered trade mark) NC-200. The cells were immobilized on the culture plate with anti-CD3 antibody and RetroNectin by the following method. An anti-CD3 antibody (UCHT1, final concentration 3000 ng/mL) and RetroNectin (final concentration 150 µg/mL) dissolved in PBS at necessary concentrations were added to the plate and the plate was stood overnight at 4°C.

### 1-2. Proliferation rate

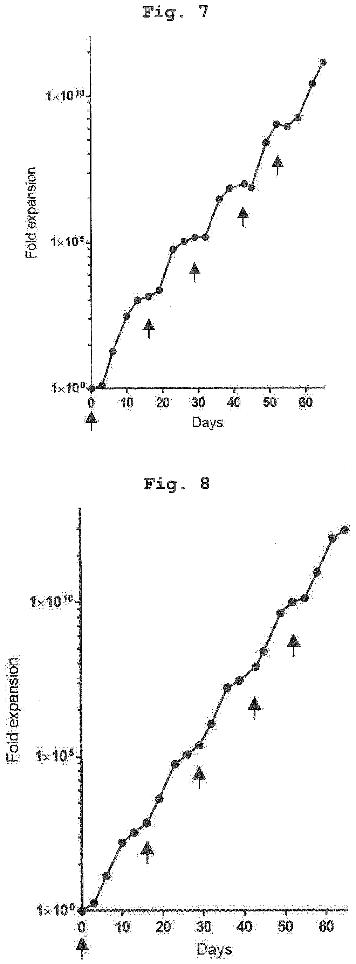

The proliferation rate of the number of iγδTC cells when the expansion culture of [Example 4] 1-1., was repeated 5 times is shown in Fig. 7. iγδTC can be expansion cultured at least not less than 10¹¹ times, and can be used as T cells constituting the master cell bank of T cell and/or working cell bank of T cell.

**[Table 4]**

| product name | manufacturer | Final conc |
|---|---|---|
| Insulin-Transferrin-Selenium Supplements | Invitrogen | 1 x |
| Ascorbic acid 2-phosphate | sigma | 50 µg/ml |
| IL-2 | Peprotech | 15 ng/ml |
| IL-7 | Peprotech | 10 ng/ml |
| IL-15 | Peprotech | 10 ng/ml |
| IL-21 | Peprotech | 20 ng/ml |
| IL-12 | Merck | 50 ng/ml |
| IL-18 | MBL | 50 ng/ml |
| TL-1A | Peprotech | 50 ng/ml |
| Z-VAD-FMK | R&D | 10 µM |
| Human CD30 Antibody | R&D | 300 ng/ml |

**[Table 5]**

| product name | manufacturer | Final conc |
|---|---|---|
| Insulin-Transferrin-Selenium Supplements | Invitrogen | 1 x |
| Ascorbic acid 2-phosphate | sigma | 50 µg/ml |
| IL-2 | Peprotech | 15 ng/ml |
| IL-7 | Peprotech | 10 ng/ml |
| IL-15 | Peprotech | 10 ng/ml |

### 2. Expansion culture of Vγ9Vδ2-iTC

### 2-1. Expansion culture

Vγ9Vδ2-iTC obtained in [Example 3] was suspended at 2,000,000 cells/mL in a medium obtained by adding cytokine shown in Table 6 to α-MEM medium containing 15% FBS, and the suspension was seeded in a plate containing anti-CD3 antibody (OKT3) and RetroNectin immobilized thereon and cultured at 5% CO₂/37°C for 3 days. The cells were harvested from the plate on day 3 of culture and the number of the cells was measured using NucleoCounter (registered trade mark) NC-200 (ChemoMetec). The cells were suspended in an appropriate amount of a medium obtained by adding cytokine shown in Table 5 to α-MEM medium containing 15% FBS, added to a non-solidified G-Rex (registered trade mark) 6-well plate (WILSONWOLF) and cultured at 5%CO₂/37°C. Thereafter, a part of the cells were collected from the plate 4 to 6 times on any of days 5, 6, 7, 8, 9, 10, 11, 14 of culture, and the number of cells was measured using NucleoCounter (registered trade mark) NC-200. The cells were immobilized on the culture plate with anti-CD3 antibody and RetroNectin by the method of [Example 4] 1-1.

### 2-2. Proliferation rate

The proliferation rate of the number of Vγ9Vδ2-iTC cells when [Example 4] 2-1., was repeated 5 times is shown in Fig. 8. Vγ9Vδ2-iTC can be expansion cultured at least not less than 10¹² times, and can be used as T cells constituting the master cell bank of T cell and/or working cell bank of T cell.

**[Table 6]**

| product name | manufacturer | Final conc |
|---|---|---|
| Insulin-Transferrin-Selenium Supplements | Invitrogen | 1 x |
| Ascorbic acid 2-phosphate | sigma | 50 µg/ml |
| IL-2 | Peprotech | 15 ng/ml |
| IL-7 | Peprotech | 10 ng/ml |
| IL-15 | Peprotech | 10 ng/ml |
| IL-21 | Peprotech | 20 ng/ml |
| IL-12 | Merck | 50 ng/ml |
| IL-18 | MBL | 50 ng/ml |
| TL-1A | Peprotech | 50 ng/ml |
| Z-VAD-FMK | R&D | 10 µM |

### 3. Expansion culture of WT1αβ-iTC

### 3-1. Expansion culture

iWT1αβTC obtained in [Example 3] was suspended at 2,000,000 cells/mL in a medium obtained by adding cytokine shown in Table 6 to α-MEM medium containing 15% FBS, and the suspension was seeded in a plate containing anti-CD3 antibody (OKT3) and RetroNectin immobilized thereon and cultured at 5% CO₂/37°C for 3 days. The cells were harvested from the plate on day 3 of culture and the number of the cells was measured using NucleoCounter (registered trade mark) NC-200 (ChemoMetec). The cells were suspended in an appropriate amount of a medium obtained by adding cytokine shown in Table 5 to α-MEM medium containing 15% FBS, added to a non-solidified G-Rex (registered trade mark) 6-well plate (WILSONWOLF) and cultured at 5%CO₂/37°C. Thereafter, a part of the cells was collected from the plate 4 to 6 times on any of days 5, 6, 7, 8, 9, 10, 11, 14, 17 of culture, and the number of cells was measured using NucleoCounter (registered trade mark) NC-200. The cells were immobilized on the culture plate with anti-CD3 antibody and RetroNectin by the following method. An anti-CD3 antibody (OKT3, final concentration 3000 ng/mL) and RetroNectin (final concentration 150 µg/mL) dissolved in PBS at necessary concentrations were added to the plate and the plate was stood overnight at 4°C.

### 3-2. Proliferation rate

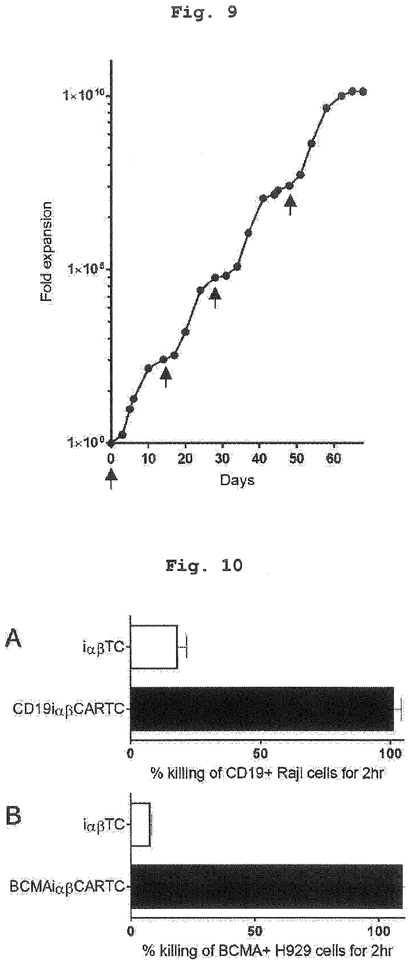

The proliferation rate of the number of iWT1αβTC cells when the expansion culture of [Example 4] 3-1., was repeated 4 times is shown in Fig. 9. iWT1αβTC can be expansion cultured at least not less than 10¹⁰ times, and can be used as T cells constituting the master cell bank of T cell and/or working cell bank of T cell.

### [Example 5]

### 1. Construction of master cell bank of T cell

The T cell culture medium obtained in [Example 4] is replaced with a cryoprotective solution (CryoStor CS10) containing a cryoprotective agent, and dispensed to a plurality of storage containers (sterile vials (polymer vials (AT-closed vial))). The T cells are frozen by standing the container containing the T cells in a freezer. A program freezer, CryoMed (Thermo Fisher), is used as the freezer. By stocking same, a frozen master cell bank of T cell is constructed.

### 2. Construction of working cell bank of T cell

A container containing T cells is recovered from the frozen master cell bank of T cell of [Example 5] 1. The container is immersed in a water bath at about 37°C for about 15 sec to thaw the T cells. The cells obtained by thawing are suspended in an appropriate amount of a medium containing cytokine shown in Table 5, added to a 6-well plate (WILSONWOLF) and cultured at 5%CO₂/37°C. After culturing for 3 days from day 1, T cells are expansion cultured by the method of [Example 4], thawed in the same manner as described above and stocked to construct a working cell bank of T cell.

### 3. Property analysis and quality evaluation

In the same manner as in [Example 5] 2., one container containing T cells is recovered from the master cell bank of T cell and/or working cell bank of T cell, and the T cells are thawed. The obtained T cells are subjected to property analysis and quality evaluation.

### 4. Construction of cell bank collection of T cell

Plural kinds of master cell banks of T cells obtained in [Example 5] 1., are collected, labeled so that the kind of master cell bank of T cell can be distinguished, and stored, whereby a master cell bank collection of T cell is constructed.

### [Example 6]

### 1. Preparation of T cell

In the same manner as in [Example 5] 2., one container was collected from master cell bank of T cell and/or working cell bank of T cell and T cells were thawed. The T cells obtained by thawing were suspended in an appropriate amount of a medium containing cytokine shown in Table 5, added to a 6-well plate, and cultured at 5%CO₂/37°C. After culturing for 3 days from day 1, the cells were suspended at 2,000,000 cells/mL in a medium obtained by adding cytokine shown in Table 4 or Table 6 to α-MEM medium containing 15% FBS, seeded in a plate containing anti-CD3 antibody (UCHT1 or OKT3) and RetroNectin immobilized thereon and cultured at 5% CO₂/37°C for 3 days. The cells were harvested from the plate on day 3 of culture and the number of the cells was measured using NucleoCounter (registered trade mark) NC-200 (ChemoMetec). The cells were suspended in an appropriate amount of a medium obtained by adding cytokine shown in Table 5 to α-MEM medium containing 15% FBS, added to a non-solidified cell culture flask (25 cm² or 75 cm²) and cultured at 5%CO₂/37°C for one day to prepare T cells.

### 2. Introduction of nucleic acid into T cell

### 2-1. Introduction of anti-CD19-CAR gene

As a gene encoding anti-CD19-CAR, an oligo DNA encoding a polypeptide (SEQ ID NO: 2) designed to align in the order shown in Table 7 from the N-terminal was artificially synthesized.

**[Table 7]**

| order from N-terminal | gene | amino acid number |
|---|---|---|
| 1 | lead sequence of immunoglobulin heavy chain | 22 |
| 2 | variable region of anti-CD19 antibody (FMC63) light chain | 104 |
| 3 | GGGGS linker | 15 |
| 4 | variable region of anti-CD19 antibody (FMC63) heavy chain | 120 |
| 5 | CD8-derived sequence (including transmembrane region) | 83 |
| 6 | intracellular domain region of CD28 | 41 |
| 7 | intracellular domain region of 4-1BB | 47 |
| 8 | intracellular domain region of CD3ζ | 112 |

The artificial oligo DNA synthesized in the above was incorporated into the multicloning site of pMEI-5 retrovirus vector. The production of the virus vector was outsourced to Unitech. iWT1αβTC produced in [Example 3] was infected with the produced retrovirus vector carrying a gene encoding anti-CD19-CAR, whereby iPS cell-derived anti-CD19-CART cells (hereinafter sometimes to be referred to as "CD19iWT1αβCARTC") was produced.

### 2-2. Introduction of anti-BCMA-CAR gene

As a gene encoding anti-BCMA-CAR, an oligo DNA encoding a polypeptide (SEQ ID NO: 3) designed to align in the order shown in Table 8 from the N-terminal was artificially synthesized.

**[Table 8]**

| order from N-terminal | gene | amino acid number |
|---|---|---|
| 1 | lead sequence of immunoglobulin heavy chain | 19 |
| 2 | variable region of anti-BCMA antibody light chain | 111 |
| 3 | linker | 18 |
| 4 | variable region of anti-BCMA-antibody weight chain | 117 |
| 5 | CD8-derived sequence (including transmembrane region) | 86 |
| 6 | intracellular domain region of CD28 | 41 |
| 7 | intracellular domain region of 4-1BB | 47 |
| 8 | intracellular domain region of CD3ζ | 112 |

The artificial oligo DNA synthesized in the above was incorporated into the multicloning site of pMEI-5 retrovirus vector. The production of the virus vector was outsourced to Unitech. iWT1αβTC produced in [Example 3] was infected with the produced retrovirus vector carrying a gene encoding anti-BCMA-CAR, whereby iPS cell-derived anti-BCMA-CART cells (hereinafter sometimes to be referred to as "BMCAiWT1αβCARTC") was produced.

### 2-3. Introduction of anti-CD19-CAR gene containing CD30-derived intracellular domain

As an anti-CD19-CAR gene containing CD30-derived intracellular domain, an oligo DNA encoding a polypeptide (SEQ ID NO: 4) designed to align in the order shown in Table 9 from the N-terminal was artificially synthesized.

**[Table 9]**

| order from N-terminal | gene | amino acid number |
|---|---|---|
| 1 | lead sequence of immunoglobulin heavy chain | 22 |
| 2 | variable region of anti-CD19 antibody (FMC60) light chain | 104 |
| 3 | GGGGS linker | 15 |
| 4 | variable region of anti-CD19 antibody (FMC60) heavy chain | 120 |
| 5 | CD8-derived sequence (including transmembrane region) | 83 |
| 6 | intracellular domain region of CD28 | 41 |
| 7 | intracellular domain region (SEQ ID NO: 6) of CD30 | 87 |
| 8 | intracellular domain region of CD3ζ | 112 |

An artificial oligo DNA synthesized above was incorporated into the multicloning site of pMY retrovirus vector. Using FLYRD18 cells for retrovirus vector production, a virus vector was produced. WT1αβ-iTC produced in [Example 1], 3., was infected with the produced retrovirus vector carrying an anti-CD19-CAR gene containing a CD30-derived intracellular domain, whereby iPS cell-derived anti-CD19-CART cell containing a CD30-derived intracellular domain (hereinafter sometimes to be referred to as "CD19-CD30-iWT1αβCARTC") was produced.

### 2-4. Introduction of anti-CD19-CAR gene

As a gene encoding anti-CD19-CAR, an oligo DNA encoding a polypeptide (SEQ ID NO: 5) designed to align in the order shown in Table 10 from the N-terminal was artificially synthesized.

**[Table 10]**

| order from N-terminal | gene | amino acid number |
|---|---|---|
| 1 | lead sequence of immunoglobulin heavy chain | 22 |
| 2 | variable region of anti-CD19 antibody (FMC63) light chain | 104 |
| 3 | GGGGS linker | 15 |
| 4 | variable region of anti-CD19 antibody (FMC63) heavy chain | 120 |
| 5 | CD8-derived sequence (including transmembrane region) | 83 |
| 6 | intracellular domain region of 4-1BB | 47 |
| 7 | intracellular domain region of CD3ζ | 112 |

An artificial oligo DNA synthesized above was incorporated into the multicloning site of pMY retrovirus vector. Using FLYRD18 cells for retrovirus vector production, a virus vector was produced. iγδTC produced in [Example 3] was infected with the produced retrovirus vector carrying a gene encoding anti-CD19-CAR, whereby iPS cell-derived anti-CD19-CART cell (hereinafter sometimes to be referred to as "CD19iγδCARTC") was produced.

### [Example 7]

### 1. Cytotoxic activity etc. of T cells

### 1-1. Cytotoxic activity of CD19iWT1αβCARTC and BMCAiWT1αβCARTC

The cytotoxic activity of CD19iWT1αβCARTC and BMCAiWT1αβCARTC obtained in [Example 6] 2-1. and 2-2. against CD19 positive Raji cancer cell and BCMA positive H929 cancer cell, respectively, was evaluated. CD19iWT1αβCARTC or iWT1αβTC was blended at a proportion of 16-fold relative to the Raji cell. The cytotoxic activity of CD19iWT1αβCARTC was evaluated based on the target cell death 2 hr later. In addition, BCMAiWT1αβCARTC or iWT1αβTC was blended at a proportion of 16-fold relative to the H929 cell. The cytotoxic activity of BCMAiWT1αβCARTC was evaluated based on the target cell death 2 hr later. It was shown that CD19iWT1αβCARTC and BMCAiWT1αβCARTC have cytotoxic activity against CD19 positive Raji cancer cell and BCMA positive H929 cancer cell, respectively, and that two kinds of CART can be produced from the same iWT1αβTC (Fig. 10).

### 1-2. Cytotoxic activity of CD19-CD30-iWT1αβCARTC

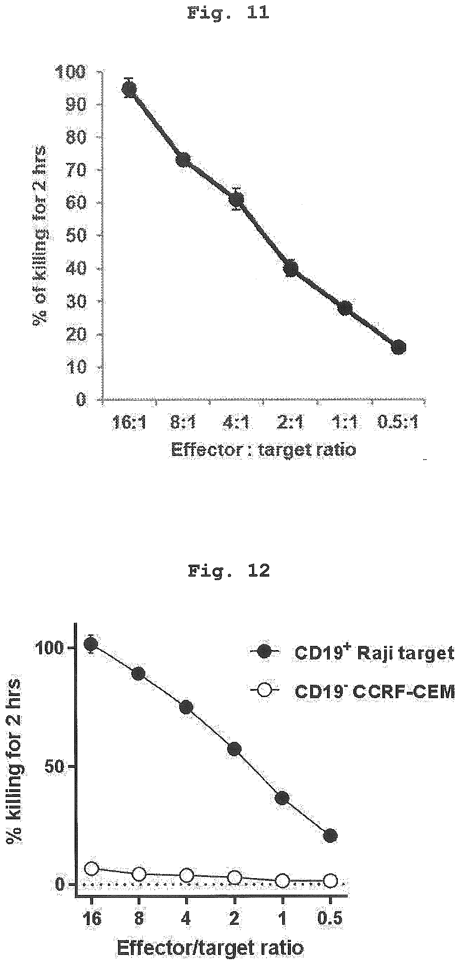

The cytotoxic activity of CD19-CD30-iWT1αβCARTC obtained in [Example 6] 2-3. against CD19 positive Raji cancer cell was evaluated. CD19-CD30-iWT1αβCARTC was blended at a proportion of 0.5, 1, 2, 4, 8, 16-fold relative to the Raji cell. The cytotoxic activity of CD19-CD30-iWT1αβCARTC was evaluated based on the target cell death 2 hr later. It was shown that CD19-CD30-iWT1αβCARTC has cytotoxic activity against CD19 positive Raji cancer cell and that different CART can be produced from iWT1αβTC (Fig. 11).

### 1-3. Cytotoxic activity of CD19iγδCARTC

The cytotoxic activity of CD19iγδCARTCobtained in [Example 6], 2-4., was evaluated. Using CD19-positive Raji cell and CD19 negative CCRF-CEN cell as target cells, CD19iγδCARTC was blended at a proportion of 0.5, 1, 2, 4, 8, 16-fold relative to the target cell. The cytotoxic activity of CD19iγδCARTC was evaluated based on the target cell death 2 hr later. It was shown that CD19iγδCARTC has cytotoxic activity against CD19 positive Raji cell, does not have cytotoxic activity against CD19 negative CCRF-CEN cell, and has antigen specific cytotoxic activity (Fig. 12).

### [Example 8]

### 1. Introduction of anti-CD19-CAR gene and IL-15Rα/IL-15 gene

As IL-15Rα/IL-15 gene, an oligo DNA encoding a polypeptide (SEQ ID NO: 7) designed to align in the order shown in Table 11 from the N-terminal was artificially synthesized.

**[Table 11]**

| order from N-terminal | gene | amino acid number |
|---|---|---|
| 1 | lead sequence of human IL-2 | 23 |
| 2 | C-terminal sequence of human IL-15 | 114 |
| 3 | GGGGS linker | 24 |
| 4 | C-terminal sequence of human IL-15RA | 239 |

An artificial oligo DNA synthesized above was incorporated into the multicloning site of pMY retrovirus vector. Using FLYRD18 cells for retrovirus vector production, a virus vector was produced. iγδTC and Vγ9Vδ2-iTC produced in [Example 3] were infected with the produced retrovirus vector carrying IL-15Rα/IL-15 gene and the retrovirus vector carrying an anti-CD19-CAR gene and produced in [Example 6] 2-4., whereby CD19/IL15iγδCARTC and CD19/IL15iVγ9Vδ2CARTC were respectively produced.

### [Example 9] 1. T cell product

### 1-1. Expansion culture of T cells (CD19/IL15iVγ9Vδ2CARTC)

CD19/IL15iVγ9Vδ2CARTC obtained in [Example 8] was suspended at 2,000,000 cells/mL in a medium obtained by adding cytokine shown in Table 6 to α-MEM medium containing 15% FBS, and the suspension was seeded in a plate containing anti-CD3 antibody (UCHT1) and RetroNectin immobilized thereon and cultured at 5% CO₂/37°C for 3 days. The cells were harvested from the plate on day 3 of culture and the number of the cells was measured using NucleoCounter (registered trade mark) NC-200 (ChemoMetec). The cells were suspended in an appropriate amount of a medium obtained by adding cytokine shown in Table 5 to α-MEM medium containing 15% FBS, added to a non-solidified G-Rex (registered trade mark) 6-well plate (WILSONWOLF) and cultured at 5%CO₂/37°C. Thereafter, a part of the cells was collected from the plate 4 to 6 times on any of days 5, 6, 7, 8, 9, 10, 11, 14, 17 of culture, and the number of cells was measured using a hemocytometer. The cells were immobilized on the culture plate with anti-CD3 antibody and RetroNectin by the following method. An anti-CD3 antibody (UCHT1, final concentration 3000 ng/mL) and RetroNectin (final concentration 150 µg/mL) dissolved in PBS at necessary concentrations were added to the plate and the plate was stood overnight at 4°C.

### 1-2. Proliferation rate

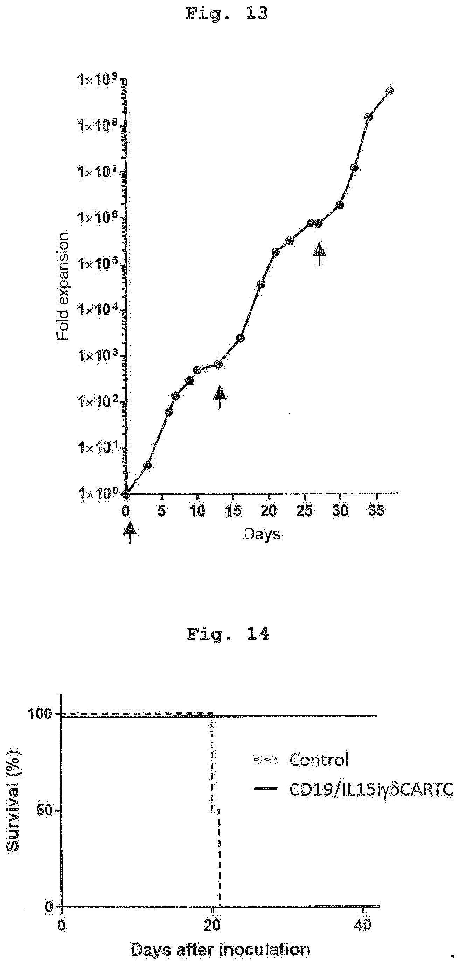

The proliferation rate of the number of CD19/IL15iVγ9Vδ2CARTC cells when the expansion culture of [Example 9] 1-1., was repeated 3 times is shown in Fig. 13. CD19/IL15iVγ9Vδ2CARTC could be expansion cultured at least not less than 10⁹ times.

In this way, CAR-T cells can be expansion cultured and provided from the master cell bank of T cell and/or working cell bank of T cell to produce a large amount of T cell product (e.g., CAR-T cell product). The expansion cultured various T cells are filled in an appropriate storage container (sterile vial, blood transfusion bag, and the like), the container is labeled and packaged to produce a T cell product.

### 1-3. Freezing of T cell product

A container containing expansion cultured various T cells is frozen in the same manner as in [Example 5] 1., to produce a frozen T cell product.

### 1-4. Construction of T cell product collection

A T cell product collection is constructed by labeling the frozen T cell products obtained above so that the kind thereof can be distinguished, and storing them.

### [Example 10]

### 1. Identification of antigen

A biological sample such as a tumor tissue section, a body fluid (e.g., blood, serum, plasma, etc.) that may contain tumor cell, and the like is collected from a test subject, mRNA is extracted from the sample, and cDNA is synthesized as necessary from the mRNA and identified using the cDNA and a nucleic acid amplification method and/or a nucleic acid detection method such as digital PCR (e.g., ddPCR), RT-PCR, biochip (e.g., microarray), RNAseq and the like.

### 2. Selection of T cell product

Based on the material (paper medium or electronic data medium) showing the identification result in [Example 10] 1., a T cell product containing T cells expressing a CAR or an exogenous TCR that recognizes and binds to the identified antigen is selected from a list of T cell products prepared in advance.

### [Example 11]

### 1. Number of survival day extending effect of CD19/IL15iγδCARTC

5×10⁵ Nalm6 cells (ATCC) were transplanted to NOD/Shi-scid, IL-2RyKO (NOG) mice (Central Institute for Experimental Animals, female, 7-8-week-old) from the tail vein to prepare Nalm6 xenograft mice. On day 4 post-transplantation, a suspension of CD19/IL15iγδCARTC (5×10⁶ (cells)) in 0.1 mL of HBSS-buffer or an equal amount of HBSS-buffer was administered from the tail vein, and the number of days of survival was confirmed. All mice transplanted with CD19 positive Nalm6 cancer cells via the tail vein died within 3 weeks in the control administration group, whereas all mice survived for at least 6 weeks in the CD19/IL15iγδCARTC administration group (Fig. 14).

### 2. Antitumor effect of CD19/IL15iVγ9Vδ2CARTC

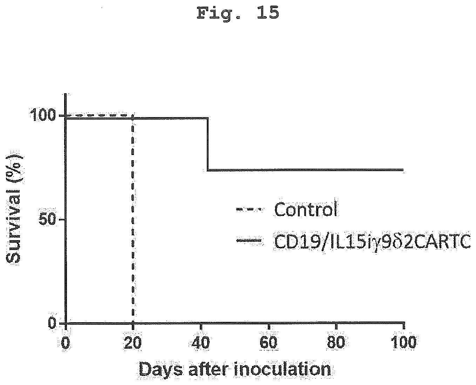

5×10⁵ luciferase-expressing Nalm6 cells (ATCC) were transplanted to NOD/Shi-scid, IL-2RγKO (NOG) mice (Central Institute for Experimental Animals, female, 7-8-week-old) from the tail vein to prepare luciferase-expressing Nalm6 xenograft mice. On day 4 post-transplantation, a suspension of CD19/IL15iVγ9Vδ2CARTC (10⁷ (cells)) obtained in [Example 6], 1-2., in 0.2 mL of HBSS-buffer or an equal amount of HBSS-buffer was administered from the tail vein, and the number of days of survival was confirmed. All mice transplanted with CD19 positive Nalm6 cancer cells via the tail vein died within 3 weeks in the control administration group, whereas all mice survived for at least 6 weeks in the CD19/IL15iγδCARTC administration group (Fig. 15).

### [INDUSTRIAL APPLICABILITY]

According to the present invention, a system for providing a high quality, off-the-shelf allogenic T cell product (particularly, plural kinds of CAR-T cell products), that can reduce human, time and financial costs and minimize difference between lots, and a method for providing same are provided. The thus-provided T cell product is useful for the prophylaxis or treatment of diseases such as cancer, tumor and the like.

This application is based on a patent application No. 2019-212718 filed in Japan (filing date: November 25, 2019), the contents of which are incorporated in full herein.

## Claims

1. A system for providing a T cell product, comprising a master cell bank of T cell and/or a working cell bank of T cell.

2. The system according to claim 1, wherein the master cell bank of T cell and/or working cell bank of T cell comprise(s) a T cell derived from an induced pluripotent stem cell.

3. The system according to claim 1 or 2, wherein the master cell bank of T cell and/or working cell bank of T cell comprise(s) a T cell with suppressed expression of at least one kind of HLA gene.

4. The system according to any one of claims 1 to 3, further comprising a step of introducing a nucleic acid comprising the exogenous gene into a T cell prepared from the master cell bank of T cell and/or working cell bank of T cell.

5. The system according to claim 4, wherein the exogenous gene is a CAR gene or an exogenous TCR gene.

6. The system according to any one of claims 1 to 5, wherein the T cell product comprises two or more kinds thereof.

7. The system according to any one of claims 1 to 6, further comprising a step of expansion culturing a T cell prepared from the master cell bank of T cell and/or working cell bank of T cell.

8. The system according to claim 7, wherein the step of expansion culturing the T cell comprises a process of stimulating the cell with a CD30 agonist.

9. The system according to any one of claims 7 to 8, further comprising a step of producing a frozen T cell product comprising the expansion cultured T cell.

10. The system according to any one of claims 6 to 9, further comprising a step of constructing a T cell product collection comprising two or more kinds of T cell products by collecting T cell products.

11. The system according to any one of claims 1 to 10, further comprising a step of identifying a tumor-specific antigen or a tumor-associated antigen expressed in a tumor of the test subject.

12. The system according to claim 11, further comprising a step of selecting a T cell product expressing a CAR or an exogenous TCR that recognizes and binds to the identified antigen from a T cell product collection comprising two or more kinds of T cell products.

13. A method for producing a T cell product, comprising a process of constructing a master cell bank of T cell and/or a working cell bank of T cell.

14. The method for producing a T cell product according to claim 13, wherein the master cell bank of T cell and/or working cell bank of T cell comprise(s) a T cell derived from an induced pluripotent stem cell.

15. The method for producing a T cell product according to claim 13 or 14, wherein the master cell bank of T cell and/or working cell bank of T cell comprise(s) a T cell with suppressed expression of at least one kind of HLA gene.

16. The method for producing a T cell product according to any one of claims 13 to 15, further comprising a process of introducing a nucleic acid comprising the exogenous gene into a T cell prepared from the master cell bank of T cell and/or working cell bank of T cell.

17. The method for producing a T cell product according to claim 16, wherein the exogenous gene is a CAR gene or an exogenous TCR gene.

18. The method for producing a T cell product according to any one of claims 13 to 17, further comprising a process of expansion culturing a T cell.

19. The method for producing a T cell product according to claim 18, wherein the process of expansion culturing the T cell comprises a process of stimulating the cell with a CD30 agonist.

20. A master cell bank of T cell and/or a working cell bank of T cell.

21. The master cell bank of T cell and/or working cell bank of T cell according to claim 20, comprising a T cell derived from an induced pluripotent stem cell.

22. The master cell bank of T cell and/or working cell bank of T cell according to claim 20 or 21, comprising a T cell with suppressed expression of at least one kind of HLA gene.

23. A master cell bank collection of T cell and/or a working cell bank collection of T cell comprising two or more kinds of the master cell banks of T cell and/or working cell banks of T cell according to any one of claims 20 to 22.

24. A method for constructing a master cell bank of T cell and/or a working cell bank of T cell, comprising the following processes:
(1) a process of differentiating an induced pluripotent stem cell free of a chimeric antigen receptor (CAR) gene into a T cell for CAR-T therapy,
(2) a process of stocking the differentiated T cell, and
(3) a process of characterization of the differentiated T cell.

25. A method for constructing a master cell bank of T cell and/or a working cell bank of T cell, comprising the following processes:
(1) a process of differentiating an induced pluripotent stem cell free of an exogenous T cell receptor (TCR) gene into a T cell for TCR-T therapy,
(2) a process of stocking the differentiated T cell, and
(3) a process of characterization of the differentiated T cell.

26. The method according to claim 24, wherein the induced pluripotent stem cell has an exogenous T cell receptor (TCR) gene.

27. The method according to any one of claims 24 to 26, wherein at least one kind of HLA gene is deleted in the induced pluripotent stem cell.

28. The method according to any one of claims 24 to 27, wherein the T cell expresses CD8αβ.

29. A master cell bank of T cell and/or a working cell bank of T cell constructed by the method according to any one of claims 24 to 28.

30. A method for producing a T cell product expressing a CAR or an exogenous TCR, comprising the following processes:
(1) a process of preparing a T cell from the master cell bank of T cell and/or the working cell bank of T cell according to claim 29,
(2) a process of introducing a CAR gene or an exogenous TCR gene into the prepared T cell, and
(3) a process of expansion culturing the T cell into which the CAR gene or exogenous TCR gene has been introduced.

31. The method according to claim 30, wherein the CAR or exogenous TCR recognizes and binds to a tumor-specific antigen or a tumor-associated antigen.

32. The method according to claim 30 or 31, wherein the process (3) comprises a process of stimulating the T cell with a CD30 agonist.

33. A T cell product produced by the method according to any one of claims 30 to 32.

34. A method for constructing a T cell product collection comprising two or more kinds of T cell products, comprising a process of collecting the T cell product according to claim 33.

35. A T cell product collection constructed by the method according to claim 34.

36. A method for providing a T cell product suitable for a test subject, comprising the following processes:
(1) a process of identifying a tumor-specific antigen or a tumor-associated antigen expressed in the tumor of a test subject, and
(2) a process of selecting a T cell product that expresses a CAR or an exogenous TCR that recognizes and binds to the identified antigen from the T cell product collection according to claim 35.


SEQUENCE LISTING

<110>  Kyoto University
       TAKEDA PHARMACEUTICAL COMPANY LIMITED

<120>  Master cell bank of T cell

<130>  093085

<150>  JP 2019-212718
<151>  2019-11-25

<160>  7

<170>  PatentIn version 3.5

<210>  1
<211>  629
<212>  PRT
<213>  Artificial Sequence

<220>
<223>  Vgamma9 Vdelta2 TCR



<220>
<221>  PEPTIDE
<222>  (1)..(315)
<223>  T cell receptor gamma

<220>
<221>  SIGNAL
<222>  (316)..(337)
<223>  P2A

<220>
<221>  PEPTIDE
<222>  (338)..(629)
<223>  T cell receptor delta

<400>  1

Met Val Ser Leu Leu His Ala Ser Thr Leu Ala Val Leu Gly Ala Leu
1               5                   10                  15


Cys Val Tyr Gly Ala Gly His Leu Glu Gln Pro Gln Ile Ser Ser Thr
            20                  25                  30


Lys Thr Leu Ser Lys Thr Ala Arg Leu Glu Cys Val Val Ser Gly Ile
        35                  40                  45


Thr Ile Ser Ala Thr Ser Val Tyr Trp Tyr Arg Glu Arg Pro Gly Glu
    50                  55                  60


Val Ile Gln Phe Leu Val Ser Ile Ser Tyr Asp Gly Thr Val Arg Lys
65                  70                  75                  80


Glu Ser Gly Ile Pro Ser Gly Lys Phe Glu Val Asp Arg Ile Pro Glu

                85                  90                  95

Thr Ser Thr Ser Thr Leu Thr Ile His Asn Val Glu Lys Gln Asp Ile
            100                 105                 110

Ala Thr Tyr Tyr Cys Ala Leu Trp Glu Ala Gln Gln Glu Leu Gly Lys
        115                 120                 125

Lys Ile Lys Val Phe Gly Pro Gly Thr Lys Leu Ile Ile Thr Asp Lys
    130                 135                 140

Gln Leu Asp Ala Asp Val Ser Pro Lys Pro Thr Ile Phe Leu Pro Ser
145                 150                 155                 160

Ile Ala Glu Thr Lys Leu Gln Lys Ala Gly Thr Tyr Leu Cys Leu Leu
                165                 170                 175

Glu Lys Phe Phe Pro Asp Val Ile Lys Ile His Trp Glu Glu Lys Lys
            180                 185                 190

Ser Asn Thr Ile Leu Gly Ser Gln Glu Gly Asn Thr Met Lys Thr Asn
        195                 200                 205

Asp Thr Tyr Met Lys Phe Ser Trp Leu Thr Val Pro Glu Lys Ser Leu
    210                 215                 220

Asp Lys Glu His Arg Cys Ile Val Arg His Glu Asn Asn Lys Asn Gly
225                 230                 235                 240

Val Asp Gln Glu Ile Ile Phe Pro Pro Ile Lys Thr Asp Val Ile Thr
                245                 250                 255

Met Asp Pro Lys Asp Asn Cys Ser Lys Asp Ala Asn Asp Thr Leu Leu
            260                 265                 270

Leu Gln Leu Thr Asn Thr Ser Ala Tyr Tyr Met Tyr Leu Leu Leu Leu
        275                 280                 285

Leu Lys Ser Val Val Tyr Phe Ala Ile Ile Thr Cys Cys Leu Leu Arg
    290                 295                 300

Arg Thr Ala Phe Cys Cys Asn Gly Glu Lys Ser Gly Ser Gly Ala Thr
305                 310                 315                 320

Asn Phe Ser Leu Leu Lys Gln Ala Gly Asp Val Glu Glu Asn Pro Gly
                325                 330                 335

Pro Met Glu Arg Ile Ser Ser Leu Ile His Leu Ser Leu Phe Trp Ala
            340                 345                 350

Gly Val Met Ser Ala Ile Glu Leu Val Pro Glu His Gln Thr Val Pro
        355                 360                 365

Val Ser Ile Gly Val Pro Ala Thr Leu Arg Cys Ser Met Lys Gly Glu
    370                 375                 380

Ala Ile Gly Asn Tyr Tyr Ile Asn Trp Tyr Arg Lys Thr Gln Gly Asn
385                 390                 395                 400

Thr Met Thr Phe Ile Tyr Arg Glu Lys Asp Ile Tyr Gly Pro Gly Phe
                405                 410                 415

Lys Asp Asn Phe Gln Gly Asp Ile Asp Ile Ala Lys Asn Leu Ala Val
            420                 425                 430

Leu Lys Ile Leu Ala Pro Ser Glu Arg Asp Glu Gly Ser Tyr Tyr Cys
        435                 440                 445

Ala Cys Asp Thr Leu Gly Met Gly Gly Glu Tyr Thr Asp Lys Leu Ile
    450                 455                 460

Phe Gly Lys Gly Thr Arg Val Thr Val Glu Pro Arg Ser Gln Pro His
465                 470                 475                 480

Thr Lys Pro Ser Val Phe Val Met Lys Asn Gly Thr Asn Val Ala Cys
                485                 490                 495

Leu Val Lys Glu Phe Tyr Pro Lys Asp Ile Arg Ile Asn Leu Val Ser
            500                 505                 510

Ser Lys Lys Ile Thr Glu Phe Asp Pro Ala Ile Val Ile Ser Pro Ser
        515                 520                 525

Gly Lys Tyr Asn Ala Val Lys Leu Gly Lys Tyr Glu Asp Ser Asn Ser
    530                 535                 540

Val Thr Cys Ser Val Gln His Asp Asn Lys Thr Val His Ser Thr Asp
545                 550                 555                 560

Phe Glu Val Lys Thr Asp Ser Thr Asp His Val Lys Pro Lys Glu Thr
                565                 570                 575

Glu Asn Thr Lys Gln Pro Ser Lys Ser Cys His Lys Pro Lys Ala Ile
            580                 585                 590

Val His Thr Glu Lys Val Asn Met Met Ser Leu Thr Val Leu Gly Leu
        595                 600                 605

Arg Met Leu Phe Ala Lys Thr Val Ala Val Asn Phe Leu Leu Thr Ala
    610                 615                 620

Lys Leu Phe Phe Leu
625

<210>  2
<211>  544
<212>  PRT
<213>  Artificial Sequence

<220>
<223>  CD19-CAR(CD8-CD28-4-1BB-CD3z)

<220>
<221>  SIGNAL
<222>  (1)..(22)
<223>  lead sequence of immunoglobulin heavy chain

<220>
<221>  PEPTIDE
<222>  (23)..(126)
<223>  variable reagion of anti-CD19 antibody light chain

<220>
<221>  PEPTIDE
<222>  (127)..(141)
<223>  Peptide Linker

<220>
<221>  PEPTIDE
<222>  (142)..(261)
<223>  variable reagion of anti-CD19 antibody heavy chain

<220>
<221>  PEPTIDE
<222>  (262)..(344)
<223>  CD8-derived sequence

<220>
<221>  PEPTIDE
<222>  (345)..(385)
<223>  intracellular domain region of CD28

<220>
<221>  PEPTIDE
<222>  (386)..(432)
<223>  intracellular domain region of 4-1BB

<220>
<221>  PEPTIDE
<222>  (433)..(544)
<223>  intracellular domain region of CD3 zeta

<400>  2

Met Asp Trp Thr Trp Arg Ile Leu Phe Leu Val Ala Ala Ala Thr Gly
1               5                   10                  15

Ala His Ser Asp Ile Gln Met Thr Gln Thr Thr Ser Ser Leu Ser Ala
            20                  25                  30

Ser Leu Gly Asp Arg Val Thr Ile Ser Cys Arg Ala Ser Gln Asp Ile
        35                  40                  45

Ser Lys Tyr Leu Asn Trp Tyr Gln Gln Lys Pro Asp Gly Thr Val Lys
    50                  55                  60

Leu Leu Ile Tyr His Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Tyr Thr Phe Gly Gly Gly Thr Lys Leu Glu Ile Thr Gly Gly
        115                 120                 125

Gly Gly Ser Gly Gly Gly Gly Ser Gly Gly Gly Gly Ser Glu Val Lys
    130                 135                 140

Leu Gln Glu Ser Gly Pro Gly Leu Val Ala Pro Ser Gln Ser Leu Ser
145                 150                 155                 160

Val Thr Cys Thr Val Ser Gly Val Ser Leu Pro Asp Tyr Gly Val Ser
                165                 170                 175

Trp Ile Arg Gln Pro Pro Arg Lys Gly Leu Glu Trp Leu Gly Val Ile
            180                 185                 190

Trp Gly Ser Glu Thr Thr Tyr Tyr Asn Ser Ala Leu Lys Ser Arg Leu
        195                 200                 205

Thr Ile Ile Lys Asp Asn Ser Lys Ser Gln Val Phe Leu Lys Met Asn
    210                 215                 220

Ser Leu Gln Thr Asp Asp Thr Ala Ile Tyr Tyr Cys Ala Lys His Tyr
225                 230                 235                 240

Tyr Tyr Gly Gly Ser Tyr Ala Met Asp Tyr Trp Gly Gln Gly Thr Ser
                245                 250                 255

Val Thr Val Ser Ser Phe Val Pro Val Phe Leu Pro Ala Lys Pro Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Ile Tyr Ile Trp
305                 310                 315                 320

Ala Pro Leu Ala Gly Thr Cys Gly Val Leu Leu Leu Ser Leu Val Ile
                325                 330                 335

Thr Leu Tyr Cys Asn His Arg Asn Arg Ser Lys Arg Ser Arg Leu Leu
            340                 345                 350

His Ser Asp Tyr Met Asn Met Thr Pro Arg Arg Pro Gly Pro Thr Arg
        355                 360                 365

Lys His Tyr Gln Pro Tyr Ala Pro Pro Arg Asp Phe Ala Ala Tyr Arg
    370                 375                 380

Ser Arg Phe Ser Val Val Lys Arg Gly Arg Lys Lys Leu Leu Tyr Ile
385                 390                 395                 400

Phe Lys Gln Pro Phe Met Arg Pro Val Gln Thr Thr Gln Glu Glu Asp
                405                 410                 415

Gly Cys Ser Cys Arg Phe Pro Glu Glu Glu Glu Gly Gly Cys Glu Leu
            420                 425                 430

Arg Val Lys Phe Ser Arg Ser Ala Asp Ala Pro Ala Tyr Gln Gln Gly
        435                 440                 445

Gln Asn Gln Leu Tyr Asn Glu Leu Asn Leu Gly Arg Arg Glu Glu Tyr
    450                 455                 460

Asp Val Leu Asp Lys Arg Arg Gly Arg Asp Pro Glu Met Gly Gly Lys
465                 470                 475                 480

Pro Arg Arg Lys Asn Pro Gln Glu Gly Leu Tyr Asn Glu Leu Gln Lys
                485                 490                 495

Asp Lys Met Ala Glu Ala Tyr Ser Glu Ile Gly Met Lys Gly Glu Arg

500                 505                 510

Arg Arg Gly Lys Gly His Asp Gly Leu Tyr Gln Gly Leu Ser Thr Ala
        515                 520                 525

Thr Lys Asp Thr Tyr Asp Ala Leu His Met Gln Ala Leu Pro Pro Arg
    530                 535                 540

<210>  3
<211>  551
<212>  PRT
<213>  Artificial Sequence

<220>
<223>  BCMA-CAR(CD8-CD28-4-1BB-CD3z)

<220>
<221>  SIGNAL
<222>  (1)..(19)
<223>  lead sequence of immunoglobulin heavy chain

<220>
<221>  PEPTIDE
<222>  (20)..(130)
<223>  variable reagion of anti-BCMA antibody light chain

<220>
<221>  PEPTIDE
<222>  (131)..(148)
<223>  Peptide Linker

<220>
<221>  PEPTIDE
<222>  (149)..(265)
<223>  variable reagion of anti-BCMA antibody heavy chain

<220>
<221>  PEPTIDE
<222>  (266)..(351)
<223>  CD8-derived sequence

<220>
<221>  PEPTIDE
<222>  (352)..(392)
<223>  intracellular domain region of CD28

<220>
<221>  PEPTIDE
<222>  (393)..(439)
<223>  intracellular domain region of 4-1BB

<220>
<221>  PEPTIDE
<222>  (440)..(551)
<223>  intracellular domain region of CD3 zeta

<400>  3

Met Asp Trp Thr Trp Arg Ile Leu Phe Leu Val Ala Ala Ala Thr Gly

1               5                   10                  15

Ala His Ser Asp Ile Val Leu Thr Gln Ser Pro Pro Ser Leu Ala Met
            20                  25                  30

Ser Leu Gly Lys Arg Ala Thr Ile Ser Cys Arg Ala Ser Glu Ser Val
        35                  40                  45

Thr Ile Leu Gly Ser His Leu Ile His Trp Tyr Gln Gln Lys Pro Gly
    50                  55                  60

Gln Pro Pro Thr Leu Leu Ile Gln Leu Ala Ser Asn Val Gln Thr Gly
65                  70                  75                  80

Val Pro Ala Arg Phe Ser Gly Ser Gly Ser Arg Thr Asp Phe Thr Leu
                85                  90                  95

Thr Ile Asp Pro Val Glu Glu Asp Asp Val Ala Val Tyr Tyr Cys Leu
            100                 105                 110

Gln Ser Arg Thr Ile Pro Arg Thr Phe Gly Gly Gly Thr Lys Leu Glu
        115                 120                 125

Ile Lys Gly Ser Thr Ser Gly Ser Gly Lys Pro Gly Ser Gly Glu Gly
    130                 135                 140

Ser Thr Lys Gly Gln Ile Gln Leu Val Gln Ser Gly Pro Glu Leu Lys
145                 150                 155                 160

Lys Pro Gly Glu Thr Val Lys Ile Ser Cys Lys Ala Ser Gly Tyr Thr
                165                 170                 175

Phe Thr Asp Tyr Ser Ile Asn Trp Val Lys Arg Ala Pro Gly Lys Gly
            180                 185                 190

Leu Lys Trp Met Gly Trp Ile Asn Thr Glu Thr Arg Glu Pro Ala Tyr
        195                 200                 205

Ala Tyr Asp Phe Arg Gly Arg Phe Ala Phe Ser Leu Glu Thr Ser Ala
    210                 215                 220

Ser Thr Ala Tyr Leu Gln Ile Asn Asn Leu Lys Tyr Glu Asp Thr Ala
225                 230                 235                 240

Thr Tyr Phe Cys Ala Leu Asp Tyr Ser Tyr Ala Met Asp Tyr Trp Gly
                245                 250                 255

Gln Gly Thr Ser Val Thr Val Ser Ser Ala Ala Ala Phe Val Pro Val
            260                 265                 270

Phe Leu Pro Ala Lys Pro Thr Thr Thr Pro Ala Pro Arg Pro Pro Thr
        275                 280                 285

Pro Ala Pro Thr Ile Ala Ser Gln Pro Leu Ser Leu Arg Pro Glu Ala
    290                 295                 300

Cys Arg Pro Ala Ala Gly Gly Ala Val His Thr Arg Gly Leu Asp Phe
305                 310                 315                 320

Ala Cys Asp Ile Tyr Ile Trp Ala Pro Leu Ala Gly Thr Cys Gly Val
                325                 330                 335

Leu Leu Leu Ser Leu Val Ile Thr Leu Tyr Cys Asn His Arg Asn Arg
            340                 345                 350

Ser Lys Arg Ser Arg Leu Leu His Ser Asp Tyr Met Asn Met Thr Pro
        355                 360                 365

Arg Arg Pro Gly Pro Thr Arg Lys His Tyr Gln Pro Tyr Ala Pro Pro
    370                 375                 380

Arg Asp Phe Ala Ala Tyr Arg Ser Arg Phe Ser Val Val Lys Arg Gly
385                 390                 395                 400

Arg Lys Lys Leu Leu Tyr Ile Phe Lys Gln Pro Phe Met Arg Pro Val
                405                 410                 415

Gln Thr Thr Gln Glu Glu Asp Gly Cys Ser Cys Arg Phe Pro Glu Glu
            420                 425                 430

Glu Glu Gly Gly Cys Glu Leu Arg Val Lys Phe Ser Arg Ser Ala Asp
        435                 440                 445

Ala Pro Ala Tyr Gln Gln Gly Gln Asn Gln Leu Tyr Asn Glu Leu Asn
    450                 455                 460

Leu Gly Arg Arg Glu Glu Tyr Asp Val Leu Asp Lys Arg Arg Gly Arg
465                 470                 475                 480

Asp Pro Glu Met Gly Gly Lys Pro Arg Arg Lys Asn Pro Gln Glu Gly
                485                 490                 495

Leu Tyr Asn Glu Leu Gln Lys Asp Lys Met Ala Glu Ala Tyr Ser Glu
            500                 505                 510

Ile Gly Met Lys Gly Glu Arg Arg Arg Gly Lys Gly His Asp Gly Leu
        515                 520                 525

Tyr Gln Gly Leu Ser Thr Ala Thr Lys Asp Thr Tyr Asp Ala Leu His
    530                 535                 540

Met Gln Ala Leu Pro Pro Arg
545                 550

<210>  4
<211>  584
<212>  PRT
<213>  Artificial Sequence

<220>
<223>  CD19-CAR(CD8-CD28-CD30-CD3z)

<220>
<221>  SIGNAL
<222>  (1)..(22)
<223>  lead sequence of immunoglobulin heavy chain

<220>
<221>  PEPTIDE
<222>  (23)..(126)
<223>  variable reagion of anti-CD19 antibody light chain

<220>
<221>  PEPTIDE
<222>  (127)..(141)
<223>  Peptide Linker

<220>
<221>  PEPTIDE
<222>  (142)..(261)
<223>  variable reagion of anti-CD19 antibody heavy chain

<220>
<221>  PEPTIDE
<222>  (262)..(344)
<223>  CD8-derived sequence

<220>
<221>  PEPTIDE
<222>  (345)..(385)
<223>  intracellular domain region of CD28

<220>
<221>  PEPTIDE
<222>  (386)..(472)
<223>  intracellular domain region of CD30

<220>
<221>  PEPTIDE
<222>  (473)..(584)
<223>  intracellular domain region of CD3 zeta

<400>  4

Met Asp Trp Thr Trp Arg Ile Leu Phe Leu Val Ala Ala Ala Thr Gly
1               5                   10                  15

Ala His Ser Asp Ile Gln Met Thr Gln Thr Thr Ser Ser Leu Ser Ala
            20                  25                  30

Ser Leu Gly Asp Arg Val Thr Ile Ser Cys Arg Ala Ser Gln Asp Ile
        35                  40                  45

Ser Lys Tyr Leu Asn Trp Tyr Gln Gln Lys Pro Asp Gly Thr Val Lys
    50                  55                  60

Leu Leu Ile Tyr His Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Tyr Thr Phe Gly Gly Gly Thr Lys Leu Glu Ile Thr Gly Gly
        115                 120                 125

Gly Gly Ser Gly Gly Gly Gly Ser Gly Gly Gly Gly Ser Glu Val Lys
    130                 135                 140

Leu Gln Glu Ser Gly Pro Gly Leu Val Ala Pro Ser Gln Ser Leu Ser
145                 150                 155                 160

Val Thr Cys Thr Val Ser Gly Val Ser Leu Pro Asp Tyr Gly Val Ser
                165                 170                 175

Trp Ile Arg Gln Pro Pro Arg Lys Gly Leu Glu Trp Leu Gly Val Ile
            180                 185                 190

Trp Gly Ser Glu Thr Thr Tyr Tyr Asn Ser Ala Leu Lys Ser Arg Leu
        195                 200                 205

Thr Ile Ile Lys Asp Asn Ser Lys Ser Gln Val Phe Leu Lys Met Asn
    210                 215                 220

Ser Leu Gln Thr Asp Asp Thr Ala Ile Tyr Tyr Cys Ala Lys His Tyr
225                 230                 235                 240

Tyr Tyr Gly Gly Ser Tyr Ala Met Asp Tyr Trp Gly Gln Gly Thr Ser
                245                 250                 255

Val Thr Val Ser Ser Phe Val Pro Val Phe Leu Pro Ala Lys Pro Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Ile Tyr Ile Trp
305                 310                 315                 320

Ala Pro Leu Ala Gly Thr Cys Gly Val Leu Leu Leu Ser Leu Val Ile
                325                 330                 335

Thr Leu Tyr Cys Asn His Arg Asn Arg Ser Lys Arg Ser Arg Leu Leu
            340                 345                 350

His Ser Asp Tyr Met Asn Met Thr Pro Arg Arg Pro Gly Pro Thr Arg
        355                 360                 365

Lys His Tyr Gln Pro Tyr Ala Pro Pro Arg Asp Phe Ala Ala Tyr Arg
    370                 375                 380

Ser His Thr Asn Asn Lys Ile Glu Lys Ile Tyr Ile Met Lys Ala Asp
385                 390                 395                 400

Thr Val Ile Val Gly Thr Val Lys Ala Glu Leu Pro Glu Gly Arg Gly
                405                 410                 415

Leu Ala Gly Pro Ala Glu Pro Glu Leu Glu Glu Glu Leu Glu Ala Asp
            420                 425                 430

His Thr Pro His Tyr Pro Glu Gln Glu Thr Glu Pro Pro Leu Gly Ser
        435                 440                 445

Cys Ser Asp Val Met Leu Ser Val Glu Glu Glu Gly Lys Glu Asp Pro
    450                 455                 460

Leu Pro Thr Ala Ala Ser Gly Lys Arg Val Lys Phe Ser Arg Ser Ala
465                 470                 475                 480

Asp Ala Pro Ala Tyr Gln Gln Gly Gln Asn Gln Leu Tyr Asn Glu Leu
                485                 490                 495

Asn Leu Gly Arg Arg Glu Glu Tyr Asp Val Leu Asp Lys Arg Arg Gly

500                 505                 510

Arg Asp Pro Glu Met Gly Gly Lys Pro Arg Arg Lys Asn Pro Gln Glu
        515                 520                 525

Gly Leu Tyr Asn Glu Leu Gln Lys Asp Lys Met Ala Glu Ala Tyr Ser
    530                 535                 540

Glu Ile Gly Met Lys Gly Glu Arg Arg Arg Gly Lys Gly His Asp Gly
545                 550                 555                 560

Leu Tyr Gln Gly Leu Ser Thr Ala Thr Lys Asp Thr Tyr Asp Ala Leu
                565                 570                 575

His Met Gln Ala Leu Pro Pro Arg
            580

<210>  5
<211>  503
<212>  PRT
<213>  Artificial Sequence

<220>
<223>  CD19-CAR(CD8-4-1BB-CD3z)

<220>
<221>  SIGNAL
<222>  (1)..(22)
<223>  lead sequence of immunoglobulin heavy chain

<220>
<221>  PEPTIDE
<222>  (23)..(126)
<223>  variable reagion of anti-CD19 antibody light chain

<220>
<221>  PEPTIDE
<222>  (127)..(141)
<223>  Peptide Linker

<220>
<221>  PEPTIDE
<222>  (142)..(261)
<223>  variable reagion of anti-CD19 antibody heavy chain

<220>
<221>  PEPTIDE
<222>  (262)..(344)
<223>  CD8-derived sequence

<220>
<221>  PEPTIDE
<222>  (345)..(391)
<223>  intracellular domain region of 4-1BB

<220>

<221>  PEPTIDE
<222>  (392)..(503)
<223>  intracellular domain region of CD3 zeta

<400>  5

Met Asp Trp Thr Trp Arg Ile Leu Phe Leu Val Ala Ala Ala Thr Gly
1               5                   10                  15

Ala His Ser Asp Ile Gln Met Thr Gln Thr Thr Ser Ser Leu Ser Ala
            20                  25                  30

Ser Leu Gly Asp Arg Val Thr Ile Ser Cys Arg Ala Ser Gln Asp Ile
        35                  40                  45

Ser Lys Tyr Leu Asn Trp Tyr Gln Gln Lys Pro Asp Gly Thr Val Lys
    50                  55                  60

Leu Leu Ile Tyr His Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Tyr Thr Phe Gly Gly Gly Thr Lys Leu Glu Ile Thr Gly Gly
        115                 120                 125

Gly Gly Ser Gly Gly Gly Gly Ser Gly Gly Gly Gly Ser Glu Val Lys
    130                 135                 140

Leu Gln Glu Ser Gly Pro Gly Leu Val Ala Pro Ser Gln Ser Leu Ser
145                 150                 155                 160

Val Thr Cys Thr Val Ser Gly Val Ser Leu Pro Asp Tyr Gly Val Ser
                165                 170                 175

Trp Ile Arg Gln Pro Pro Arg Lys Gly Leu Glu Trp Leu Gly Val Ile
            180                 185                 190

Trp Gly Ser Glu Thr Thr Tyr Tyr Asn Ser Ala Leu Lys Ser Arg Leu
        195                 200                 205

Thr Ile Ile Lys Asp Asn Ser Lys Ser Gln Val Phe Leu Lys Met Asn
    210                 215                 220

Ser Leu Gln Thr Asp Asp Thr Ala Ile Tyr Tyr Cys Ala Lys His Tyr

225                 230                 235                 240

Tyr Tyr Gly Gly Ser Tyr Ala Met Asp Tyr Trp Gly Gln Gly Thr Ser
                245                 250                 255

Val Thr Val Ser Ser Phe Val Pro Val Phe Leu Pro Ala Lys Pro Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Ile Tyr Ile Trp
305                 310                 315                 320

Ala Pro Leu Ala Gly Thr Cys Gly Val Leu Leu Leu Ser Leu Val Ile
                325                 330                 335

Thr Leu Tyr Cys Asn His Arg Asn Arg Phe Ser Val Val Lys Arg Gly
            340                 345                 350

Arg Lys Lys Leu Leu Tyr Ile Phe Lys Gln Pro Phe Met Arg Pro Val
        355                 360                 365

Gln Thr Thr Gln Glu Glu Asp Gly Cys Ser Cys Arg Phe Pro Glu Glu
    370                 375                 380

Glu Glu Gly Gly Cys Glu Leu Arg Val Lys Phe Ser Arg Ser Ala Asp
385                 390                 395                 400

Ala Pro Ala Tyr Gln Gln Gly Gln Asn Gln Leu Tyr Asn Glu Leu Asn
                405                 410                 415

Leu Gly Arg Arg Glu Glu Tyr Asp Val Leu Asp Lys Arg Arg Gly Arg
            420                 425                 430

Asp Pro Glu Met Gly Gly Lys Pro Arg Arg Lys Asn Pro Gln Glu Gly
        435                 440                 445

Leu Tyr Asn Glu Leu Gln Lys Asp Lys Met Ala Glu Ala Tyr Ser Glu
    450                 455                 460

Ile Gly Met Lys Gly Glu Arg Arg Arg Gly Lys Gly His Asp Gly Leu
465                 470                 475                 480

Tyr Gln Gly Leu Ser Thr Ala Thr Lys Asp Thr Tyr Asp Ala Leu His
                485                 490                 495

Met Gln Ala Leu Pro Pro Arg
            500

<210>  6
<211>  87
<212>  PRT
<213>  Homo sapiens

<400>  6

His Thr Asn Asn Lys Ile Glu Lys Ile Tyr Ile Met Lys Ala Asp Thr
1               5                   10                  15

Val Ile Val Gly Thr Val Lys Ala Glu Leu Pro Glu Gly Arg Gly Leu
            20                  25                  30

Ala Gly Pro Ala Glu Pro Glu Leu Glu Glu Glu Leu Glu Ala Asp His
        35                  40                  45

Thr Pro His Tyr Pro Glu Gln Glu Thr Glu Pro Pro Leu Gly Ser Cys
    50                  55                  60

Ser Asp Val Met Leu Ser Val Glu Glu Glu Gly Lys Glu Asp Pro Leu
65                  70                  75                  80

Pro Thr Ala Ala Ser Gly Lys
                85

<210>  7
<211>  400
<212>  PRT
<213>  Homo sapiens

<220>
<221>  SIGNAL
<222>  (1)..(23)
<223>  human IL-2 lead sequence

<220>
<221>  PEPTIDE
<222>  (24)..(137)
<223>  human IL-15

<220>
<221>  PEPTIDE
<222>  (138)..(161)
<223>  Peptide Linker

<220>
<221>  PEPTIDE
<222>  (162)..(400)

<223>  human IL-15Ra

<400>  7

Met Tyr Arg Met Gln Leu Leu Ser Cys Ile Ala Leu Ser Leu Ala Leu
1               5                   10                  15

Val Thr Asn Ser Ala Thr Ser Asn Trp Val Asn Val Ile Ser Asp Leu
            20                  25                  30

Lys Lys Ile Glu Asp Leu Ile Gln Ser Met His Ile Asp Ala Thr Leu
        35                  40                  45

Tyr Thr Glu Ser Asp Val His Pro Ser Cys Lys Val Thr Ala Met Lys
    50                  55                  60

Cys Phe Leu Leu Glu Leu Gln Val Ile Ser Leu Glu Ser Gly Asp Ala
65                  70                  75                  80

Ser Ile His Asp Thr Val Glu Asn Leu Ile Ile Leu Ala Asn Asn Ser
                85                  90                  95

Leu Ser Ser Asn Gly Asn Val Thr Glu Ser Gly Cys Lys Glu Cys Glu
            100                 105                 110

Glu Leu Glu Glu Lys Asn Ile Lys Glu Phe Leu Gln Ser Phe Val His
        115                 120                 125

Ile Val Gln Met Phe Ile Asn Thr Ser Ser Gly Gly Gly Ser Gly Gly
    130                 135                 140

Gly Gly Ser Gly Gly Gly Gly Ser Gly Gly Gly Gly Ser Gly Gly Gly
145                 150                 155                 160

Ser Leu Gln Ile Thr Cys Pro Pro Pro Met Ser Val Glu His Ala Asp
                165                 170                 175

Ile Trp Val Lys Ser Tyr Ser Leu Tyr Ser Arg Glu Arg Tyr Ile Cys
            180                 185                 190

Asn Ser Gly Phe Lys Arg Lys Ala Gly Thr Ser Ser Leu Thr Glu Cys
        195                 200                 205

Val Leu Asn Lys Ala Thr Asn Val Ala His Trp Thr Thr Pro Ser Leu
    210                 215                 220

Lys Cys Ile Arg Asp Pro Ala Leu Val His Gln Arg Pro Ala Pro Pro
225                 230                 235                 240

Ser Thr Val Thr Thr Ala Gly Val Thr Pro Gln Pro Glu Ser Leu Ser
                245                 250                 255

Pro Ser Gly Lys Glu Pro Ala Ala Ser Ser Pro Ser Ser Asn Asn Thr
            260                 265                 270

Ala Ala Thr Thr Ala Ala Ile Val Pro Gly Ser Gln Leu Met Pro Ser
        275                 280                 285

Lys Ser Pro Ser Thr Gly Thr Thr Glu Ile Ser Ser His Glu Ser Ser
    290                 295                 300

His Gly Thr Pro Ser Gln Thr Thr Ala Lys Asn Trp Glu Leu Thr Ala
305                 310                 315                 320

Ser Ala Ser His Gln Pro Pro Gly Val Tyr Pro Gln Gly His Ser Asp
                325                 330                 335

Thr Thr Val Ala Ile Ser Thr Ser Thr Val Leu Leu Cys Gly Leu Ser
            340                 345                 350

Ala Val Ser Leu Leu Ala Cys Tyr Leu Lys Ser Arg Gln Thr Pro Pro
        355                 360                 365

Leu Ala Ser Val Glu Met Glu Ala Met Glu Ala Leu Pro Val Thr Trp
    370                 375                 380

Gly Thr Ser Ser Arg Asp Glu Asp Leu Glu Asn Cys Ser His His Leu
385                 390                 395                 400